# EUROPEAN PATENT SPECIFICATION

(11) **EP 1 874 778 B1**
(45) Date of publication and mention of the grant of the patent: **23.12.2009**
(21) Application number: 06758390.6
(22) Date of filing: 18.04.2006
(51) Int. Cl.: C07D 487/04, A61K 31/53, A61P 29/00

(54) **PROCESS FOR PREPARING SALTS OF 4-[[5-[(CYCLOPROPYLAMINO)CARBONYL]-2-METHYLPHENYL]AMINO]-5-METHYL-N-PROPYLPYRROLO[2,1-F][1,2,4]TRIAZINE-6-CARBOXAMIDE AND NOVEL STABLE FORMS PRODUCED THEREIN**
VERFAHREN ZUR HERSTELLUNG VON SALZEN VON 4-[[5-[(CYCLOPROPYLAMINO)CARBONYL]-2-METHYLPHENYL]AMINO]-5-METHYL-N-PROPYLPYRROLO[2,1-F][1,2,4]TRIAZIN-6-CARBOXAMID UND DAMIT HERGESTELLTE NEUE STABILE FORMEN
PROCEDE POUR LA PREPARATION DE SELS DE 4-[[5-[(CYCLOPROPYLAMINO)CARBONYL]-2-METHYLPHENYL]AMINO]-5-METHYL-N-PROPYLPYRROLO[2,1-F][1,2,4]TRIAZINE-6-CARBOXAMIDE ET NOUVELLES FORMES STABLES PRODUITES DANS CE PROCEDE

(30) Priority: 18.04.2005 US 672255 P
(43) Date of publication of application: 09.01.2008
(73) Proprietor: Bristol-Myers Squibb Company, Princeton NJ 08543-4000 (US)
(72) Inventor: KIM, Soonjin, Demarest, New Jersey 07627 (US); MALLEY, Mary F., Lawrenceville, New Jersey 08648 (US); SHI, Zhongping, West Windsor, New Jersey 08550 (US)
(74) Representative: Reitstötter - Kinzebach
(86) International application number: PCT/US2006/014504
(87) International publication number: WO 2006/113682

(56) References cited:
- WO-A-03/090912

## Description

### REFERENCE TO OTHER APPLICATIONS

The present application takes priority from U.S. provisional application No. 60/672,255 filed April 18, 2005, the disclosure of which is incorporated herein by reference.

### FIELD OF THE INVENTION

The present invention relates to a process for preparing novel stable crystalline salt forms, including Form N-1 and Form N-4 crystalline forms of the monohydrochloride salt of the free base, and Form N-1 crystalline form of the methanesulfonic acid salt of the free base, of the kinase p38 inhibitor 4-[[5-[(cyclopropylamino)carbonyl]-2-methylphenyl]amino]-5-methyl-*N*-propylpyrrolo[2,1-f][1,2,4]triazine-6-carboxamide, to such novel Form N-1 and Form N-4 crystalline forms, to pharmaceutical compositions containing such novel Form N-1 and Form N-4 crystalline forms, and to methods of treating a mammal to inhibit the activity of p38 kinase, and treating p38 kinase-associated conditions such as rheumatoid arthritis employing such novel N-1 (methanesulfonic acid salt and hydrochloric acid salt) and N-4 (hydrochloric acid salt) crystalline forms.

### BACKGROUND OF THE INVENTION

A large number of cytokines participate in the inflammatory response, including IL-1, IL-6, IL-8 and TNF-α. Overproduction of cytokines such as IL-1 and TNF-α are implicated in a wide variety of diseases, including inflammatory bowel disease, rheumatoid arthritis, psoriasis, multiple sclerosis, endotoxin shock, osteoporosis, Alzheimer's disease, and congestive heart failure, among others [Henry et al., Drugs Fut., 24:1345-1354 (1999); Salituro et al., Curr. Med. Chem., 6:807-823 (1999)]. Evidence in human patients indicates that protein antagonists of cytokines are effective in treating chronic inflammatory diseases, such as, for example, monoclonal antibody to TNF-α (Enbrel) [Rankin et al., Br. J. Rheumatol., 34:334-342 (1995)], and soluble TNF-α receptor-Fc fusion protein (Etanercept) [Moreland et al., Ann. Intern. Med., 130:478-486 (1999)].

The biosynthesis of TNF-α occurs in many cell types in response to an external stimulus, such as, for example, a mitogen, an infectious organism, or trauma. Important mediators of TNF-α production are the mitogen-activated protein (MAP) kinases, and in particular, p38 kinase. These kinases are activated in response to various stress stimuli, including but not limited to proinflammatory cytokines, endotoxin, ultraviolet light, and osmotic shock. Activation of p38 requires dual phosphorylation by upstream MAP kinase kinases (MKK3 and MKK6) on threonine and tyrosine within a Thr-Gly-Tyr motif characteristic of p38 isozymes.

There are four known isoforms of p38, *i.e.*, p38-α, p38β, p38γ, and p38δ. The α and β isoforms are expressed in inflammatory cells and are key mediators of TNF-α production. Inhibiting the p38α and β enzymes in cells results in reduced levels of TNF-α expression. Also, administering p38α and β inhibitors in animal models of inflammatory disease has proven that such inhibitors are effective in treating those diseases. Accordingly, the p38 enzymes serve an important role in inflammatory processes mediated by IL-1 and TNF-α. Compounds that reportedly inhibit p38 kinase and cytokines such as IL-1 and TNF-α for use in treating inflammatory diseases are disclosed in US Pats. Nos. 6,277,989 and 6,130,235 to Scios, Inc; US Pats. Nos. 6,147,080 and 5,945,418 to Vertex Pharmaceuticals Inc; US Pats Nos. 6,251,914, 5,977,103 and 5,658,903 to Smith-Kline Beecham Corp.; US Pats. Nos. 5,932,576 and 6,087,496 to G.D. Searle & Co.; WO 00/56738 and WO 01/27089 to Astra Zeneca; WO 01/34605 to Johnson & Johnson; WO 00/12497 (quinazoline derivatives as p38 kinase inhibitors); WO 00/56738 (pyridine and pyrimidine derivatives for the same purpose); WO 00/12497 (discusses the relationship between p38 kinase inhibitors); and WO 00/12074 (piperazine and piperidine compounds useful as p38 inhibitors).

U.S. Application Serial No. 10/420,399 filed April 22, 2003 (hereinafter the 10/420,399 application) discloses compounds which are inhibitors of p38 kinase, which may be used for treating p38 kinase associated conditions including rheumatoid arthritis, and which compounds have the formula (I) 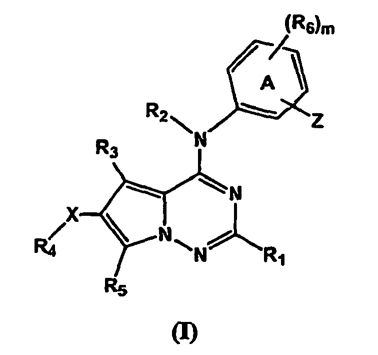 enantiomers, diastereomers, salts, and solvates thereof, wherein
X is selected from -O-, -OC(=O)-, -S-, -S(=O)-, -SO₂-, -C(=O)-, -CO₂-, -NR₈-, -NR₈C(=O)-, -NR₈C(=O)NR₉-, NR₈CO₂-, -NR₈SO₂-, -NR₈SO₂NR₉-, -SO₂NR₈-, -C(=O)NR₈-, halogen, nitro, and cyano, or X is absent;
Z is -C(=O)NR₁₀-B^{b}, -(CH₂)-C(=O)NR₁₀-B^{c}, -NR₁₀ₐ C(=O)-B^{a}, -(CH₂)-NR₁₀ₐC(=O)-B^{c}, -NR₁₀ₐC(=O)NR₁₀-B, -NR₁₀SO₂-B, -SO₂NR₁₀-B, -C(=O)-B^{a}, -CO₂-B^{e}, -OC(=O)-B^{a}, -C(=O)NR₁₀-NR₁₀ₐ-B^{d}, -NR₁₀CO₂-B^{a} or -C(=O)NR₁₀-(CH₂)C(=O)B^{a};
B is
   (a) optionally-substituted cycloalkyl, optionally-substituted heterocyclo, or optionally substituted heteroaryl; or
   (b) aryl substituted with one R₁₁ and zero to two R₁₂;
B^{a} is optionally substituted alkyl, optionally-substituted cycloalkyl, optionally-substituted heterocyclo, optionally substituted aryl, or optionally substituted heteroaryl;
B^{b} is
   (a) optionally-substituted cycloalkyl, optionally-substituted heterocyclo, or optionally substituted heteroaryl;
   (b) aryl substituted with one R₁₁ and zero to two R₁₂; or
   (c) -C(=O)R₁₃, -CO₂R₁₃, -C(=O)NR₁₃R₁₃ₐ;
B^{c} is optionally substituted alkyl, optionally substituted alkoxy, optionally-substituted cycloalkyl, optionally-substituted heterocyclo, optionally substituted aryl, or optionally substituted heteroaryl;
B^{d} is hydrogen, -C(=O)R₁₃, or -CO₂R₁₃;
B^{e} is hydrogen, optionally substituted alkyl, optionally-substituted cycloalkyl, optionally-substituted heterocyclo, optionally substituted aryl, or optionally substituted heteroaryl;
R₁ and R₅ are independently selected from hydrogen, alkyl, substituted alkyl, -OR₁₄, -SR₁₄, -OC(=O)R₁₄, -CO₂R₁₄, -C(=O)NR₁₄R₁₄ₐ, -NR₁₄R₁₄ₐ, -S(=O)R₁₄, -SO₂R₁₄, -SO₂NR₁₄R₁₄ₐ, -NR₁₄SO₂NR₁₄ₐR_{14b}, -NR₁₄ₐSO₂R₁₄, -NR₁₄C(=O)R₁₄ₐ, -NR₁₄CO₂R₁₄ₐ, -NR₁₄C(=O)NR₁₄ₐR_{14b}, halogen, nitro, and cyano;
R₂ is hydrogen or C₁₋₄alkyl;
R₃ is hydrogen, methyl, perfluoromethyl, methoxy, halogen, cyano, NH₂, or NH(CH₃);
R₄ is selected from:
   (a) hydrogen, provided that R₄ is not hydrogen if X is -S(=O)-, -SO₂-, -NR₈CO₂-, or -NR₈SO₂-;
   (b) alkyl, alkenyl, and alkynyl optionally independently substituted with keto and/or one to four R₁₇;
   (c) aryl and heteroaryl either of which may be optionally independently substituted with one to three R₁₆; and
   (d) heterocyclo and cycloalkyl either of which may be optionally independently substituted with keto and/or one to three R₁₆; or
   (e) R₄ is absent if X is halogen, nitro, or cyano;
R₆ is attached to any available carbon atom of phenyl ring A and at each occurrence is independently selected from alkyl, halogen, trifluoromethoxy, trifluoromethyl, hydroxy, alkoxy, alkanoyl, alkanoyloxy, thiol, alkylthio, ureido, nitro, cyano, carboxy, carboxyalkyl, carbamyl, alkoxycarbonyl, alkylthiono, arylthiono, arylsulfonylamine, alkylsulfonylamine, sulfonic acid, alkysulfonyl, sulfonamido, phenyl, benzyl, aryloxy, and benzyloxy, wherein each R₆ group in turn may be further substituted by one to two R₁₈;
R₈ and R₉ are independently selected from hydrogen, alkyl, substituted alkyl, aryl, cycloalkyl, heterocyclo, and heteroaryl;
R₁₀ and R₁₀ₐ are independently selected from hydrogen, alkyl, substituted alkyl, alkoxy, and aryl;
R₁₁ is selected from
   (a) alkyl, haloalkyl, alkoxy, haloalkoxy, -SO₂alkyl, cycloalkyl, heterocyclo, and heteroaryl any of which may be optionally substituted; or
   (b) halo, cyano, amino, alkylamino, and dialkylamino;
R₁₂ is selected from alkyl, R₁₇, and C₁₋₄alkyl substituted with keto (=O) and/or one to three R₁₇;
R₁₃ and R₁₃ₐ are independently selected from hydrogen, optionally substituted alkyl, optionally substituted cycloalkyl and optionally subsituted aryl;
R₁₄, R₁₄ₐ and R_{14b} are independently selected from hydrogen, alkyl, substituted alkyl, aryl, cycloalkyl, heterocyclo, and heteroaryl, except when R₁₄ is joined to a sulphonyl group as in -S(=O)R₁₄, -SO₂R₁₄, and -NR₁₄ₐSO₂R₁₄, then R₁₄ is not hydrogen;
R₁₆ is selected from alkyl, R₁₇, and C₁₋₄alkyl substituted with keto (=O) and/or one to three R₁₇;
R₁₇ is selected from
   (a) halogen, haloalkyl, haloalkoxy, nitro, cyano, -SR₂₃, -OR₂₃, -NR₂₃R₂₄, -NR₂₃SO₂R₂₅, -SO₂R₂₅, -SO₂NR₂₃R₂₄, -CO₂R₂₃, -C(=O)R₂₃, -C(=O)NR₂₃R₂₄, -OC(=O)R₂₃, -OC(=O)NR₂₃R₂₄, -NR₂₃C(=O)R₂₄, -NR₂₃CO₂R₂₄;
   (b) aryl or heteroaryl either of which may be optionally substituted with one to three R₂₆; or
   (c) cycloalkyl or heterocyclo optionally substituted with keto(=O) and/or one to three R₂₆;
R₁₈ and R₂₆ are independently selected from C₁₋₆alkyl, C₂₋₆alkenyl, halogen, haloalkyl, haloalkoxy, cyano, nitro, amino, C₁₋₄alkylamino, aminoC₁₋₄alkyl, hydroxy, hydroxyC₁₋₄alkyl, alkoxy, C₁₋₄alkylthio, aryl, heterocyclo, (aryl)alkyl, aryloxy, and (aryl)alkoxy;
R₂₃ and R₂₄ are each independently selected from hydrogen, alkyl, alkenyl, substituted alkyl, substituted alkenyl, aryl, cycloalkyl, heteroaryl, and heterocyclo;
R₂₅ is selected from alkyl, substituted alkyl, aryl, heteroaryl, cycloalkyl and heterocyclo; and
*m is* 0, 1, 2 or 3.

The 10/420,399 application further discloses that the compound of formula (I) may be prepared using the following reaction sequences:

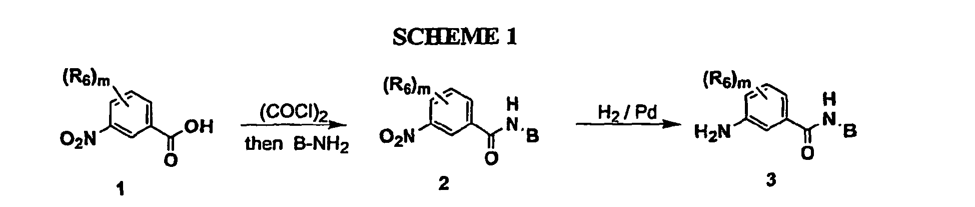

Scheme 1 is described as follows:

"Commercially-available compound (1) can be reacted with oxalyl chloride with heating and then concentrated *in vacuo* and reacted with an amine B-NH₂ in the presence of a base, such as diisopropylamine, in an organic solvent, such as dichloromethane (DCM) to yield compound (2). Compound (2) can be reacted with hydrogen in the presence of a catalyst, such as Pd, in an alcoholic solvent, such as ethanol (EtOH), at room temperature to afford compound (3). Compound (3) can then be used as in Scheme 2 to produce compounds (8) of Scheme 2."

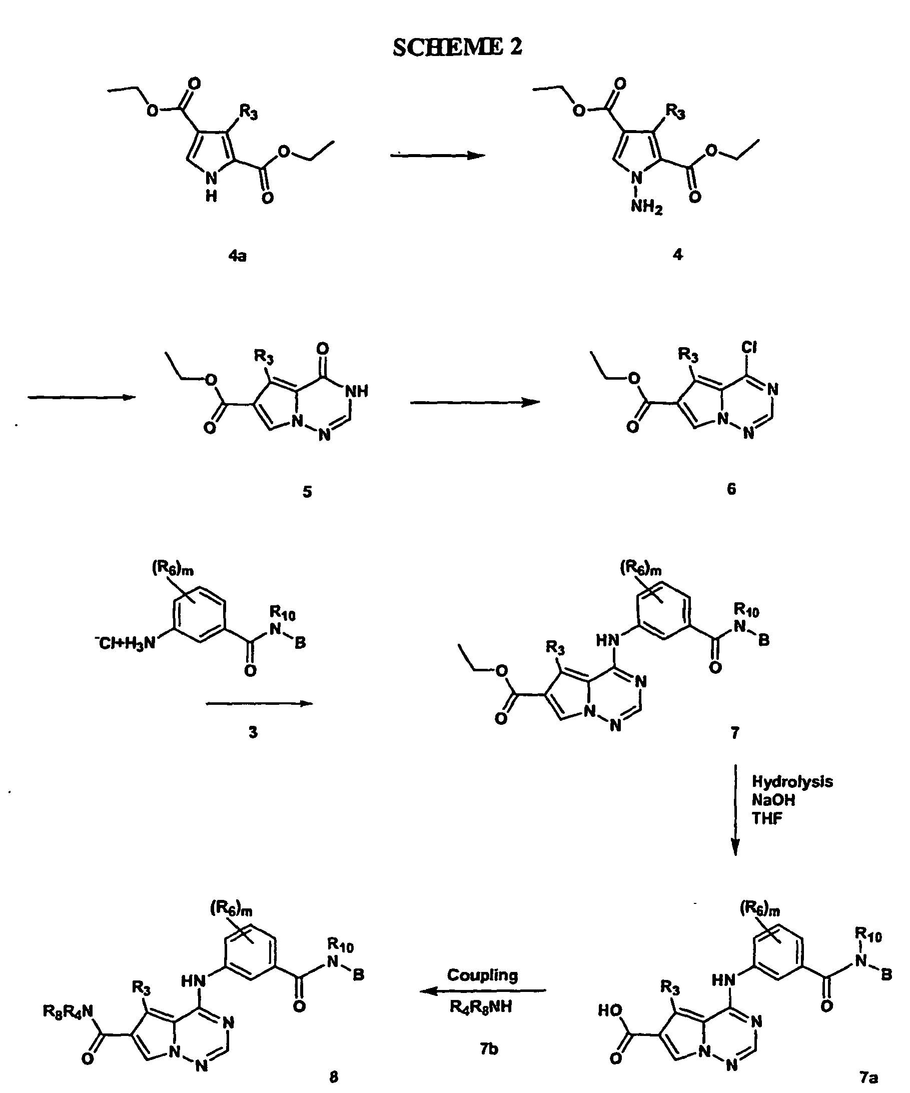

Scheme 2 is described as follows:

"3-methyl-1-pyrrole-2,4-diethyl ester can be reacted with chloramine in ether to produce compound (4). Reacting compound (4) in formamide with acetic acid produces compound (5). Compound (5) can be reacted with DIPEA and POCl₃ in toluene to produce compound (6). Compound (6) can be reacted with DIPEA and compound (3) in DMF to produce compound (7)." Compound (7) is hydrolyzed in THF with NaOH to produce acid intermediate 7a which upon treatment with HOBt, EDCI and the appropriate amine 7b in DMF produces compound 8.

U.S. Application Serial No. 10/420,399 also discloses that compounds of formula (I) form pharmaceutically acceptable (*i*.*e*. non-toxic, physiologically acceptable) salts. Such salts include salts formed with a variety of organic and inorganic acids which include salts formed with hydrochloric acid, hydrobromic acid, methanesulfonic acid, sulfuric acid, acetic acid, trifluoroacetic acid, oxalic acid, maleic acid, benzenesulfonic acid, toluenesulfonic acid and various others (*e*.*g.*, nitrates, phosphates, borates, tartrates, citrates, succinates, benzoates, ascorbates, salicylates and the like). It is further disclosed that such acid "salts can be formed as known to those skilled in the art."

Included among the many compounds covered by the 10/420,399 application is the compound of the structure 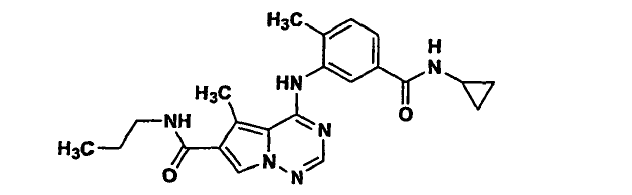 also referred to as 4-[[5-[(cyclopropylamino)carbonyl]-2-methylphenyl]amino]-5-methyl-*N*-propylpytrolo[2,1-f][1,2,4]triazine-6-carboxyamide or the free base.

### BRIEF DESCRIPTION OF THE INVENTION

In accordance with one aspect of the invention, novel crystalline salt forms of the free base of the structure I 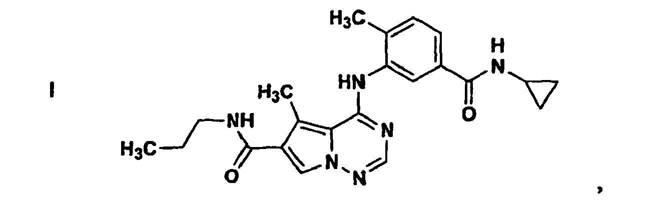 and a process for selectively preparing such novel crystalline salt forms of the free base I are provided.

The novel crystalline forms of the invention include Form N-1 of the hydrochloride salt of the free base I, Form N-4 of the hydrochloride salt of the free base I, the Form N-1 methanesulfonic acid (MSA) salt of the free base I, the SA-2 solvate of the hydrochloric acid salt of the free base I, the SB-2 solvate of the hydrochloric acid salt of the free base I and the H1.5-3 sesquihydrate of the hydrochloric acid salt of the free base I. Preferred are Form N-1 crystals of the hydrochloric acid salt of the free base I, and Form N-4 crystals of the hydrochloric acid salt of the free base I, both of which are non-hygroscopic from 25 up to 75% RH at 25°C and 30°C, respectively, and can be isolated and remain stable in the solid state form.

It has been found that Form N-4 of the hydrochloride salt can be consistently obtained. In addition, the processes of the invention produce hydrochloric acid salts having a controlled desired particle size which is smaller (D90 < 30 µm), and thus more desirable than obtainable with previous processes.

Form N-1 of the hydrochloric acid salt of the free base I crystallizes from organic solvents, preferably, THF, as small rods. PXRD patterns of lab batches of Form N-1 of the hydrochloric acid salt of the free base I match the hybrid PXRD pattern at room temperature. Solid state NMR also indicates a single phase. Thermal analysis using DSC indicates Form N-1 melts with disproportionation in the range from about 125 to about 225°C with negligible weight loss up to about 100°C and a weight loss of about 8.2% up to about 225°C.

The term "melts with disproportionation" as employed herein refers to the disassociation of the salt upon melting.

The terms "hydrochloric acid salt of the free base I", "hydrogen chloride salt of the free base I" or "hydrochloride salt" or "hydrochloride acid salt" are used interchangeably herein to refer to the HCl salt of the free base I.

A moisture sorption study indicates that the Form N-1 hydrochloride salt is non-hygroscopic in the range from about 25 to about 75% RH at 25°C.

Form N-4 of the hydrochloric acid salt of the free base I crystallizes from organic solvents, preferably DMF/acetone. PXRD patterns of lab batches of Form N-4 of the hydrochloric acid salt match the pattern simulated from the single crystal structure. Solid state NMR also indicates a single phase. Thermal analysis via DSC and TGA indicates Form N-4 melts with decomposition at from about 130 to about 220°C (variable) and has negligible weight loss up to about 125°C. A moisture sorption study indicates that the Form N-4 salt is non-hygroscopic in the range from about 25 to about 75% RH at 30°C. Slurries of Form N-1 and Form N-4 of the hydrochrloride salt in THF, acetonitrile, acetone and DMF/acetone convert to Form N-4 at room temperature indicating that Form N-4 is the stable form at room temperature.

The Form N-4 salt will preferably have an average particle size distribution of 95% < 60 µm.

Form N-1 of the methanesulfonic acid salt of the free base I crystallizes from organic solvents, preferably DMF, DMF/acetone or aqueous acetonitrile, as thin, elongated plates which have a neat crystal structure, N-1. PXRD of lab batches of Form N-1 of the methanesulfonic acid salt of the free base I match the PXRD pattern simulated from the single crystal structure. Solid state NMR also indicates a single phase. Thermal analysis via DSC and TGA indicates that Form N-1 of the methanesulfonic acid salt of the free base I melts with decomposition with endotherm onset at 216°C and has negligible weight loss up to about 150°C.

The SA-2 solvate of the hydrochloric acid salt of the free base I is a mixed solvate (methanol/water). Single crystal structures of hydrated methanolate are obtained from methylethyl ketone/methanol. The crystals are unstable at room temperature.

The SB-2 solvate of the hydrochloric acid salt of the free base I is a mixed solvate (isopropyl alcohol/water). Single crystal structures of hydrated isopropylate are obtained from isopropyl alcohol. The crystals are unstable at room temperature.

The H1.5-3 form of the hydrochloric acid salt of the free base I is an unstable sesquihydrate form obtained as plates from 95% ethanol. Hot stage indicates desolvation at ∼45°C and single crystals are unstable in a stream of dry N₂ at -50°C.

The Form N-1 of the hydrochloric acid salt of the free base I and Form N-4 of the hydrochloride acid salt of the free base I are preferred. The Form N-4 salt is the most preferred form.

The various forms of the salts of the free base I according to the invention may be characterized using various techniques, the operation of which are well known to those of ordinary skill in the art. The forms may be characterized and distinguished using single crystal X-ray diffraction, which is based on unit cell measurements of a single crystal of a form at a fixed analytical temperature. A detailed description of unit cells is provided in Stout & Jensen, X-Ray Structure Determination: A Practical Guide, Macmillan Co., New York (1968), Chapter 3, which is herein incorporated by reference. Alternatively, the unique arrangement of atoms in spatial relation within the crystalline lattice may be characterized according to the observed fractional atomic coordinates. Another means of characterizing the crystalline structure is by powder X-ray diffraction analysis in which the experimental or observed diffraction profile is compared to a simulated profile representing pure powder material, both run at the same analytical temperature, and measurements for the subject form characterized as a series of 29 values.

Other means of characterizing the form may be used, such as solid state nuclear magnetic resonance (SSNMR), differential scanning calorimetry and thermogravimetric analysis. These parameters may also be used in combination to characterize the subject form.

In one aspect of the invention, Form N-1 of the hydrochloric acid salt of the free base I may be characterized by unit cell parameters substantially equal to the following:

**Cell dimensions**

| | Single Crystal at -50°C | Hybrid at RT |
|---|---|---|
| a | 22.50(3) Å | 22.73 Å |
| b | 14.667(8) Å | 14.710 Å |
| c | 14.96(1) Å | 15.04 Å |
| α | 90° | 90° |
| β | 116.78(5)° | 117.13 |
| γ | 90° | 90° |

| | | |
|---|---|---|
| Space group C2/c Molecules/asymmetric unit 1 | | |

In a different aspect of the invention, Form N-1 HCl salt may be characterized by fractional atomic coordinates substantially as listed in Table 4.

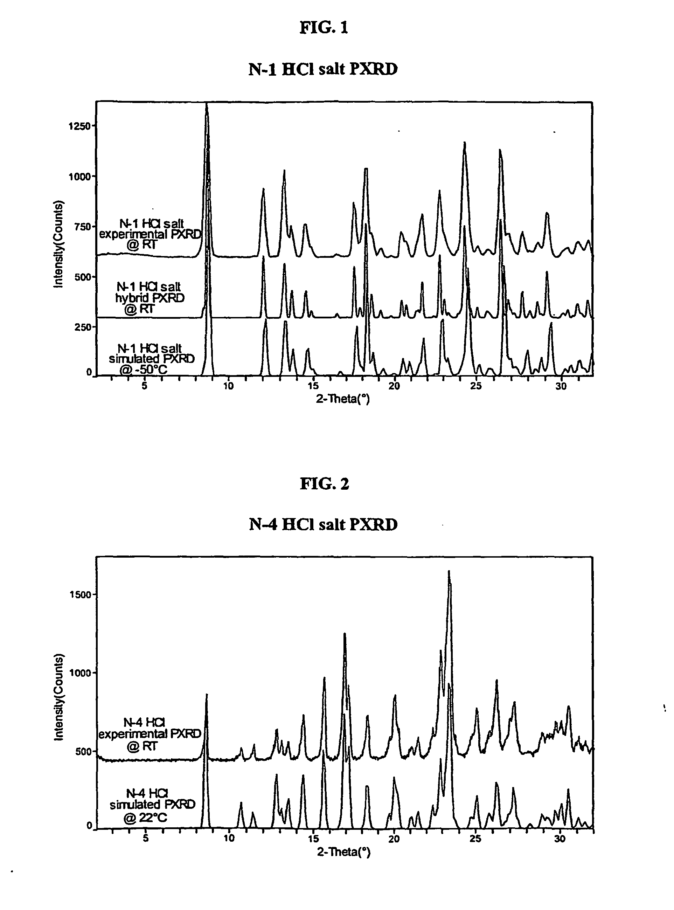

In a different aspect of the present invention, Form N-1 of the hydrochloric acid salt of the free base I may be characterized by simulated, hybrid and observed powder X-ray diffraction patterns as shown in Figure 1.

In a different aspect of the invention, Form N-1 HCl salt may be characterized by a powder X-ray diffraction pattern having the following 2θ values (CuKα λ= 1.5418 Å) 8.7 ± 0.1, 12.1 ± 0.1, 13.3 ± 0.1, 13.7 ± 0.1, 14.6 ± 0.1, 17.5 ± 0.1, 18.2 ± 0.1, 21.7 ± 0.1, 22.8 ± 0.1 and 24.3 ± 0.1, at about RT.

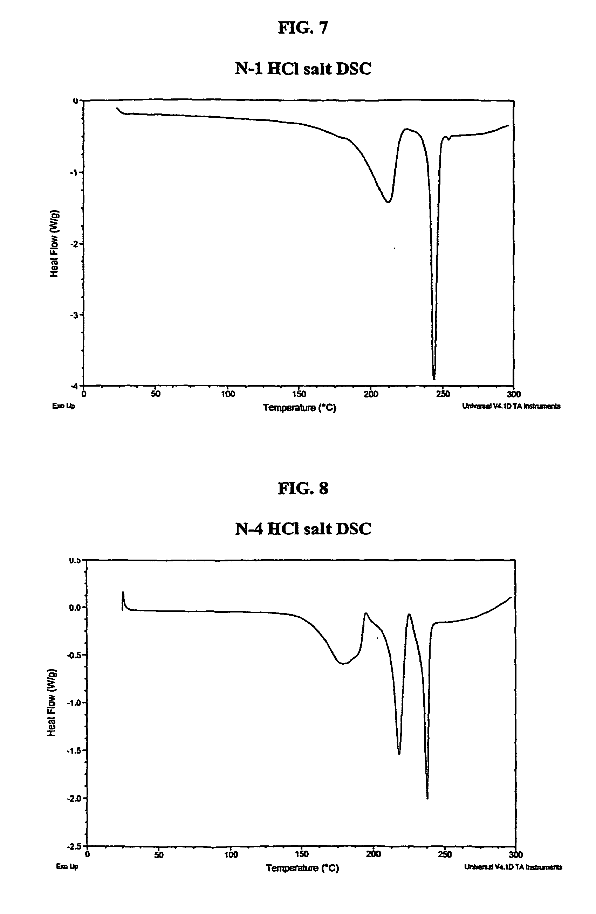

In a different aspect of the invention, Form N-1 HCl salt may be characterized by a differential scanning calorimetry thermogram having an endotherm typically within the range from about 125 to about 225°C as shown in Figure 7.

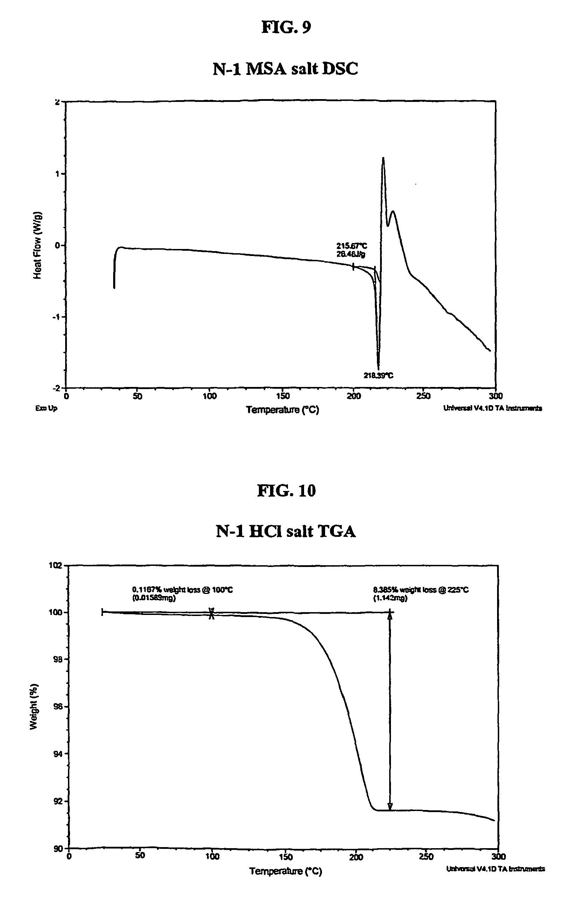

In a different aspect of the invention, Form N-1 may be characterized by a thermal gravimetric analysis curve having a negligible weight loss at about 100°C and a weight loss up to about 8.2% at about 225°C as shown in Figure 10.

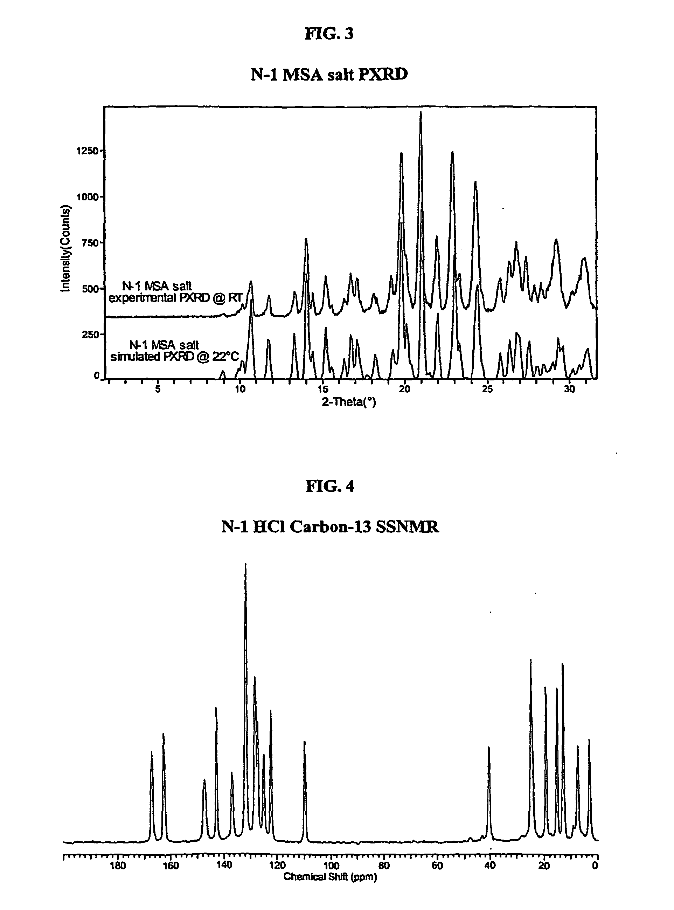

In a different aspect of the present invention, Form N-1 of the hydrochloric acid salt of the free base I may be characterized by the SSNMR chemical shifts shown in Table 3 and by the spectrum shown in Figure 4.

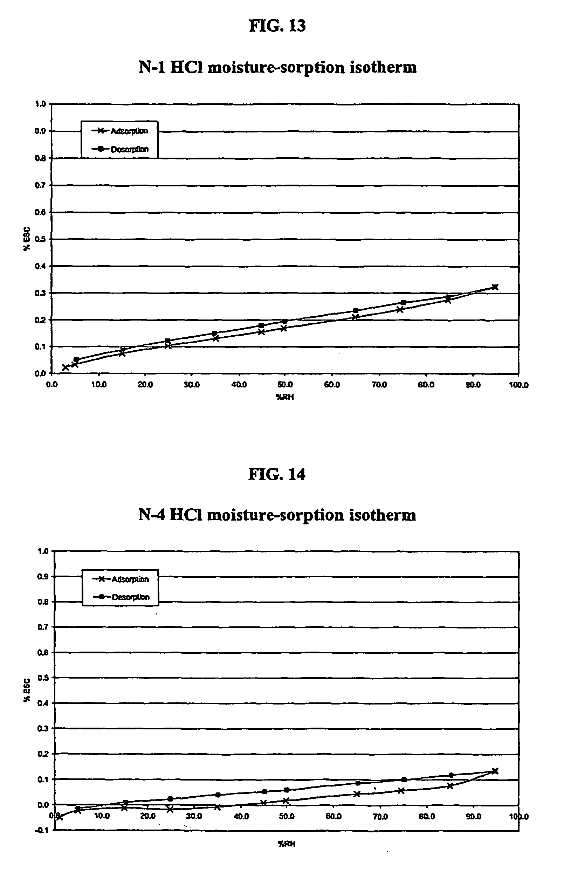

In a different aspect of the present invention, Form N-1 HCl salt may be characterized by the moisture-sorption isotherm shown in Figure 13 with negligible water uptake in the range from 25 to 75% RH at 25°C.

In another aspect of the present invention, Form N-4 of the hydrochloric acid salt of the free base I may be characterized by unit cell parameters substantially equal to the following:

**Cell dimensions:**
a = 20.9498(5) Å
b= 13.8719(3) Å
c = 7.9133(2) Å
α = 90°
β = 100.052(1)°
γ = 90°
Space group P2₁/n
Molecules/asymmetric unit I
wherein the crystalline form is at about +22°C.

In a different aspect of the invention, Form N-4 of the hydrochloric acid salt of the free base I may be characterized by fractional atomic coordinates substantially as listed in Table 5.

In a different aspect of the invention, Form N-4 of the hydrochloric acid salt of the free base I may be characterized by simulated and observed powder X-ray diffraction patterns as shown in Figure 2.

In a different aspect of the invention, Form N-4 of the hydrochloric acid salt of the free base I may be characterized by a powder X-ray diffraction pattern having the following 2θ values (CuKα λ= 1.5418 Å) 8.6 ± 0.1, 10.7 ± 0.1, 11.4 ± 0.1, 12.8 ± 0.1, 14.4 ± 0.1, 15.6 ± 0.1, 16.9 ± 0.1, 20.0 ± 0.1 and 23.4 ± 0.1, at about RT.

In a different aspect of the invention, Form N-4 of the hydrochloric acid salt of the free base I may be characterized by a differential scanning calorimetry thermogram as shown in Figure 8 having an endotherm typically in the range from about 130 to about 220°C (variable).

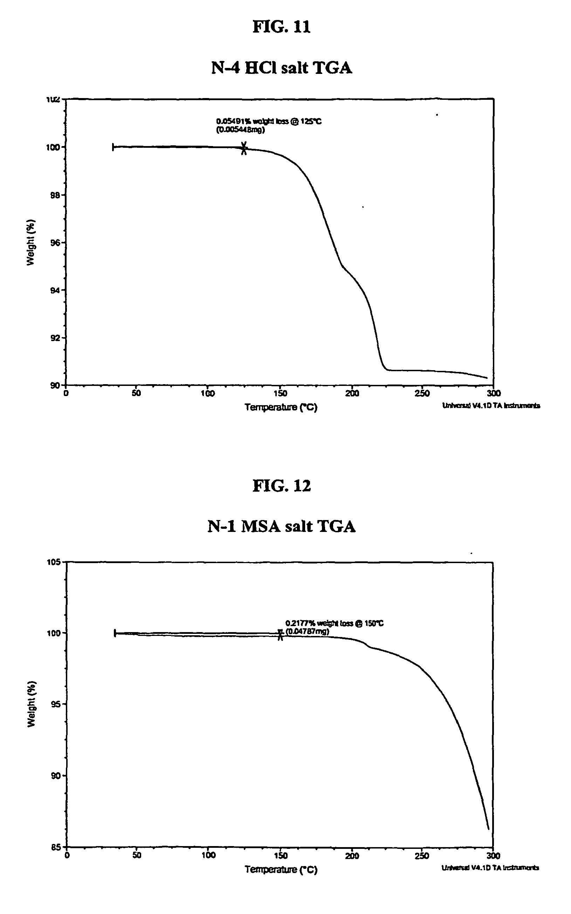

In a different aspect of the invention, Form N-4 of the hydrochloric acid salt of the free base I may be characterized by a thermal gravimetric analysis curve having a negligible weight loss up to at about 125°C as shown in Figure 11.

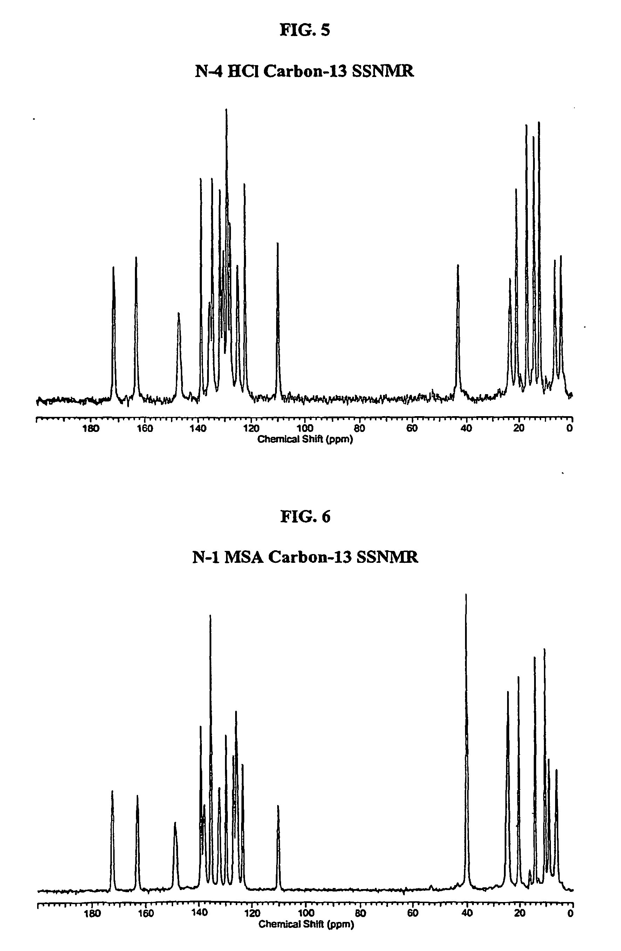

In a different aspect of the present invention, Form N-4 of the hydrochloric acid salt of the free base I may be characterized by the SSNMR chemical shifts shown in Table 3 and the spectrum shown in Figure 5.

In a different aspect of the invention, Form N-4 of the hydrochloric acid salt of the free base I may be characterized by the moisture-sorption isotherm shown in Figure 14 with negligible water uptake in the range from 25 to 75% RH at 30°C.

In another aspect of the invention, Form N-1 of the methanesulfonic acid salt of the free base I may be characterized by unit cell parameters substantially equal to the following:

**Cell dimensions:**
a = 9.818(1) Å
b= 11.127(1) Å
c = 13.004(1) Å
α = 97.32(1)°
β = 110.17(1)°
γ = 111.48(1)°
Space group P-1
Molecules/asymmetric unit 1
wherein the crystalline form is at about +22°C.

In a different aspect of the present invention, Form N-1 MSA salt of the free base I may be characterized by fractional atomic coordinates substantially as listed in Table 6.

In a different aspect of the invention, Form N-1 of the methanesulfonic acid salt of the free base I may be characterized by simulated and observed powder X-ray diffraction patterns as shown in Figure 3.

In a different aspect of the invention, Form N-1 MSA salt of the free base I may be characterized by a powder X-ray diffraction pattern comprising the following 2θ values (CuKα λ= 1.5418 Å) 10.7 ± 0.1, 11.7 ± 0.1, 13.3 ± 0.1, 14.0 ± 0.1, 15.2 ± 0.1, 19.8 ± 0.1, 21.0 ± 0.1, 22.0 ± 0.1, 23.0 ± 0.1 and 24.4 ± 0.1, at about RT.

In a different aspect of the invention, Form N-1 MSA salt of the free base I may be characterized by a differential scanning calorimetry thermogram as shown in Figure 9 having an endotherm with peak onset at about 216°C.

In a different aspect of the invention, Form N-1 MSA salt of the free base I may be characterized by a thermal gravimetric analysis curve having a negligible weight loss up to about 150°C as shown in Figure 12.

In a different aspect of the present invention, Form N-1 MSA salt of the free base I may be characterized by the SSNMR chemical shifts shown in Table 3 and the spectrum shown in Figure 6.

The term "negligible weight loss", as employed herein, as characterized by TGA indicates the presence of a neat (non-solvated) crystal form.

The term "negligible % water uptake", as employed herein, as characterized by moisture-sorption isotherm indicates that the form tested is non-hygroscopic.

In accordance with another aspect of the invention, a process is provided for preparing the hydrochloric acid salt of free base I in the form of Form N-1 crystals, which includes the steps of
a) providing the free base having the structure I 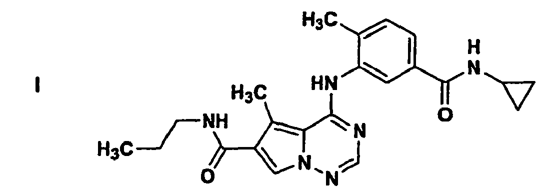 suspended in an organic solvent, preferably tetrahydrofuran;
b) reacting the free base I with an aqueous solution of hydrochloric acid;
c) seeding the reaction mixture from b) with Form N-1 seed crystals of the hydrochloric acid salt of the free base I; and
d) recovering hydrochloric acid salt in the form of Form N-1 crystals.

An alternative preferred embodiment of the process of the invention for preparing the hydrochloric acid salt of free base I in the form of Form N-1 crystals includes the steps of:
a) providing the free base I suspended or dissolved in N,N-dimethylformamide or N,N-dimethylacetamide;
b) reacting the free base I with an aqueous solution of hydrochloric acid;
c) seeding the reaction mixture from b) with Form N-1 seed crystals of the hydrochloric acid salt of the free base I;
d) adding acetone or methylethyl ketone (MEK) to the reaction mixture from c); and
e) recovering hydrochloric acid salt in the form of Form N-1 crystals.

The Form N-1 seed crystals of the HCl salt (employed in the above processes of the invention) may be prepared by:
a) suspending the free base I in an organic solution such as tetrahydrofuran or acetonitrile;
b) reacting the free base I with an aqueous solution of hydrochloric acid; and
c) recovering hydrochloric acid salt in the form of Form N-1 crystals.

Further, in accordance with another aspect of the invention, a preferred process is provided for preparing the Form N-1 methanesulfonic acid salt of the free base having the structure I which includes the steps of
a) providing a solution of a free base having the structure I 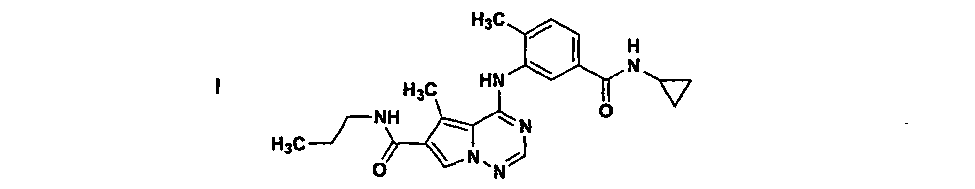 in N,N-dimethylformamide;
b) reacting the free base with methanesulfonic acid;
c) adding acetone to the reaction mixture;
d) seeding the reaction mixture with crystals of Form N-1 methanesulfonic acid salt of the free base I; and
e) recovering crystals of Form N-1 methanesulfonic acid salt.

Alternatively, in accordance with still another aspect of the present invention, a preferred process is provided for preparing the Form N-1 methanesulfonic acid salt of the free base I which includes the steps of:
a) providing a suspension of free base I in an organic solvent such as DMF, isopropyl alcohol, ethanol, ethyl acetate or acetonitrile, preferably DMF or acetonitrile;
b) reacting the free base with methanesulfonic acid;
c) seeding the reaction mixture with crystals of Form N-1 methanesulfonic acid salt of the free base I; and
d) recovering crystals of Form N-1 methanesulfonic acid salt.

The Form N-1 seed crystals of the methanesulfonic acid salt employed in the above processes of the invention may be prepared by:
a) suspending the free base I in an organic solvent such as DMF, isopropyl alcohol, ethanol, ethyl acetate or acetonitrile, preferably DMF or acetonitrile;
b) reacting the free base I with methanesulfonic acid; and
c) recovering methanesulfonic acid salt in the form of Form N-1 crystals.

Still further in accordance with another aspect of the invention, a preferred process is provided for selectively preparing the hydrochloric acid salt of the free base of the structure I in the form of Form N-4 crystals, which includes the steps of
a) providing a slurry of free base of the structure I 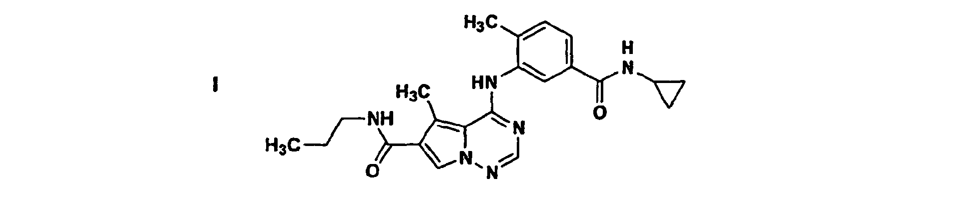 in formic acid and methylethyl ketone, or formic acid and acetone;
b) admixing an aqueous hydrochloric acid solution with the slurry of step a);
c) optionally filtering the reaction mixture of step b);
d) (in a so-called reversed addition procedure) adding the filtered reaction mixture of c) to a slurry of seeds of Form N-4 crystals of the hydrochloride salt of free base I in methylethyl ketone or acetone, preferably employing the same solvent as employed in step a); and
e) recovering the hydrochloric acid salt of the free base in the form of Form N-4 crystals.

In addition, in accordance with still another aspect of the invention, a preferred process is provided for preparing the hydrochloric acid salt of the free base of the structure I in the form of Form N-4 crystals, which includes the steps of
a) providing a slurry or solution of free base of the structure I 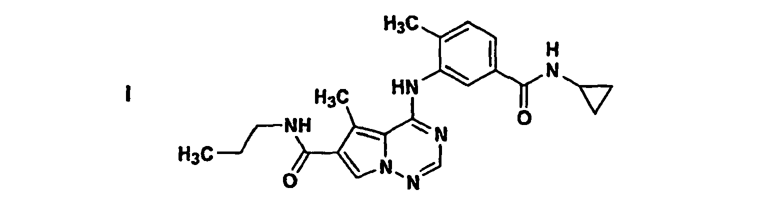 in formic acid and acetone or in formic acid and methylethyl ketone (MEK);
b) (in a so-called normal addition procedure) adding an aqueous hydrochloric acid solution to the slurry or solution of step a);
c) optionally filtering the resulting reaction mixture;
d) optionally adding acetone to the filtered reaction mixture;
e) adding seeds of Form N-4 crystals of the hydrochloride salt of the free base I and acetone or MEK to the reaction mixture of steps b), c) and d); and
f) recovering the hydrochloric acid salt of the free base in the form of Form N-4 crystals.

Alternatively, the solution of the free base can be added to a mixture of acetone or MEK, hydrochloric acid and seeds of Form N-4 crystals of the hydrochloride salt to effect precipitation of small crystals of Form N-4.

In addition, in accordance with yet another aspect of the invention, a preferred process is provided for preparing the hydrochloric acid salt of free base I in the form of N-4 crystals, which includes the steps of:
a) providing a suspension or solution of free base I in an organic solvent, preferably ethanol, acetone or tetrahydrofuran;
b) adding an aqueous hydrochloric acid solution to the suspension or solution of step a);
c) adding seeds of Form N-4 crystals of the hydrochloric acid salt of the free base I to the reaction mixture of step b); and
d) recovering the hydrochloric acid salt of the free base I in the form of Form N-4 crystals.

In yet another embodiment of the preferred process of the invention, the hydrochloric acid salt of free base I in the form of Form N-4 crystals is prepared via the following steps:
a) providing a suspension or solution of free base I in N,N-dimethylformamide;
b) adding a solution of aqueous hydrochloric acid to the suspension of step a) to form a solution;
c) adding acetone or MEK to the solution of step b);
d) adding to the mixture of step c) seeds of Form N-4 hydrochloric acid salt of the free base I; and
e) recovering Form N-4 crystals of the hydrochloric acid salt of the free base I.

In still yet another embodiment of the invention, a preferred process is provided for preparing the hydrochloric acid salt of the free base I in the form of N-4 crystals, which includes the steps of:
a) providing a solution of free base I in N,N-dimethylacetamide;
b) adding an aqueous hydrochloric acid solution to the solution of step a);
c) adding seeds of Form N-4 crystals of the hydrochloric acid salt of the free base I to the reaction mixture of step b);
d) adding acetone or MEK to the reaction mixture of step c); and
e) recovering the hydrochloric acid salt of the free base I in the form of Form N-4 crystals.

In another embodiment of the process of the invention, a preferred process is provided for preparing the hydrochloric acid salt of free base I in the form of Form N-4 crystals, which includes the steps of:
a) providing a slurry of Form N-1 crystals of the hydrochloric acid salt in an organic solvent such as acetonitrile, tetrahydrofuran, ethanol or acetone and seeds of Form N-4 crystals of the hydrochloric acid salt of the free base I;
b) heating the resulting reaction mixture of step a) at a temperature within the range from about 20 to about 50°C; and
c) recovering the hydrochloric acid salt of the free base I in the form of Form N-4 crystals.

Further, in accordance with another aspect of the invention, a preferred process is provided for preparing the hydrochloric acid salt of a free base of the structure I in the form of N-4 crystals, which includes the steps of
a) providing a solution of a free base of the structure I dissolved in N,N-dimethylacetamide at a temperature within the range from about 50 to about 75°C;
b) providing a solution of aqueous hydrochloric acid and cooled acetone or NMK;
c) adding into the acetone/HCl solution or MEK/HCl solution seeds of Form N-4 hydrochloric acid salt of the free base I;
d) adding the solution of free base I in N,N-dimethylacetamide from step

a) maintained at a temperature within the range from about 60 to about 65°C, into the seeded cooled acetone/HCl solution or MEK/HCl solution of step c) while stirring, to form a slurry; and
e) recovering Form N-4 crystals of the hydrochloric acid salt of the free base I.

Yet further in accordance with another aspect of the invention, a preferred process is provided for preparing the hydrochloric acid salt of the free base of the structure I in the form of N-4 crystals, which includes the steps of
a) providing a slurry of a free base of the structure I dissolved in N,N-dimethylformamide, N,N-dimethylformamide/acetone (most preferred) or N,N-dimethylformamide/MEK;
b) adding a solution of aqueous hydrochloric acid and acetone or MEK to the slurry of step a) to form a solution;
c) optionally filtering off insoluble solids from the solution of step b);
d) adding into the acetone/HCl solution the solution of step b) or c) seeds of Form N-4 hydrochloric acid salt of the free base I in acetone; and
e) recovering Form N-4 crystals of the hydrochloric acid salt of the free base I.

Still further, in accordance with yet another aspect of the invention, a preferred process is provided for preparing the hydrochloric acid salt of the free base of the structure I in the form of N-4 crystals, which includes the steps of
a) providing a slurry of a free base of the structure I dissolved in N,N-dimethylformamide, N,N-dimethylformamide/acetone or N,N-dimethylformamide/MEK;
b) adding a solution of aqueous hydrochloric acid and acetone or MEK to the slurry of step a) to form a solution;
c) optionally filtering off insoluble solids from the solution of step b);
d) adding seeds of Form N-4 hydrochloric acid salt as a slurry in acetone to the solution obtained in step c); and
e) recovering Form N-4 crystals of the hydrochloric acid salt of the free base I.

The Form N-4 seed crystals employed in the above processes of the invention may be prepared by:
a) suspending the free base I in ethanol;
b) reacting the suspension of free base I with an aqueous solution of hydrochloric acid; and
c) recovering hydrochloric acid salt of Form N-4 crystals.

### BRIEF DESCRIPTION OF THE FIGURES

Figure 1 shows calculated (simulated) (-50°C) and the hybrid (RT) and observed (experimental at room temperature) powder X-ray diffraction patterns (CuKα λ = 1.5418 Å) of Form N-1 crystals of the hydrochloric acid salt of 4-[[5-[(cyclopropylamino)carbonyl]-2-methylphenyl]amino]-5-methyl-*N*-propylpyrrolo[2,1-f][1,2,4]triazine-6-carboxamide;
Figure 2 shows calculated (simulated) (22°C) and observed (experimental at room temperature) powder X-ray diffraction patterns (CuKα λ = 1.5418 Å) of Form N-4 crystals of the hydrochloric acid salt of 4-[[5-[(cyclopropylamino)carbonyl]-2-methylphenyl]amino]-5-methyl-*N*-propylpyrrolo[2,1-f][1,2,4]triazine-6-carboxamide;
Figure 3 shows calculated (simulated) (22°C) and observed (experimental at room temperature) powder X-ray diffraction patterns (CuKα λ = 1.5418 Å) of the methanesulfonic acid (MSA) salt of 4-[[5-[(cyclopropylamino)carbonyl]-2-methylphenyl]amino]-5-methyl-*N*-propylpyrrolo[2,1-f][1,2,4]triazine-6-carboxamide;
Figure 4 shows a C-13 solid state NMR of Form N-1 crystals of the hydrochloric acid salt of 4-[[5-[(cyclopropylamino)carbonyl]-2-methylphenyl]amino]-5-methyl-*N*-propylpyrrolo[2,1-f][1,2,4]triazine-6-carboxamide;
Figure 5 shows a C-13 solid state NMR of Form N-4 crystals of the hydrochloric acid salt of 4-[[5-[(cyclopropylamino)carbonyl]-2-methylphenyl]amino]-5-methyl-*N*-propylpyrrolo[2,1-f][1,2,4]triazine-6-carboxamide;
Figure 6 shows a C-13 solid state NMR of Form N-1 crystals of the methanesulfonic acid salt of 4-[[5-[(cyclopropylamino)carbonyl]-2-methylphenyl]amino]-5-methyl-*N*-propylpyrrolo[2,1-f][1,2,4]triazine-6-carboxamide;
Figure 7 shows a differential scanning calorimetry (DSC) thermogram of Form N-1 crystals of the hydrochloric acid salt of 4-[[5-[(cyclopropylamino)carbonyl]-2-methylphenyl]amino]-5-methyl-*N*-propylpyrrolo[2,1-f][1,2,4]triazine-6-carboxamide;
Figure 8 shows a differential scanning calorimetry (DSC) thermogram of Form N-4 crystals of the hydrochloric acid salt of 4-[[5-[(cyclopropylamino)carbonyl]-2-methylphenyl]amino]-5-methyl-*N*-propylpyrrolo[2,1-f][1,2,4]triazine-6-carboxamide;
Figure 9 shows a differential scanning calorimetry (DSC) thermogram of Form N-1 crystals of the methanesulfonic acid salt of 4-[[5-[(cyclopropylamino)carbonyl]-2-methylphenyl]amino]-5-methyl-*N*-propylpyrrolo[2,1-f][1,2,4]triazine-6-carboxamide;
Figure 10 shows a thermogravimetric analysis (TGA) curve of Form N-1 crystals of the hydrochloric acid salt of 4-[[5-[(cyclopropylamino)carbonyl]-2-methylphenyl]amino]-5-methyl-*N*-propylpyrrolo[2,1-f][1,2,4]triazine-6-carboxamide;
Figure 11 shows a thermogravimetric analysis (TGA) curve of Form N-4 crystals of the hydrochloric acid salt of 4-[[5-[(cyclopropylamino)carbonyl]-2-methylphenyl]amino]-5-methyl-*N*-propylpyrrolo[2,1-f][1,2,4]triazine-6-carboxamide;
Figure 12 is a thermogravimetric analysis (TGA) curve of Form N-1 crystals of the methanesulfonic acid salt of 4-[[5-[(cyclopropylamino)carbonyl]-2-methylphenyl]amino]-5-methyl-*N*-propylpyrrolo[2,1-f][1,2,4]triazine-6-carboxamide;
Figure 13 is a moisture-sorption isotherm of Form N-1 crystals of the hydrochloride acid salt of 4-[[5-[(cyclopropylamino)carbonyl]-2-methylphenyl]amino]-5-methyl-*N*-propylpyrrolo[2,1-f][1,2,4]triazine-6-carboxamide; and
Figure 14 is a moisture-sorption isotherm of Form N-4 crystals of the hydrochloric acid salt of 4-[[5-[(cyclopropylamino)carbonyl]-2-methylphenyl]amino]-5-methyl-*N*-propylpyrrolo[2,1-f][1,2,4]triazine-6-carboxamide.

### DETAILED DESCRIPTION OF THE INVENTION

The present invention provides, at least in part, crystalline forms of free base I as a novel material, in particular in pharmaceutically acceptable form. The term "pharmaceutically acceptable", as used herein, refers to those compounds, materials, compositions, and/or dosage forms which are, within the scope of sound medical judgment, suitable for contact with the tissues of human beings and animals without excessive toxicity, irritation, allergic response, or other problem complications commensurate with a reasonable benefit/risk ratio. In certain preferred embodiments, crystalline salt forms of free base I are in substantially pure form. The term "substantially pure", as used herein, means a compound having a purity greater than about 90% including, for example, about 91%, about 92%, about 93%, about 94%, about 95%, about 96%, about 97%, about 98%, about 99%, and about 100%.

The term "reversed addition procedure" as employed herein refers to the step of the process of the invention wherein a slurry or solution of free base I and hydrochloric acid is added to a slurry of seeds of Form N-4 crystals of the hydrochloric acid salt of the free base I.

The term "normal addition procedure" as employed herein refers to the step of the process of the invention wherein a slurry of seeds of Form N-4 crystals of the hydrochloric acid salt of the free base I is added to a slurry or solution of free base I and hydrochloric acid.

As used herein "polymorph" refers to crystalline forms having the same chemical composition but different spatial arrangements of the molecules, atoms, and/or ions forming the crystal.

As used herein "solvate" refers to a crystalline form of a molecule, atom, and/or ions that further contains molecules of a solvent or solvents incorporated into the crystalline structure. The solvent molecules in the solvate may be present in a regular arrangement and/or a non-ordered arrangement. The solvate may contain either a stoichiometric or nonstoichiometric amount of the solvent molecules. For example, a solvate with a nonstoichiometric amount of solvent molecules may result from partial loss of solvent from the solvate.

Samples of the crystalline forms may be provided with substantially pure phase homogeneity, indicating the presence of a dominant amount of a single crystalline form and optionally minor amounts of one or more other crystalline forms. The presence of more than one crystalline form in a sample may be determined by techniques such as powder X-ray diffraction (PXRD) or solid state nuclear magnetic resonance spectroscopy (SSNMR). For example, the presence of extra peaks in the comparison of an experimentally measured PXRD pattern with a simulated PXRD pattern may indicate more than one crystalline form in the sample. The simulated PXRD may be calculated from single crystal X-ray data. see Smith, D.K, "A FORTRAN Program for Calculating X-Ray Powder Diffraction Patterns," Lawrence Radiation Laboratory, Livermore, California, UCRL-7196 (April 1963). Preferably, the crystalline form has substantially pure phase homogeneity as indicated by less than 10%, preferably less than 5 %, and more preferably less than 2 % of the total peak area in the experimentally measured PXRD pattern arising from the extra peaks that are absent from the simulated PXRD pattern. Most preferred is a crystalline form having substantially pure phase homogeneity with less than 1% of the total peak area in the experimentally measured PXRD pattern arising from the extra peaks that are absent from the simulated PXRD pattern.

Procedures for the preparation of crystalline forms are known in the art. The crystalline forms may be prepared by a variety of methods, including for example, crystallization or recrystallization from a suitable solvent, sublimation, growth from a melt, solid state transformation from another phase, crystallization from a supercritical fluid, and jet spraying. Techniques for crystallization or recrystallization of crystalline forms from a solvent mixture include, for example, evaporation of the solvent, decreasing the temperature of the solvent mixture, crystal seeding a supersaturated solvent mixture of the molecule and/or salt, freeze drying the solvent mixture, and addition of antisolvents (countersolvents) to the solvent mixture.

Crystals of drugs, including polymorphs, methods of preparation, and characterization of drug crystals are discussed in Solid-State Chemistry of Drugs, S.R. Byrn, R.R. Pfeiffer, and J.G. Stowell, 2nd Edition, SSCI, West Lafayette, Indiana (1999).

For crystallization techniques that employ solvent, the choice of solvent or solvents is typically dependent upon one or more factors, such as solubility of the compound, crystallization technique, and vapor pressure of the solvent. Combinations of solvents may be employed, for example, the compound may be solubilized into a first solvent to afford a solution, followed by the addition of an antisolvent to decrease the solubility of the compound in the solution and to afford the formation of crystals. An antisolvent is a solvent in which the compound has low solubility. Suitable solvents for preparing crystals include polar and nonpolar solvents.

In one method to prepare crystals, free base I or a salt thereof is suspended and/or stirred in a suitable solvent to afford a slurry, which may be heated to promote dissolution. The term "slurry", as used herein, means a saturated solution of free base I or a salt thereof, which may also contain an additional amount of free base I or salt thereof to afford a heterogeneous mixture of free base I or salt thereof and a solvent at a given temperature. Suitable solvents in this regard include, for example, polar aprotic solvents, and polar protic solvents, and mixtures of two or more of these as disclosed herein.

Seed crystals may be added to any crystallization mixture to promote crystallization. As will be clear to the skilled artisan, seeding is used as a means of controlling growth of a particular crystalline form or as a means of controlling the particle size distribution of the crystalline product Accordingly, calculation of the amount of seeds needed depends on the size of the seed available and the desired size of an average product particle as described, for example, in "Programmed cooling of batch crystallizers," J.W. Mullin and J. Nyvlt, Chemical Engineering Science (1971) 26:369-377. In general, seeds of small size are needed to effectively control the growth of crystals in the batch. Seeds of small size may be generated by sieving, milling, or micronizing of larger crystals, or by micro-crystallization of solutions. Care should be taken that milling or micronizing of crystals does not result in any change in crystallinity from the desired crystal form (i.e. change to amorphous or to another polymorph).

A cooled mixture may be filtered under vacuum, and the isolated solids may be washed with a suitable solvent, such as cold recrystallization solvent, and dried under a nitrogen purge to afford the desired crystalline form. The isolated solids may be analyzed by a suitable spectroscopic or analytical technique, such as SSNMR, DSC, PXRD, or the like, to assure formation of the preferred crystalline form of the product. The resulting crystalline form is typically produced in an amount of greater than about 70 weight % isolated yield, but preferably greater than 90 weight % based on the weight of free base I originally employed in the crystallization procedure. The product may be comilled or passed through a mesh screen to delump the product, if necessary.

Crystalline forms may be prepared directly from the reaction medium of the final process step for preparing free base I. This may be achieved, for example, by employing in the final process step a solvent or mixture of solvents from which free base I may be crystallized. Alternatively, crystalline forms may be obtained by distillation or solvent addition techniques. Suitable solvents for this purpose include any of those solvents described herein, including protic polar solvents such as alcohols, and aprotic polar solvents such as ketones.

By way of general guidance, the reaction mixture may be filtered to remove any undesired impurities, inorganic salts, and the like, followed by washing with reaction or crystallization solvent. The resulting solution may be concentrated to remove excess solvent or gaseous constituents. If distillation is employed, the ultimate amount of distillate collected may vary, depending on process factors including, for example, vessel size, stirring capability, and the like. By way of general guidance, the reaction solution may be distilled to about {fraction (1/10)} the original volume before solvent replacement is carried out. The reaction may be sampled and assayed to determine the extent of the reaction and the wt % product in accordance with standard process techniques. If desired, additional reaction solvent may be added or removed to optimize reaction concentration. Preferably, the final concentration is adjusted to about 50 wt % at which point a slurry typically results.

It may be preferable to add solvents directly to the reaction vessel without distilling the reaction mixture. Preferred solvents for this purpose are those which may ultimately participate in the crystalline lattice as discussed above in connection with solvent exchange. Although the final concentration may vary depending on desired purity, recovery and the like, the final concentration of free base I in solution is preferably about 4% to about 7%. The reaction mixture may be stirred following solvent addition and simultaneously warmed. By way of illustration, the reaction mixture may be stirred for about 1 hour while warming to about 70°C. The reaction is preferably filtered hot and washed with either the reaction solvent, the solvent added or a combination thereof. Seed crystals may be added to any crystallization solution to initiate crystallization.

The various forms described herein may be distinguishable from one another through the use of various analytical techniques known to one of ordinary skill in the art. Such techniques include, but are not limited to, solid state nuclear magnetic resonance (SSNMR) spectroscopy, X-ray powder diffraction (PXRD), differential scanning calorimetry (DSC), and/or thermogravimetric analysis (TGA).

One of ordinary skill in the art will appreciate that an X-ray diffraction pattern may be obtained with a measurement error that is dependent upon the measurement conditions employed. In particular, it is generally known that intensities in a X-ray diffraction pattern may fluctuate depending upon measurement conditions employed and the shape or morphology of the crystal. It should be further understood that relative intensities may also vary depending upon experimental conditions and, accordingly, the exact order of intensity should not be taken into account. Additionally, a measurement error of diffraction angle for a conventional X-ray diffraction pattern is typically about 0.2% or less, preferably about 0.1% (as discussed hereinafter), and such degree of measurement error should be taken into account as pertaining to the aforementioned diffraction angles. Consequently, it is to be understood that the crystal forms of the instant invention are not limited to the crystal forms that provide X-ray diffraction patterns completely identical to the X-ray diffraction patterns depicted in the accompanying Figures disclosed herein. Any crystal forms that provide X- ray diffraction patterns substantially identical to those disclosed in the accompanying Figures fall within the scope of the present invention. The ability to ascertain substantial identities of X-ray diffraction patterns is within the purview of one of ordinary skill in the art.

In carrying out a preferred process for preparing Form N-1 crystals of the hydrochloric acid salt of the free base I, the free base I is suspended in an organic solvent which is preferably tetrahydrofuran (THF) although other organic solvents may be employed as well such as N,N-dimethylformamide (DMF), acetone, ethanol, DMF and acetone, or acetonitrile. The amount of free base I employed will be within the range from about 0.4 to about 1.2 g free base per 10 ml of organic solvent, preferably from about 0.5 to about 1.0 g free base per 10 ml of organic solvent. Where acetone is employed with DMF, the acetone will be used in volume ratio to DMF within the range from about 0.3:1 to about 1:1, preferably from about 0.4:1 to about 0.6:1.

Aqueous hydrochloric acid (from about 30 to about 40% by weight HCl, preferably from about 35 to about 38% by weight HCl) is added to the suspension of the free base I which preferably will turn into a clear solution. The hydrochloric acid will be present in a molar ratio (HCl) to free base within the range from about 1:1 to about 5:1, preferably from about 1.3:1 to about 2.2:1.

The resulting HCl salt solution will be seeded with seeds formed of crystals of Form N-1 hydrochloric acid salt of the free base I employing an amount of seeds in a molar ratio of Form N-1 crystals to starting free base I within the range from about 0.001:1 to about 0.2:1, preferably from about 0.01:1 to about 0.05:1. The solution will thereby form a slurry which is stirred for a period from about 5 to about 15 hours, preferably from about 5 to about 10 hours, filtered, washed with THF or other organic solvent as described above, and dried *in vacuo* to the Form N-1 crystals HCl salt of the free base I.

In carrying out the process for preparing seeds of Form N-1 crystals of the hydrochloric acid salt of the free base I, the free base I is suspended in an organic solvent which is preferably tetrahydrofuran (THF) although other organic solvents may be employed as well such as acetonitrile. The amount of free base I employed will be within the range from about 0.4 to about 1 g free base per 10 ml of organic solvent, preferably from about 0.5 to about 0.6 g free base per 10 ml of organic solvent.

Aqueous hydrochloric acid (from about 30 to about 40% by weight HCl, preferably from about 35 to about 38% by weight HCl) is added to the suspension of the free base I which preferably will turn into a clear solution. The hydrochloric acid will be present in a molar ratio (HCl) to free base within the range from about 1:1 to about 4:1, preferably from about 1.3:1 to about 2.8:1.

The suspension becomes clear and the resulting solution is stirred for a period from about 5 to about 15 hours, preferably from about 5 to about 10 hours at 20 to 25°C, filtered, washed with THE or other organic solvent as described above, and dried *in vacuo* to provide the seeds of Form N-1 crystals HCl salt of the free base I.

In carrying out a preferred process for preparing Form N-1 crystals of the methanesulfonic acid salt of the free base I, the free base I is dissolved in an organic solvent which is preferably N,N-dimethylformamide (DMF) although other organic solvents may be employed as well such as DMF/acetone, isopropyl alcohol (IPA), acetonitrile, THF, methylethyl ketone (MEK), MTBE, toluene or ethanol. The amount of free base I employed will be within the range from about 1 to about 5 g free base per 10 ml of organic solvent, preferably from about 1.1 to about 4 g free base per 10 ml of organic solvent.

Methanesulfonic acid is added to the solution of the free base I. The methanesulfonic acid will be present in a molar ratio to free base within the range from about 1:1 to about 2:1, preferably from about 1.1:1 to about 1.3:1.

Acetone or other organic solvent such as methyethyl ketone (MEK) is added to the resulting methanesulfonic acid salt solution so that the organic solvent will be in a volume ratio to DMF within the range from about 0.5:1 to about 2:1, preferably from about 1:1 to about 1.7:1. The resulting solution will be seeded with seeds formed of crystals of Form N-1 methanesulfonic acid salt of the free base I employing an amount of seeds of Form N-1 crystals in a molar ratio to starting free base I within the range from about 0.001:1 to about 0.2:1, preferably from about 0.01:1 to about 0.05:1. The solution will thereby form a slurry which is stirred for a period from about 5 to about 15 hours, preferably from about 5 to about 10 hours, filtered, washed with acetone or other organic solvent as described above and dried *in vacuo* to the Form N-1 crystals methanesulfonic acid salt of the free base I.

In carrying out a preferred process for preparing seeds of Form N-1 crystals of the methanesulfonic acid salt of the free base I, the free base I is suspended in an organic solvent which is preferably DMF/acetone or DMF, although other organic solvents may be employed as well such as ethyl acetate, acetonitrile, isopropyl alcohol or ethanol. The amount of free base I employed will be within the range from about 0.4 to about 0.8 g free base per 10 ml of organic solvent, preferably from about 0.5 to about 0.6 g free base per 10 ml of organic solvent.

Methanesulfonic acid is added to the suspension of the free base I. The methanesulfonic acid will be present in a molar ratio to free base within the range from about 1:1 to about 3:1, preferably from about 1.3:1 to about 2.0:1.

The suspension will become clear and the resulting solution will be stirred for a period from about 5 to about 15 hours, preferably from about 5 to about 10 hours at 20 to 25°C, filtered, washed with ethanol, isopropyl alcohol or other organic solvent as described above and dried *in vacuo* to the seeds of Form N-1 crystals methanesulfonic acid salt of the free base I.

In carrying out a preferred process for preparing Form N-4 crystals of the hydrochloric acid salt of the free base I (employing a reversed addition procedure using formic acid and acetone or formic acid and MEK), the free base I slurried in formic acid and acetone or formic acid and methylethylketone (MEK), is added to aqueous hydrochloric acid solution. The amount of free base I employed will be within the range from about 1 to about 5 g free base per 10 ml of formic acid-acetone or formic acid-MEK, preferably from about 1.8 to about 2.5 g free base per 10 ml of formic acid-acetone or formic acid-MEK.

The aqueous hydrochloric acid will contain from about 15 to about 40% by weight HCl, preferably from about 35 to about 38% by weight HCl.

The formic acid will be employed in a volume ratio to the acetone or MEK within the range from about 0.2:1 to about 1:1, preferably from about 0.35:1 to about 0.6:1.

The hydrochloric acid will be present in a molar ratio (HCl) to free base I within the range from about 1:1 to about 2.5:1, preferably from about 1.2:1 to about 1.6:1.

The reaction mixture preferably will turn into a clear solution which is filtered to remove insoluble solids.

The resulting HCl salt solution filtrate is added to slurry of seeds formed of crystals of Form N-4 hydrochloric acid salt of the free base I in acetone or MEK while stirring at a temperature within the range from about 10°C to about 20°C, employing an amount of seeds of Form N-4 crystals in a molar ratio to starting free base I within the range from about 0.0005:1 to about 0.2:1, preferably from about 0.005:1 to about 0.05:1. The mixture is stirred for a period from about 1 to about 72 hours, preferably from about 4 to about 18 hours, filtered, washed with acetone or MEK and dried *in vacuo* to provide the Form N-4 crystals of the HCl salt of the free base.

In carrying out a preferred process for preparing Form N-4 crystals of the hydrochloric acid salt of the free base I (employing normal addition procedure and formic acid-acetone or formic acid-MEK), the free base is stirred in formic acid-acetone or formic acid-MEK to which is added aqueous hydrochloric acid solution. The amount of free base employed will be within the range from about 1 to about 4 g free base per 10 ml of formic acid-acetone or formic acid-MEK, preferably from about 1.5 to about 2.5 g free base per 10 ml of formic acid-acetone or formic acid-MEK.

The aqueous hydrochloric acid will contain from about 15 to about 40% by weight HCl, preferably from about 35 to about 38% by weight HCl.

The formic acid will be employed in a volume ratio to the acetone or MEK within the range from about 0.2:1 to about 1:1, preferably from about 0.35:1 to about 0.6:1.

The hydrochloric acid will be present in a molar ratio (HCl) to free base within the range from about 1.1:1 to about 2.5:1, preferably from about 1.2:1 to about 1.6:1.

The reaction mixture preferably will turn into a clear solution which is filtered to remove insoluble solids.

To the resulting HCl salt solution are added seeds formed of crystals of Form N-4 hydrochloric acid salt of the free base I and acetone or MEK while stirring, employing an amount of seeds in a molar ratio to starting free base within the range from about 0.0005:1 to about 0.2:1, preferably from about 0.005:1 to about 0.05:1 and an amount of acetone or MEK in a volume ratio to acetone or MEK used to dissolve free base I within the range from about 15:1 to about 5:1, preferably from about 12:1 to about 10:1.

Alternatively, the solution of free base in formic acid and acetone or formic acid and MEK can be added to the pool of acetone (or MEK)/HCl/N-4 seeds mixture to effect precipitation of small crystals of Form N-4.

The mixture is stirred for a period from about 2 to about 72 hours, preferably from about 4 to about 16 hours at from about 10 to about 25°C, filtered, the filter cake washed with acetone or MEK and the filter cake dried *in vacuo* to the Form N-4 crystals HCl salt of the free base.

In carrying out a preferred process for preparing Form N-4 crystals of the hydrochloric acid salt of the free base I, employing a N,N-dimethyl acetamide (DMA)-acetone or DMA-MEK system, the free base is dissolved in DMA at a temperature within the range from about 50 to about 70°C, preferably from about 60 to about 65°C to which is added aqueous hydrochloric acid solution.

The aqueous hydrochloric acid will contain from about 30 to about 40% by weight HCl, preferably from about 35 to about 38% by weight HCl.

To the resulting HCl-free base I solution is added seeds formed of crystals of Form N-4 hydrochloric acid salt of the free base I and acetone or MEK. The seeds of Form N-4 crystals will be employed in a molar ratio to starting free base within the range from about 0.001:1 to about 0.2:1, preferably from about 0.01:1 to about 0.05:1.

Alternatively, the solution of the free base I can be added to a pool of acetone (or MEK)/HCl/N-4 seeds to effect precipitation of small crystals of Form N-4.

The DMA is employed in a volume ratio to the acetone or MEK within the range from about 0.1:1 to about 0.3:1, preferably from about 0.15:1 to about 0.25:1.

The resulting mixture is stirred for a period from about 5 to about 15 hours, preferably from about 5 to about 6 hours at from about 10 to about 25°C, filtered and the wet cake dried *in vacuo* to the Form N-4 HCl salt of the free base I.

The amount of free base employed will be within the range from about 1 to about 4 g free base per 10 ml of DMA or DMA, preferably from about 2 to about 3 g free base per 10 ml of DMA-acetone or DMA-MEK.

The hydrochloric acid will be present in a molar ratio (HCl) to free base within the range from about 1:1 to about 1.8:1, preferably from about 1.2:1 to about 1.6:1.

In carrying out a preferred process for preparing Form N-4 crystals of the hydrochloric acid salt of the free base I, employing a DMF-acetone or DMF-MEK system, the free base I is suspended in DMF and aqueous hydrochloric acid solution is added to the resulting slurry. The amount of free base employed will be within the range from about 1 to about 5 g free base per 10 ml of DMF, preferably from about 1.5 to about 2.5 g free base per 10 ml of DMF.

The aqueous hydrochloric acid will contain from about 15 to about 40% by weight HCl, preferably from about 35 to about 38% by weight HCl.

The hydrochloric acid will be present in a molar ratio (HCl) to free base within the range from about 1:1 to about 3:1, preferably from about 1.1:1 to about 2.2:1.

The reaction mixture preferably will turn into a clear solution. To the resulting HCl salt solution is added acetone or MEK and the solution will be seeded with seeds formed of crystals of Form N-4 hydrochloric acid salt of the free base in acetone, or seeds of Form N-4 hydrochloric acid salt of the free base as a slurry in acetone are added to the HCl solution, employing an amount of seeds in a molar ratio to starting free base within the range from about 0.0005:1 to about 0.2:1, preferably from about 0.005:1 to about 0.05:1. The solution will thereby form a slurry which is stirred for a period from about 1 to about 72 hours, preferably from about 4 to about 16 hours at from about 10 to about 25°C, filtered, washed with acetone or MEK and the wet cake dried *in vacuo* at from about 40 to about 45°C to the Form N-4 crystals HCl salt of the free base.

The DMF will be employed in a volume ratio to the acetone or MEK within the range from about 1:1 to about 5:1, preferably from about 1.5:1 to about 2:1.

In carrying out a preferred process for preparing Form N-4 crystals of the hydrochloric acid salt of the free base I (employing normal addition procedure and ethanol or acetone or THF), the free base is suspended in an organic solvent which is ethanol, acetone or THF to which is added aqueous hydrochloric acid solution. The amount of free base employed will be within the range from about 0.5 to about 2 g free base per 10 ml of organic solvent, preferably from about 0.6 to about 1.2 g free base per 10 ml of organic solvent.

The aqueous hydrochloric acid will contain from about 30 to about 40% by weight HCl, preferably from about 35 to about 38% by weight HCl.

The hydrochloric acid will be present in a molar ratio (HCl) to free base within the range from about 1:1 to about 4:1, preferably from about 1.2:1 to about 2.5:1.

The reaction mixture preferably will turn into a clear solution which is filtered to remove insoluble solids.

To the resulting HCl salt solution are added seeds formed of crystals of Form N-4 hydrochloric acid salt of the free base I while stirring, employing an amount of seeds in a molar ratio to starting free base within the range from about 0.001:1 to about 0.2:1, preferably from about 0.01:1 to about 0.05:1. The mixture is stirred for a period from about 15 to about 200 hours, preferably from about 15 to about 80 hours, filtered, the filter cake washed with ethanol, acetone or THF and the filter cake dried *in vacuo* to the Form N-4 crystals HCl salt of the free base.

In carrying out a preferred process for preparing Form N-4 crystals of the hydrochloric acid salt of the free base I, employing Form N-1 crystals, the hydrochloric acid salt of the free base I in Form N-1 is slurried in an organic solvent which is acetonitrile, THF, ethanol or acetone at a temperature within the range from about 30 to about 50°C, preferably from about 35 to about 45°C.

To the slurry is added seeds of crystals of Form N-4 hydrochloric acid salt of the free base I. The seeds of Form N-4 crystals will be employed in a molar ratio to starting free base within the range from about 0.001:1 to about 0.2:1, preferably from about 0.01:1 to about 0.05:1.

The resulting slurry is stirred for a period from about 90 to about 120 hours, preferably from about 90 to about 100 hours at from about 25 to about 45°C, filtered and wet cake washed with THF or acetone and dried *in vacuo* to the Form N-4 HCl salt of the free base I.

The amount of free base employed will be within the range from about 0.5 to about 2 g free base per 10 ml of organic solvent, preferably from about 1 to about 1.5 g free base per 10 ml of organic solvent.

In carrying out a preferred process for preparing seeds of Form N-4 crystals of the hydrochloric acid salt of the free base I, the free base I is suspended in an organic solvent which is preferably absolute ethanol. The amount of free base I employed will be within the range from about 0.4 to about 0.7 g free base per 10 ml of organic solvent, preferably from about 0.5 to about 0.6 g free base per 10 ml of organic solvent.

Aqueous hydrochloric acid (from about 30 to about 40% by weight HCl, preferably from about 35 to about 38% by weight HCl) is added to the suspension of the free base I which preferably will turn into a clear solution. The hydrochloric acid will be present in a molar ratio (HCl) to free base within the range from about 1:1 to about 2:1, preferably from about 1.1:1 to about 1.5:1.

The suspension becomes clear and the resulting solution is stirred for a period from about 20 to about 40 hours, preferably from about 20 to about 24 hours at from about 20 to about 25°C, filtered, washed with ethanol or other organic solvent as described above, and dried *in vacuo* to the seeds of Form N-4 crystals HCl salt of the free base L

The preferred solvent system employed in the process of the invention for preparing Form N-4 crystals is the formic acid/MEK process, preferably employing reverse addition.

Use of the preferred embodiment enables formation of Form N-4 crystals of desired particle size (D90 < 30 µm) and suitable flow properties to facilitate manufacturing.

It has been found that the desirable Form N-4 crystals of the hydrochloric acid salt of the free base can be consistently obtained in the DMF/acetone, DMA/acetone, formic acid/acetone and formic acid/MEK solvent systems. However, the various processes of the invention can be employed to selectively form either Form N-1 crystals or Form N-4 crystals depending on the procedure and the solvent system employed.

The free base I (also referred to as the amide I) of the structure 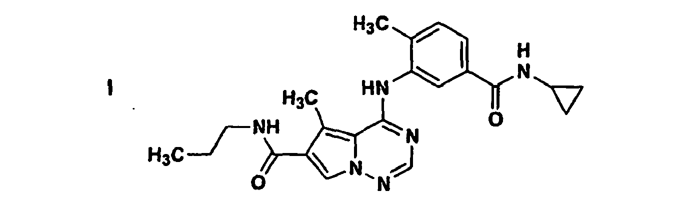 may be prepared employing the following reaction scheme

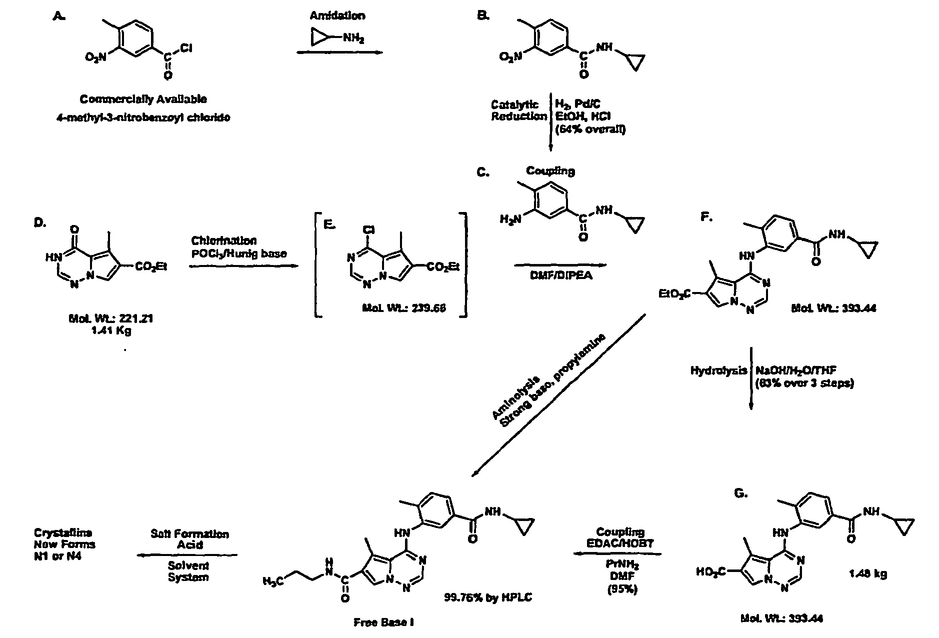

The intermediate C is prepared by the amidation of 4-methyl-3-nitrobenzyl chloride with cyclopropylamine followed by catalytic reduction and hydrogen chloride salt formation to obtain the intermediate C.

The preparation of intermediate E starts with condensation of ethyl acetoacetate with dimethylformamide dimethylacetal then glycine ethyl ester hydrochloride to obtain intermediate E¹

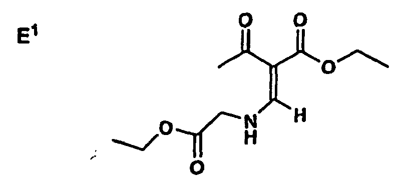

Base promoted cyclization of intermediate E¹ yields the pyrrole E²

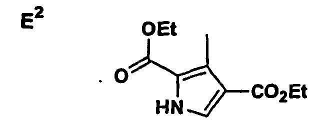

Conversion of pyrrole E² to the 1-amino pyrrole E³ 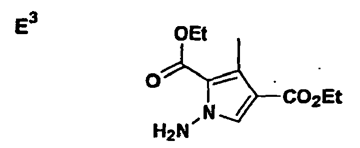 is followed by condensation of E³ with formamide and acid catalyzed cyclization yielding intermediate D. Chlorination of D yields the intermediate E.

Coupling intermediate C with intermediate E yields intermediate F which is hydrolyzed to give intermediate G. Coupling G with propylamine provides free base I, which is subjected to salt formation yielding the desired salt which is the form of Form N-4 or N-1 crystals.

A full disclosure of the above process is disclosed in U.S. application Serial No. 10/420,399 filed April 22, 2003 which is incorporated herein by reference.

An alternative embodiment for the desired aminolysis of ester F to free base I includes the steps of treating the ester F with n-propyl amine and trimethylaluminum while maintaining the reaction at a temperature within the range from about 55 to about 60°C to form the free base I.

Yet another alternative embodiment for the direct aminolysis of ester F to amide I includes the steps of treating the ester F with an n-propyl amine (in the presence of 2,2,2-trifluoroethanol) and n-butyllithium while maintaining the reaction at a temperature within the range from about 80 to about 90°C, to form the free base L

In an alternative embodiment, ester F may be subjected to direct aminolysis by reacting ester F with a strong base and n-propylamine to form free base I.

The above direct aminolysis reaction may be carried out by treating the ester F with n-propyl amine and an alkyllithium, preferably n-butyllithium, to form the free base I.

### UTILITY

The novel salt forms (N-1 and N-4) of the invention (including the Forms N-1 and N-4 of the hydrochloride salt, and the Form N-1 of the methanesulfonic acid salt) are selective inhibitors of p3 8 kinase activity, and in particular, isoforms p38α and p38β. Accordingly, the novel salt forms of the invention have utility in treating conditions associated with p38 kinase activity. Such conditions include diseases in which cytokine levels are modulated as a consequence of intracellular signaling via p38, and in particular, diseases that are associated with an overproduction of cytokines IL-1, IL-4, IL-8, and TNF-α. As used herein, the terms "treating" or "treatment" encompass either or both responsive and prophylaxis measures, *e*.*g*., measures designed to inhibit or delay the onset of the disease or disorder, achieve a full or partial reduction of the symptoms or disease state, and/or to alleviate, ameliorate, lessen, or cure the disease or disorder and/or its symptoms. When reference is made herein to inhibition of "p-38α/β kinase," this means that either p38α and/or p38β kinase are inhibited. Thus, reference to an IC₅₀ value for inhibiting p-38α/β kinase means that the compound has such effectiveness for inhibiting at least one of, or both of, p38α and p38β kinases.

In view of their activity as inhibitors of p-38α/β kinase, the novel salt forms of the invention are useful in treating p-38 associated conditions including, but not limited to, inflammatory diseases, autoimmune diseases, destructive bone disorders, proliferative disorders, angiogenic disorders, infectious diseases, neurodegenerative diseases, and viral diseases.

More particularly, the specific conditions or diseases that may be treated with the novel salt forms of the invention include, without limitation, pancreatitis (acute or chronic), asthma, allergies, adult respiratory distress syndrome, chronic obstructive pulmonary disease, glomerulonephritis, rheumatoid arthritis, systemic lupus erythematosis, scleroderma, chronic thyroiditis, Graves' disease, autoimmune gastritis, diabetes, autoimmune hemolytic anemia, autoimmune neutropenia, thrombocytopenia, atopic dermatitis, chronic active hepatitis, myasthenia gravis, multiple sclerosis, inflammatory bowel disease, ulcerative colitis, Crohn's disease, psoriasis, graft vs. host disease, inflammatory reaction induced by endotoxin, tuberculosis, atherosclerosis, muscle degeneration, cachexia, psoriatic arthritis, Reiter's syndrome, gout, traumatic arthritis, rubella arthritis, acute synovitis, pancreatic β-cell disease; diseases characterized by massive neutrophil infiltration; rheumatoid spondylitis, gouty arthritis and other arthritic conditions, cerebral malaria, chronic pulmonary inflammatory disease, silicosis, pulmonary sarcoisosis, bone resorption disease, allograft rejections, fever and myalgias due to infection, cachexia secondary to infection, meloid formation, scar tissue formation, ulcerative colitis, pyresis, influenza, osteoporosis, osteoarthritis and multiple myeloma-related bone disorder, acute myelogenous leukemia, chronic myelogenous leukemia, metastatic melanoma, Kaposi's sarcoma, multiple myeloma, sepsis, septic shock, and Shigellosis; Alzheimer's disease, Parkinson's disease, cerebral ischemias or neurodegenerative disease caused by traumatic injury; angiogenic disorders including solid tumors, ocular neovasculization, and infantile haemangiomas; viral diseases including acute hepatitis infection (including hepatitis A, hepatitis B and hepatitis C), HIV infection and CMV retinitis, AIDS, ARC or malignancy, and herpes; stroke, myocardial ischemia, ischemia in stroke heart attacks, organ hyposia, vascular hyperplasia, cardiac and renal reperfusion injury, thrombosis, cardiac hypertrophy, thrombin-induced platelet aggregation, endotoxemia and/or toxic shock syndrome, and conditions associated with prostaglandin endoperoxidase syndase-2.

In addition, the novel salt p38 inhibitors of this invention inhibit the expression of inducible pro-inflammatory proteins such as prostaglandin endoperoxide synthase-2 (PGHS-2), also referred to as cyclooxygenase-2 (COX-2). Accordingly, additional p38-associated conditions include edema, analgesia, fever and pain, such as neuromuscular pain, headache, pain caused by cancer, dental pain and arthritis pain. The inventive salt forms also may be used to treat veterinary viral infections, such as lentivirus infections, including, but not limited to equine infectious anemia virus; or retro virus infections, including feline immunodeficiency virus, bovine immunodeficiency virus, and canine immunodeficiency virus.

When the terms "p38 associated condition" or "p38 associated disease or disorder" are used herein, each is intended to encompass all of the conditions identified above as if repeated at length, as well as any other condition that is affected by p38 kinase activity.

The present invention thus provides methods for treating such conditions, comprising administering to a subject in need thereof an effective amount of at least one novel salt form of the invention. The methods of treating p38 kinase-associated conditions may comprise administering novel salt forms of the invention alone or in combination with each other and/or other suitable therapeutic agents useful in treating such conditions. Exemplary of such other therapeutic agents include corticosteroids, rolipram, calphostin, CSAIDs, 4-substituted imidazo [1,2-A]quinoxalines as disclosed in US Pat. No. 4,200,750; Interleukin-10, glucocorticoids, salicylates, nitric oxide, and other immunosuppressants; nuclear translocation inhibitors, such as deoxyspergualin (DSG); non-steroidal anti-inflammatory drugs (NSAIDs) such as ibuprofen, celecoxib and rofecoxib; steroids such as prednisone or dexamethasone; antiviral agents such as abacavir; antiproliferative agents such as methotrexate, leflunomide, FK506 (tacrolimus, Prograf); cytotoxic drugs such as azathiprine and cyclophosphamide; TNF-α inhibitors such as tenidap, anti-TNF antibodies or soluble TNF receptor, and rapamycin (sirolimus or Rapamune) or derivatives thereof.

The above other therapeutic agents, when employed in combination with the novel salt forms of the present invention, may be used, for example, in those amounts indicated in the Physicians' Desk Reference (PDR) or as otherwise determined by one of ordinary skill in the art. In the methods of the present invention, such other therapeutic agent(s) may be administered prior to, simultaneously with, or following the administration of the inventive compounds.

The present invention also provides pharmaceutical compositions containing novel salt forms of the invention capable of treating p38-kinase associated conditions, including TNF-α, IL-1, and/or IL-8 mediated conditions, as described above. The inventive compositions may optionally contain other therapeutic agents as described above, and may be formulated, for example, by employing conventional solid or liquid vehicles or diluents, as well as pharmaceutical additives of a type appropriate to the mode of desired administration (*e*.*g*., excipients, binders, preservatives, stabilizers, flavors, etc.) according to techniques such as those well known in the art of pharmaceutical formulation.

The novel salt forms of the invention may be administered by any means suitable for the condition to be treated, which may depend on the need for site-specific treatment or quantity of drug to be delivered. Topical administration is generally preferred for skin-related diseases, and systematic treatment preferred for cancerous or pre-cancerous conditions, although other modes of delivery are contemplated. For example, the compounds may be delivered orally, such as in the form of tablets, capsules, granules, powders, or liquid formulations including syrups; topically, such as in the form of solutions, suspensions, gels or ointments; sublingually; bucally, parenterally, such as by subcutaneous, intravenous, intramuscular or intrasternal injection or infusion techniques (*e*.*g*., as sterile injectable aq. or non-aq. solutions or suspensions); nasally such as by inhalation spray; topically, such as in the form of a cream or ointment; rectally such as in the form of suppositories; or liposomally. Dosage unit formulations containing non-toxic, pharmaceutically acceptable vehicles or diluents may be administered. The compounds may be administered in a form suitable for immediate release or extended release. Immediate release or extended release may be achieved with suitable pharmaceutical compositions or, particularly in the case of extended release, with devices such as subcutaneous implants or osmotic pumps.

Tablets are preferred. Most preferred are tablets containing the Form N-4 hydrochloride salt of the free base I.

Exemplary compositions for topical administration include a topical carrier such as PLASTIBASE^{®} (mineral oil gelled with polyethylene).

Exemplary compositions for oral administration include suspensions which may contain, for example, microcrystalline cellulose for imparting bulk, alginic acid or sodium alginate as a suspending agent, methylcellulose as a viscosity enhancer, and sweeteners or flavoring agents such as those known in the art; and immediate release tablets which may contain, for example, microcrystalline cellulose, dicalcium phosphate, starch, magnesium stearate and/or lactose and/or other excipients, binders, extenders, disintegrants, diluents and lubricants such as those known in the art. The inventive compounds may also be orally delivered by sublingual and/or buccal administration, *e.g.*, with molded, compressed, or freeze-dried tablets. Exemplary compositions may include fast-dissolving diluents such as mannitol, lactose, sucrose, and/or cyclodextrins. Also included in such formulations may be high molecular weight excipients such as celluloses (AVICEL^{®}) or polyethylene glycols (PEG); an excipient to aid mucosal adhesion such as hydroxypropyl cellulose (HPC), hydroxypropyl methyl cellulose (HPMC), sodium carboxymethyl cellulose (SCMC), and/or maleic anhydride copolymer (*e*.*g*., GANTREZ^{®}); and agents to control release such as polyacrylic copolymer (*e*.*g*., CARBOPOL 934^{®}). Lubricants, glidants, flavors, coloring agents and stabilizers may also be added for ease of fabrication and use.

Exemplary compositions for nasal aerosol or inhalation administration include solutions which may contain, for example, benzyl alcohol or other suitable preservatives, absorption promoters to enhance absorption and/or bioavailability, and/or other solubilizing or dispersing agents such as those known in the art.

Exemplary compositions for parenteral administration include injectable solutions or suspensions which may contain, for example, suitable non-toxic, parenterally acceptable diluents or solvents, such as mannitol, 1,3-butanediol, water, Ringer's solution, an isotonic sodium chloride solution, or other suitable dispersing or wetting and suspending agents, including synthetic mono- or diglycerides, and fatty acids, including oleic acid.

Exemplary compositions for rectal administration include suppositories which may contain, for example, suitable non-irritating excipients, such as cocoa butter, synthetic glyceride esters or polyethylene glycols, which are solid at ordinary temperatures but liquefy and/or dissolve in the rectal cavity to release the drug.

The effective amount of a novel salt form of the present invention may be determined by one of ordinary skill in the art, and includes exemplary dosage amounts for a mammal of from about 0.05 to 100 mg/kg of body weight of active compound per day, which may be administered in a single dose or in the form of individual divided doses, such as from 1 to 4 times per day. It will be understood that the specific dose level and frequency of dosage for any particular subject may be varied and will depend upon a variety of factors, including the activity of the specific compound employed, the metabolic stability and length of action of that compound, the species, age, body weight, general health, sex and diet of the subject, the mode and time of administration, rate of excretion, drug combination, and severity of the particular condition. Preferred subjects for treatment include animals, most preferably mammalian species such as humans, and domestic animals such as dogs, cats, horses, and the like. Thus, when the term "patient' is used herein, this term is intended to include all subjects, most preferably mammalian species, that are affected by mediation of p38 enzyme levels.

The novel salt forms of the invention, including the compounds described in the examples hereof, have been tested in one or more of the assays described below and have shown activity as inhibitors of p38α/β enzymes and TNF-α.

### BIOLOGICAL ASSAYS

### Generation of p38 Kinases

cDNAs of human p38α, β and γ isozymes were cloned by PCR. These cDNAs were subcloned in the pGEX expression vector (Pharmacia). GST-p38 fusion protein was expressed in E. Coli and purified from bacterial pellets by affinity chromatography using glutathione agarose. p38 fusion protein was activated by incubating with constitutively active MKK6. Active p38 was separated from MKK6 by affinity chromatography. Constitutively active MKK6 was generated according to Raingeaud et al. [Mol. Cell. Biol., 1247-1255 (1996)].

### TNF-α Production by LPS-Stimulated PBMCs

Heparinized human whole blood was obtained from healthy volunteers. Peripheral blood mononuclear cells (PBMCs) were purified from human whole blood by Ficoll-Hypaque density gradient centrifugation and resuspended at a concentration of 5 x 10⁶/ml in assay medium (RPMI medium containing 10% fetal bovine serum). 50 ul of cell suspension was incubated with 50 ul of test compound (4X concentration in assay medium containing 0.2% DMSO) in 96-well tissue culture plates for 5 minutes at RT. 100 µl of LPS (200 ng/ml stock) was then added to the cell suspension and the plate was incubated for 6 hours at 37°C. Following incubation, the culture medium was collected and stored at -20°C. TNF-α concentration in the medium was quantified using a standard ELISA kit (Pharmingen-San Diego, CA). Concentrations of TNF-α and IC₅₀ values for test compounds (concentration of compound that inhibited LPS-stimulated TNF-α production by 50%) were calculated by linear regression analysis.

### p38 Assay

The assays were performed in V-bottomed 96-well plates. The final assay volume was 60 µl prepared from three 20 µl additions of enzyme, substrates (MBP and ATP) and test compounds in assay buffer (50 mM Tris pH 7.5, 10 mM MgCl₂, 50 mM NaCl and 1 mM DTT). Bacterially expressed, activated p38 was pre-incubated with test compounds for 10 min. prior to initiation of reaction with substrates. The reaction was incubated at 25°C for 45 min. and terminated by adding 5 µl of 0.5 M EDTA to each sample. The reaction mixture was aspirated onto a pre-wet filtermat using a Skatron Micro96 Cell Harvester (Skatron, Inc.), then washed with PBS. The filtermat was then dried in a microwave oven for 1 min., treated with MeltilLex A scintillation wax (Wallac), and counted on a Microbeta scintillation counter Model 1450 (Wallac). Inhibition data were analyzed by nonlinear least-squares regression using Prizm (GraphPadSoftware). The final concentration of reagents in the assays are ATP, 1 µM; [γ-³³P]ATP, 3 nM; MBP (Sigma, #M1891), 2µg/well; p38, 10 nM; and DMSO, 0.3%.

### TNF-α Production by LPS-Stimulated Mice

Mice (Balb/c female, 6-8 weeks of age, Harlan Labs; n=8/treatment group) were injected intraperitoneally with 50ug/kg lipopolysaccharide (LPS; *E coli* strain 0111:B4, Sigma) suspended in sterile saline. Ninety minutes later, mice were sedated by CO₂:O₂ inhalation and a blood sample was obtained. Serum was separated and analyzed for TNF-alpha concentrations by commercial ELISA assay per the manufacturer's instructions (R&D Systems, Minneapolis, MN).

Test compounds were administered orally at various times before LPS injection. The compounds were dosed either as suspensions or as solutions in various vehicles or solubilizing agents.

### ABBREVIATIONS

For ease of reference, the following abbreviations are employed herein, including the methods of preparation and Examples that follow:
Ph = phenyl
Bz = benzyl
t-Bu = tertiary butyl
Me = methyl
Et = ethyl
Pr = propyl
Iso-P = isopropyl
MeOH = methanol
EtOH = ethanol
EtOAc = ethyl acetate
Boc = tert-butyloxycarbonyl
DCM = dichloromethane
DCE = 1,2-dichloroethane
DMA = N,N-dimethyl acetamide
DMF = N,N-dimethyl formamide
DMSO = dimethyl sulfoxide
DTT = dithiothreitol
TFA = trifluoroacetic acid
THF = tetrahydrofuran
HATU = *O*-(7-Azabenzotriazol-1-yl-*N,N,N',N*'-tetramethyluronim hexafluorophosphate
KOH = potassium hydroxide
K₂CO₃ = potassium carbonate
POCl₃ =phosphorous oxychloride
EDC or EDCI = 1-(3-Dimethylaminopropyl)-3-ethylcarbodiimide hydrochloride
DIPEA = diisopropylethylamine
HOBt= 1-hydroxybenzotriazole hydrate
m-CPBA = m-chloroperbenzoic acid
NaH = sodium hydride
NaOH = sodium hydroxide
Pd = palladium
Pd/C = palladium on carbon
min = minute(s)
µL = microliter
ng = nanogram
µM = micromolar
nM = nanomolar
mM = millimolar
L = liter
ml or mL = milliliter
µL or µl = microliter
g = gram(s)
mg = milligram(s)
mol = moles
mmol = millimole(s)
meq = milliequivalent
RT or rt = room temperature (20 to 25°C)
ret. t. = HPLC retention time (minutes)
sat or sat'd = saturated
aq. = aqueous
TLC = thin layer chromatography
HPLC = high performance liquid chromatography
RP HPLC = reverse phase HPLC
LC/MS = high performance liquid chromatography/mass spectrometry
MS = mass spectrometry
NMR = nuclear magnetic resonance
mp = melting point
RH = relative humidity

In the Examples, designations associated with HPLC data reflect the following conditions:
a. Column: YMC ODSA S-5 5u C18 4.6 x 50 mm; Solvent: solvent A = 10% MeOH/90% water/0.1% THF, and solvent B = 90% MeOH/10%water/0.1% THF; Method: 4 min gradient;
b. Column: YMC s5 ODS 4.6 x 50 mm; Solvent: solvent A = 10% MeOH/90% water/0.2% H₃PO₄, and solvent B = 90% MeOH/10% water/0.2% H₃PO₄; Method: 4 min gradient.

### EXAMPLES

The invention will now be further described by the following working examples, which are preferred embodiments of the invention. HPLC purifications were done on C18 reverse phase (RP) columns using water MeOH mixtures and TFA as buffer solution. These examples are illustrative rather than limiting. There may be other embodiments that fall within the spirit and scope of the invention as defined by the appended claims.

### EXAMPLE 1

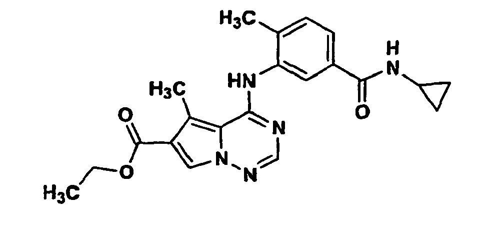

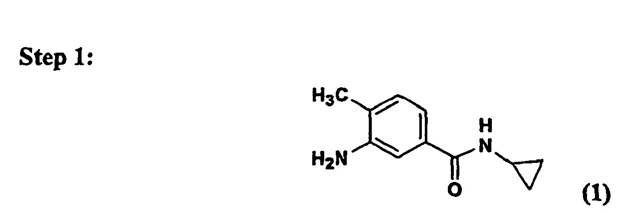

To a solution of 3-amino-4-methylbenzoic acid (5.12 g, 33.9 mmol, 1.0 eq.), EDC (9.97 g, 52.0 mol, 1.5 eq.) and 4-(dimethylamino)pyridine (0.89 g, 7.3 mol, 0.2 eq.) in DMF (100 mL) at 0°C was added cyclopropylamine (4.0 mL, 57.7 mol, 1.7 eq.) dropwise. After stirring for 15 min., the cold bath was removed, and the reaction mixture was stirred at rt overnight. Volatiles were removed at 50°C under reduced pressure. The residue was diluted with water and extracted with DCM (3x). The organic layers were combined, dried over sodium sulfate, and concentrated *in vacuo* to give an oil. Silica gel chromatography using DCM:MeOH (20:1) afforded compound (1) as a yellow oil (6.98 g, 108 % yield). HPLC Ret. t. = 0.637 min.; LC/MS (M+H)⁺ = 191.09⁺.

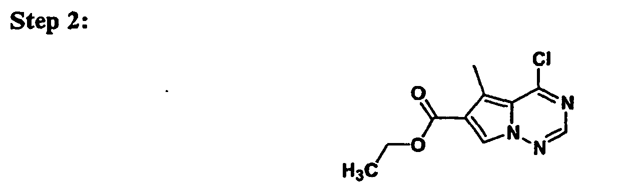

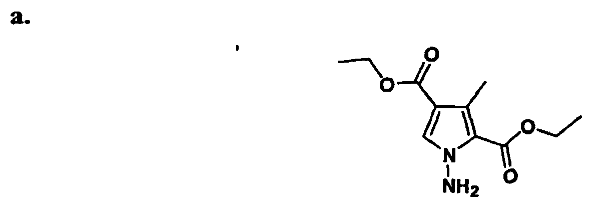

To a solution of the 3-methyl-1-pyrrole-2,4-diethyl ester (100 mg) (J. Heterocyclic Chem., Vol. 34 (1997), at pp. 177-193; Heterocycles, Vol. 50 (1999), at pp. 853-866; Synthesis (1999), at pp. 479-482; generally, the synthesis of pyrroles is described by the commonly assigned patent documents referenced herein and the procedure of M. Suzuki, M. Miyoshi, and K. Matsumoto, J. Org. Chem. 1974, 39 (1980)) in DMF (0.44M) was added either NaH or KOtBu (1.2 equiv) at rt. This solution was stirred for 30-45 minutes. Chloramine in ether (ca. 0.15M, 1 eq.) was added via syringe. The solution was stirred for 1.5 h or until starting material was converted to product as judged by HPLC analysis. The reaction was then quenched with aq. Na₂S₂O₃ and extracted with EtOAc or Et₂O. The organic extracts were washed with water and brine and then dried over sodium sulfate. Compound a. was obtained in >90% yield. NH₂Cl in ether was prepared according to the procedure of Nunn, J. Chem. Soc. (C), (1971) at p. 823.

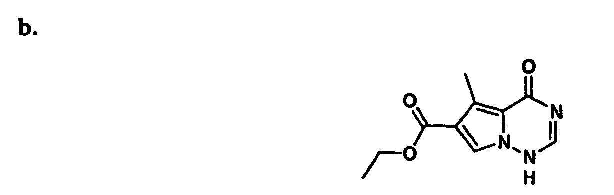

To a solution of compound a. (2 g) in formamide (8 mL) was added acetic acid (20% by weight), and the mixture was heated at 120°C for 24h. The reaction mixture was cooled and water added (32 mL) to precipitate the product. The solids were collected by filtration and washed with EtOAc to furnish to the compound b. as a yellow solid (90%).

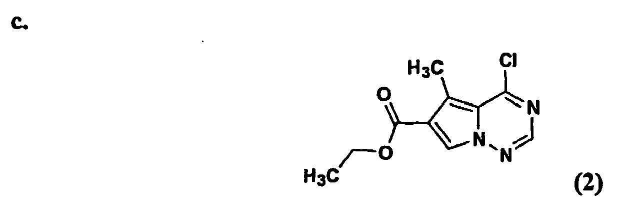

To a suspension of compound b. oxopyrrolotriazine (3.00 g, 13.6 mmol) in toluene (45 mL) was added dropwise phosphorus oxychloride (1.90 mL, 20.4 mmol) and *N,N*-DIPEA (2.37 mL, 13.6 mmol) successively at rt. The resulting mixture was heated at reflux for 36h, allowed to cool to rt, and then poured into an ice-cold mixture of sat'd sodium bicarbonate solution (150 mL) and toluene (60 mL). The organic layer was separated and the aqueous layer extracted with toluene (3 x 50 mL). The combined extract was washed with sat'd sodium bicarbonate solution and brine and dried over anhydrous MgSO₄. Evaporation of solvent *in vacuo* afforded compound c. (3.26 g, 100% yield) as a yellow solid.

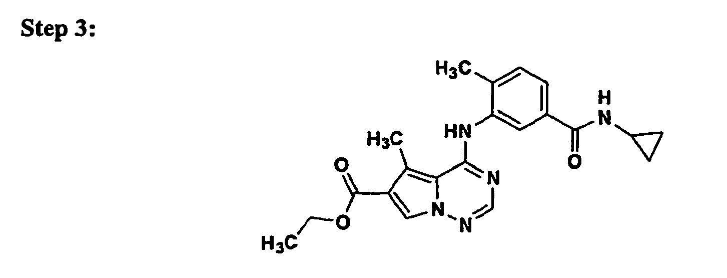

A solution of products of Step 1 (1.60 g, 8.40 mmol, 1.6 eq.) and Step 2 (1.30 g, 5.40 mmol, 1.0 eq.) in DMF (13 mL) was stirred at rt overnight. Water was added and the precipitate collected by filtration, washed with water, and dried. Trituration with diethyl ether afforded Example 1 title compound (1.70 g, 80% yield) as an off-white solid. HPLC Ret. t. = 3.190 min.; LC/MS (M+H)⁺ = 394.31⁺.

### EXAMPLE 1A

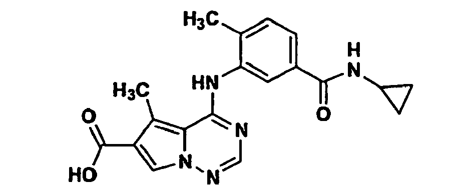

A solution of title Example 1 compound (0.86 g, 2.20 mmol, 1.0 eq.) in THF (4.0 mL) and 1 *N* aqueous NaOH (9.0 mL, 4.1 eq.) was stirred at 60°C overnight. After cooling to rt, the reaction mixture was concentrated *in vacuo* but not to dryness. To the solution at 0°C was added 1 *N* aqueous hydrochloric acid until it was acidic and the precipitate was collected and dried to afford crude title Example 1A compound (0.51 g, 64.0 % yield). HPLC Ret. t. = 2.400 min.; LC/MS (M+H) ⁺ = 366.06⁺. The filtrate was then extracted with EtOAc (3x) and the organic layers were combined, dried over sodium sulfate, and concentrated *in vacuo* to give Example 1A compound (0.035 g, 4.4 % yield) which is an intermediate compound in preparation of the free base I.

### EXAMPLE 2

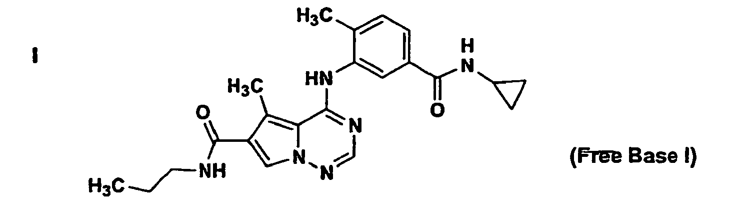

A solution of title Example 1A compound (0.026 g, 0.071 mmol, 1.0 eq.), EDC (0.021 g, 0.11 mmol, 1.5 eq.), HOBt (0.015 g, 0.11 mmol, 1.5 eq), *n-*propylamine (0.015 mL, 0.15 mmol, 2.1 eq.) and DIPEA (0.040 mL, 0.23 mmol, 3.2 eq.) in DMF (0.20 mL) was shaken at rt overnight. Water (1 mL) was added and the precipitate collected by filtration, washed with water, and dried to give title Example 2 product (0.021 g, 70% yield); HPLC Ret. t. = 2.883 min.; LC/MS (M+H) ⁺ = 421.18 ⁺. The product obtained was the crystalline free base I.

### EXAMPLE 2A

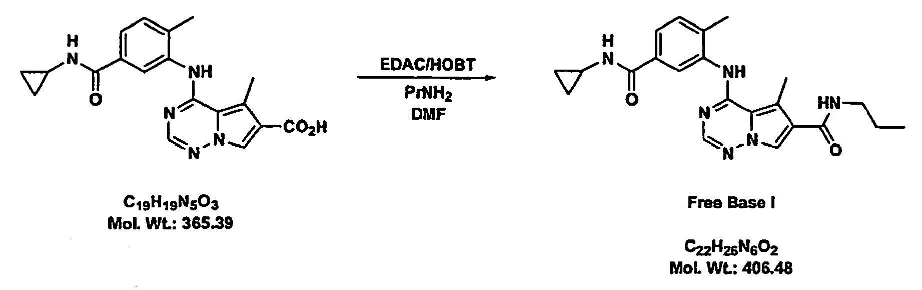

20.0 grams of the above starting acid (which may be prepared as set out in Example 1A) (0.055 mole, 1 equiv., MW 365.39), 12.0 g 1-[3-dimethylamino)propyl]-3-ethylcarbodiimide hydrochloride (EDAC) (0.063 mole, 1.19 equiv., MW 191.71), 8.9 g 1-hydroxybenzotriazole hydrate (HOBT) (0.058 mole, 1.06 equiv., MW 153.16) and 120 mL N,N-dimethylformamide (DMF) (6 mL/g) are charged to a round bottom flask. The slurry is stirred at 18-23°C and will slowly become a cloudy solution. The reaction mixture is stirred at 18-23°C until the starting acid is less than or equal to 0.6% (relative area percent by HPLC). A solution of 4.8 g n-propylamine (0.081 mole, 1.48 equiv., MW 59.11) and 80 mL DMF (4 mL/g) are then added to the reaction while maintaining the reaction temperature below 35°C. After the propylamine addition is complete the reaction is stirred at 30-40°C until the HOBT derivative is less than or equal to 0.2% (relative area percent by HPLC). The reaction mixture is then polish filtered. 35 mL of purified water (1.75 mL/g) are then added to the resulting free base I rich reaction mixture while maintaining a temperature of 30-40°C. The resulting slurry is stirred at 30-40°C for 1-2 hours. 205 mL of purified water (10.25 mL/g) are then added over a period of 1 hour. The slurry is stirred at 30-40°C for 1 hour and then cooled to 0-5°C. The slurry is stirred at 0-5°C until the filtered mother liquor shows a concentration of free base I less than or equal to 2 mg/mL. The slurry is then filtered and washed with 200 mL purified water (10 mL/g) followed by 76 mL acetone (3.79 mL/g). The wet solids are dried at 40-45°C until LOD is less than or equal to 1%. Free base I is isolated to yield typically 89-95 M% with purity greater than 99% (area percent by HPLC).

### EXAMPLE 3

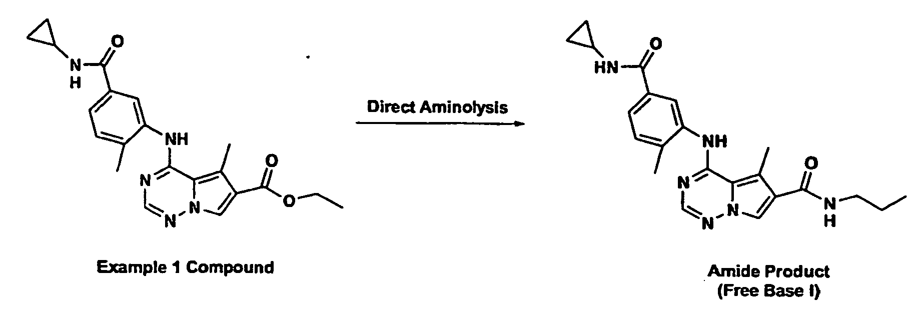

### Method A:

A solution of n-propylamine (6.5 eq) in THF (20 ml/g of SM) was cooled to ∼ -5°C and was slowly treated with 2.5 M solution of n-butyllithium (6.1 eq). The mixture was stirred for 10 min. At the end of the period, a slurry of Example 1 compound (1 eq) in THF (14 ml/g of SM) was cannulated into the preformed Li-NHPr solution. The reaction mixture was warmed to 25°C and stirred till all Example 1 compound was consumed (∼ 3 hr). After the reaction was judged to be completed by HPLC, the reaction mixture was cooled to ∼ 0°C and was slowly treated with acetic acid (5 ml/g of SM). The slurry was then warmed to ∼ 20°C and was stirred for 1 hr. At the end of the period, the solvent was distilled under vacuum to the minimum volume and the concentrated slurry was diluted with a solution of acetone (10 ml/g of SM) and water (20 ml/g of SM). The slurry was stirred for 1 hr and was cooled to ∼ 5°C. The slurry was filtered and the cake was washed with acetone (5 ml/g of SM). The cake was dried to give the amide product (typically in 85% yield and 99 AP).

### Method B:

A solution of n-propylamine (20 eq) in 2,2,2-trifluoroethanol (10 ml/g of SM) was slowly treated with 2.5 M solution of n-butyllithium (1.5 eq). The mixture was stirred for 5 min. At the end of the period, the starting material, Example 1 compound (1 eq), was added and the reaction mixture was warmed to 90°C. The reaction mixture was held at 90°C for 24 hr and was allowed to cool to ∼ 20°C. The reaction mixture was then analyzed by HPLC. Typically, analysis indicated there was only 1.57A% of starting material left.

### Method C:

A solution of n-propylamine (2 eq) in methylene chloride (10 ml/g of SM) at 20°C was slowly treated with 2.0 M solution of trimethylaluminum (4 eq) in hexanes. The mixture was stirred for 15 min. At the end of the period, the starting material, Example 1 compound (1 eq), was added and the reaction mixture was warmed to 60°C. The reaction mixture was held at 60°C for 24 hr and was allowed to cool to ∼ 20°C. The reaction mixture was then slowly quenched with aq. HCl solution and analyzed by HPLC. Typically, analysis indicated there was 96.8A% of amide product with 0.03A% of the di-propylamide impurity.

The product obtained in each of Methods A, B and C was the crystalline form of the free base I.

### EXAMPLE 4

### Preparation of Seeds of Form N-1 HCl Salt of Example 2 Compound

50-60 mg of Example 2 free base was suspended in 1 ml of THF or acetonitrile. 15-30 µL of HCl solution (37% aqueous) was added to the above suspension of Example 2 free base. The mixture turned into a clear solution. The solution was vigorously stirred at 20°C for 15 hours. The solution turned cloudy and into a white crystal slurry. The slurry was filtered and washed with cold THF and then air-dried or dried *in vacuo* at 40°C to produce a product in the form of a white powder identified as Form N-1 HCl salt of the Example 2 free base via powder X-ray diffraction.

Calculated, hybrid and observed powder X-ray diffraction patterns of the Example 4 Form N-1 hydrochloride salt are shown in Figure 1.

### EXAMPLE 5

### Form N-1 HCl Salt of Example 2 Compound from THF

4 g of Example 2 free base was suspended in 40 ml of THF. 1.8 mL of HCl solution (37% aqueous) (2.2 molar equivalent) was added to the suspension of Example 2 free base. The mixture turned into a clear solution. The solution was seeded with a small quantity (10-50 mg) of N-1 crystals of the Example 2 compound HCl salt. The solution turned cloudy and into a slurry at RT. The slurry was stirred at 20°C for 15 hr, filtered and washed with cold THF (∼ 50 ml) and then dried *in vacuo* at 45°C to produce a product in the form of a white powder at 95% yield identified as Form N-1 HCl salt of the Example 2 free base.

A powder X-ray diffraction pattern of the Example 5 hydrochloride salt is shown in Figure 1.

### The Form N-1 of HCl Salt of Example 2 Compound characterized by physical data

1. The simulated, experimental and hybrid PXRD patterns of Form N-1 HCl salt are shown in Figure 1. Characteristic PXRD peaks at room temperature are 8.7 ± 0.1, 12.1 ± 0.1, 13.3 ± 0.1, 13.7 ± 0.1, 14.6 ± 0.1, 17.5 ± 0.1, 18.2 ± 0.1,21.7 ± 0.1, 22.8 ± 0.1 and 24.3 ± 0.1.
2. The material melts with disproportionation as observed by DSC (Figure 7) and TGA (Figure 10). A broad endotherm typically in the range from about 125 to about 225°C is observed by DSC. TGA shows negligible weight loss up to about 100°C and a weight loss of about 8.2% up to about 225°C. A moisture-sorption isotherm of Form N-1 crystals of the hydrochloric acid salt of free base I is shown in Figure 13 which shows a negligible water uptake in the range from about 25 to about 75% RH at 25°C.
3. Elemental analysis calc: C, 59.65; H, 6.14; N, 18.97; Cl, 8.00, found: C: 59.42; H, 6.17; N, 18.87; Cl, 7.93.

A C-13 SSNMR spectrum of the Example 5 hydrochloride salt Form N-1 is shown in Figure 6 with peaks substantially as shown in Table 4.

### Single crystal X-ray diffraction measurement HCl salt Form N-1

a: 22.50(1) Å
b: 14.667(8) Å
c: 14.96(1) Å
V: 4405(9) Å
Space group: C2/c
D_{calc} (g-cm⁻³): 1.336
α: 90°
β: 116.78(5)°
γ: 90°
Z: 8
V/Z: 551 Å³
Temperature (°C): -50
R: 0.10

- R:: residual index, calculated to assess the agreement between the observations and the calculations of the structure factors and used to interpret correctness of the model
- V or V_{c}:: unit cell volume
- Z:: number of drug molecules per cell

The above abbreviations apply to tables in Examples 7 and 10 as well.

**Cell dimensions from hybrid:**
a: 22.73
b: 14.710
c: 15.04
α: 90
p: 117.13
γ: 90
V(Å³): 4475.02

### EXAMPLE 6

### Form N-1 HCl Salt of Example 2 Compound From DMF/Acetone

1 g of Example 2 free base was dissolved in approximately 20 mL of DMF at 35-40°C. To the resulting solution was added 1 mL of aqueous HCl solution (37% by wt) (about 5 molar equivalent). Form N-1 seed crystals of the Example 2 compound HCl salt were added to the reaction mixture and the mixture was stirred at 20°C. 10 mL of acetone was added and the mixture was stirred at 20°C for 5-15 hours. A white crystal slurry was formed which was filtered and the filter cake was washed with cold acetone. The wet cake was dried in vacuum at 40-45°C to produce a product in the form of a white powder identified as Form N-1 HCl salt of the Example 2 free base.

Calculated, hybrid and observed powder X-ray diffraction patterns of the Example 6 HCl salt is shown in Figure 1.

### EXAMPLE 7

### Form N-1 MSA Salt of Example 2 Compound in DMF/Acetone (Preferred)

4 g of Example 2 free base was dissolved in 30 ml of DMF at RT. 1.0 ml of methanesulfonic acid (MSA) was added. The initially clear colorless free base solution turned into a clear yellow solution. 50 ml of acetone was added. Seed crystals of Form N-1 of the Example 2 compound in the form of its MSA salt were added to the solution. The solution turned cloudy and into a slurry at RT. The slurry was stirred at RT for 5 hr, filtered and washed with cold acetone (∼ 50 ml), dried *in vacuo* at 45°C, to yield a product in the form of a white powder at 95% yield identified as Form N-1 MSA salt of the free base (1:1 salt).

Calculated and observed powder X-ray diffraction patterns of the Example 7 Form N-1 methanesulfonic acid salt are shown in Figure 3.

A C-13 SSNMR spectrum of the Example 7 MSA salt Form N-1 is shown in Figure 6 with peaks substantially as shown in Table 3.

### Single-crystal X-ray diffraction measurement MSA salt Form N-1

a: 9.818(1)Å
b: 11.127(1)Å
c: 13.004(1) Å
α: 97.32(1)°
β: 110.17(1)°
γ = 111.48(1)°
V: 1187.5(2) Å³
Space group: P 1 bat
D_{calc}(g-cm³): 1.403
Z: 2
Temperature (°C): +22
R: 0.06

### The Form N-1 polymorph of the MSA salt, characterized by physical data

1. The calculated PXRD pattern of Form N-1 MSA salt is shown in Figure 3. The diffractogram exhibits 2θ values at room temperature of 10.7 ± 0.1, 11.7 ± 0.1, 13.3 ± 0.1, 14.0 ± 0.1, 15.2 ± 0.1, 19.8 ± 0.1, 21.0 ± 0.1, 22.0 ± 0.1, 23.0 ± 0.1 and 24.4 ± 0.1.
2. The material typically exhibits a melt with decomposition with endotherm onset at about 216°C according to the differential scanning calorimetry (DSC) (Figure 9) and thermogravimetric analysis (TGA) (Figure 12). TGA shows a negligible weight loss up to about 150°C.
3. Elemental analysis calc: C, 54.97; H, 6.02; N, 16.72; S, 6.38, found: C: 54.95; H, 6.12; N, 16.51; S, 6.28.

### EXAMPLE 8

### Preparation of Form N-1 MSA Salt of Example 2 Compound in Isopropyl Alcohol

4.3 g of Example 2 free base was suspended in 40 mL of isopropyl alcohol. 0.9 mL of methanesulfonic acid (about 1.3 eq.) was added while stirring. The suspension became clear. The solution was stirred at 20°C and seeds of Form N-1 crystals of the Example 2 compound in the form of its MSA salt was added to the solution. The resulting hazy solution turned to a thick slurry in 10-20 min. The slurry was stirred at 20°C overnight, then filtered and the filter cake washed with cold isopropyl alcohol, and dried in vacuum at 45°C to yield Form N-1 crystals of the methanesulfonic acid salt of the Example 2 compound (1:1 salt).

Calculated and observed powder X-ray diffraction patterns of the Example 8 Form N-1 MSA salt are shown in Figure 3.

### EXAMPLE 9

### Preparation of Form N-1 MSA Salt of Example 2 Compound in Ethanol

10 g of Example 2 free base was suspended in 20 mL of ethanol. 2 mL of methanesulfonic acid (about 1.3 eq.) was added while stirring. The suspension became clear. The solution was stirred at 20°C and seeds of Form N-1 crystals of the Example 2 compound in the form of its MSA salt was added to the solution. The resulting hazy solution turned to a thick slurry in 10-20 min. The slurry was stirred at 20°C overnight, then filtered and the filter cake washed with cold ethanol, and dried in vacuum at 45°C to yield Form N-1 crystals of the methanesulfonic acid salt of the Example 2 compound (1:1 salt).

Calculated and observed powder X-ray diffraction patterns of the Example 10 Form N-1 MSA salt are shown in Figure 3.

### EXAMPLE 10

### Preparation of Seeds of Form N-4 HCl Salt of Example 2 Compound

59 mg of Example 2 free base was suspended in about 1 mL of absolute ethanol. About 15 µL of aqueous HCl solution (37% by wt) (1.3 molar equivalent) was added to the suspension. The suspension became a clear solution which was vigorously stirred at 20°C for at least 1 day. A white crystal slurry was formed which was filtered to recover the filter cake which was washed with cold ethanol. The wet cake was vacuum-dried or air-dried at 40°C to produce a product in the form of a white solid (1:1 salt).

The powder X-ray diffraction of this product shows the pattern in Figure 2.

### EXAMPLE 11

### Preparation of Form N-4 Crystals of HCl Salt of Example 2 Compound in Methylethyl Ketone-Formic Acid - Reversed Addition

A hydrochloric acid solution (∼ 37%, 14.0 g) was added to a slurry of Example 2 free base (35.0 g) in formic acid (51.2 g, 42 mL) - methylethyl ketone (MEK, 56.4 g, 70 mL) at room temperature to give a clear solution. The solution was filtered to remove insoluble solids and a rinse with MEK-HCOOH (22 mL-12 mL) was applied. The filtrate was slowly added to a slurry of seeds of N-4 crystals of the HCl salt of the Example 2 compound (285 mg) in MEK (665 mL) while stirring at 10-12°C over 57 minutes. The mixture was stirred at 11-14°C for 2 hours. The white solid was collected by filtration and washed with MEK (300 mL). The wet cake (46 g) was dried under a vacuum at ∼ 35°C for 22 h and then at 50-60°C for 3 days to give an off white solid (35.2 g, 92%) with an HPLC AP of 99.63. Form: N-4 by X-ray powder pattern. Residual solvent: MEK (1.68%); HCOOH (0.08%). Particle size: D95 (9.6 µm), D90 (7.1 µm). D50 (2.5 µm) (1:1 salt).

### The Form N-4 polymorph, characterized by physical data

1. The calculated PXRD pattern of Form N-4 HCl salt is shown in Figure 2. The diffractogram exhibits 2θ values at room temperature of 8.6 ± 0.1, 10.7 ± 0.1, 11.4 ± 0.1, 12.8 ± 0.1, 14.4 ± 0.1, 15.6 ± 0.15, 16.9 ± 0.1 and 23.4 ± 0.1.
2. The material melts with decomposition typically in the range from about 130 to about 220°C (variable) as shown by DSC (Figure 8). The thermogravimetric analysis (TGA) curve (Figure 11) shows a negligible weight loss up to about 125°C. A moisture-sorption isotherm of Form N-4 crystals of free base I is shown in Figure 14 which shows a negligible water uptake in the range from about 25 to about 75% RH at 30°C.
3. Elemental analysis calc: C, 59.65; H, 6.14; N, 18.97; Cl, 8.00, found: C: 59.64; H, 6.16; N, 18.84; Cl, 7.93.

A C-13 SSNMR spectrum of the Example 10 HCl salt Form N-4 is shown in Figure 5 with peaks substantially as shown in Table 3.

**Single crystal X-ray diffraction measurement HCl salt Form N-4**
a: 20.9498(5) Å
b: 13.8719(3) Å
c: 7.9133(2) Å
α: 90°
β: 100.052(1)°
γ: 90°
V: 2264.4(1) Å³
Space group: P2₁/n
D_{calc}(g-cm³): 1.2999
Z: 4
Temperature (°C): +22
R: 0.06

### EXAMPLE 12

### Preparation of Form N-4 Crystals of HCl Salt of Example 2 Compound in Acetone-Formic Acid - Reversed Addition

A hydrochloric acid solution (∼ 37%, 9.9 g) was added to a slurry of Example 2 free base (30.0 g) in formic acid (54.8 g, 45 mL)-acetone (71.2 g, 90 mL) at room temperature to give a clear solution. The solution was filtered to remove insoluble solids. The filtrate was slowly added to a slurry of seeds of N-4 crystals of the HCl salt of the Example 2 free base (300 mg) in acetone (540 mL) while stirring at room temperature over 20 minutes. The mixture was stirred at room temperature for 20 hours. The white solid was collected by filtration and washed with acetone (320 mL). The wet cake (37 g) was dried under vacuum at ∼ 50°C for 20 h to give an off white solid (30.95 g, 94%) with an HPLC AP of 99.61. Form: N-4 by X-ray powder pattern. Residual solvent: acetone (1.1%); HCOOH (0.37%). Particle size: D95 (76.4 µm), D90 (50.8 µm). D50 (6.1 µm) (1:1 salt).

Calculated and observed powder X-ray diffraction patterns of this product are shown in Figure 2.

### EXAMPLE 13

### Preparation of Form N-4 Crystals of HCl Salt of Example 2 Compound in Formic Acid and Acetone or Formic Acid and MEK

A hydrochloric acid solution (∼ 37%, 14.0 g) (1.6 molar equivalent) was added to a solution of Example 2 free base (35 g) in formic acid (52.5 mL)-acetone (115.5 mL) (or MEK). Seeds of N-4 crystals of the HCl salt of the Example 2 free base are added to the mixture. The solution was filtered to remove insoluble solids. Acetone (630 mL) (or MEK) is added to the mixture and the mixture is stirred at 10-20°C for 2-10 hours.

Alternatively, the solution of the free base can be added to the pool of acetone (or MEK)/HCl/N-4 seeds mixture to effect precipitation of small crystals of Form N-4.

A white solid is collected by filtration and washed with cold acetone or MEK. The wet cake is dried under vacuum at 40-45°C to give Form N-4 crystals as a white solid.

Calculated and observed powder X-ray diffraction patterns of this product are shown in Figure 2.

### EXAMPLE 14

### Preparation of Form N-4 HCl Salt of Example 2 Compound (DMA/Acetone or DMA/MEK System)

45 g of Example 2 free base were dissolved in 180 ml DMA at 65°C. 15 g HCl solution (37%) (1.4 molar equivalent) were added. 240 mg of seeds of Form N-4 Example 2 HCl salt were added into the HCl solution. 900 mL of acetone or MEK were added and the mixture stirred at 20°C for 5 to 6 hours.

Alternatively, the solution of the free base in DMA can be added to the pool of acetone (or MEK)/HCl/Form N-4 seeds mixture to effect precipitation of small crystals of Form N-4.

The slurry was filtered, and the wet cake was washed with cold acetone or MEK and dried under vacuum at 40-45°C. A white crystalline powder was obtained at 96% yield (1:1 salt).

Calculated and observed powder X-ray diffraction patterns of this product are shown in Figure 2.

### EXAMPLE 15

### Preparation of Form N-4 HCl Salt of Example 2 Compound (DMF-Acetone System - most preferred)

A hydrochloric acid solution (∼ 37%, 25.4 g) was added to a slurry of Example 2 free base (81.0 g) in dimethyl formamide (DMF) (612 g, 648 mL) at 20-25°C to give a clear yellow solution after 20-30 minutes stirring. The solution was then polish filtered to remove insoluble solid and the filter was rinsed with DMF (10-20 mL). A slurry of seeds of Form N-4 Example 2 HCl salt (1.6 g) in acetone (769 g, 972 mL) was then added to the filtrate while stirring over 2-3 minutes. Crystallization started immediately after the addition. The slurry was stirred at 20-25°C for 3.5 hours. The white solid was collected by filtration and washed with acetone (162 mL). The wet cake (92 g) was dried under vacuum at ∼ 45-50°C for 16 h to give a white solid (85 g, 96%) with an HPLC AP of 99.73 Form N-4 by X-ray powder pattern. Residual solvent by GC: DMF (1%); acetone (1%). Particle size: D90 30-60 µm (1:1 salt).

Calculated and observed powder X-ray diffraction patterns of this product are shown in Figure 2.

### EXAMPLE 16

### Preparation of Form N-4 HCl Salt of Example 2 Compound (in Acetone)

8.4 g of Example 2 free base were dissolved in 126 ml of acetone. 3.7 mL of HCl solution (37%) (2.2 molar equivalent) were added. Seeds of Form N-4 Example 2 HCl salt were added into the acetone/HCl solution, and the mixture was stirred at 20-40°C for at least 15 hours. A white crystal slurry was formed which was filtered, and the wet cake was washed with cold acetone, dried under vacuum at 40 to 45°C to produce a white solid (1:1 salt).

Calculated and observed powder X-ray diffraction patterns of Form N-4 crystals of the hydrochloric acid salt of Example 2 compound are shown in Figure 2.

### EXAMPLE 17

### Preparation of Form N-4 HCl Salt of Example 2 Compound (DMF-Acetone (most preferred) or MEK System)

A hydrochloric acid solution (∼ 37%, 1.4-3.2 mL) (1.1-2.2 molar equivalent) was added to a slurry of Example 2 free base (7.3 g) in about 40 mL dimethylformamide (DMF) (5-8 mL/g of free base) to give a clear solution. 60-80 mL of acetone or MEK was added. Seeds of Form N-4 Example 2 HCl salt were then added and the mixture was stirred at 20°C for 3 to 15 hours. A white solid crystal slurry was formed which was filtered and the filter cake washed with acetone. The wet cake (92 g) was dried under vacuum at ∼ 40-45°C to give a white crystalline powder (95-96% yield) (1:1 salt).

Calculated and observed powder X-ray diffraction patterns of this product are shown in Figure 2.

### EXAMPLE 18

### Preparation of Form N-4 Crystals of HCl Salt of Example 2 Compound in Ethanol

A hydrochloric acid solution (∼ 37%, 0.8 mL) was added to a slurry of Example 2 free base (3 g) in absolute ethanol (about 30 mL) to give a clear solution. Seeds of N-4 crystals of the HCl salt of the Example 2 free base (50 mg) were added to the mixture. The mixture was stirred at 20-40°C for 15 hours. A white crystal slurry was formed which was filtered and the recovered cake was washed with cold ethanol (100 mL). The wet cake was dried under vacuum at 40-50°C for 15 h to give a white solid (1:1 salt).

Calculated and observed powder X-ray diffraction patterns of Form N-4 crystals of the hydrochloric acid salt of Example 18 are shown in Figure 2.

### EXAMPLE 19

### Form N-4 HCl Salt of Example 2 Compound in THF

1.2 g of Example 2 free base was suspended in about 10 mL of THF. 0.5 mL of aqueous HCl solution (37% by wt) (2.2 molar equivalent) was added. Seed crystals of Form N-4 of the Example 2 compound in the form of its HCl salt were added to the mixture and the resulting slurry was stirred at 40°C for 4 days or at 20°C for 7 days. The resulting crystal slurry was filtered and washed with cold THF, and dried *in vacuo* at 40-45°C, to yield a product in the form of a white powder at 95% yield identified as Form N-4 HCl salt of the free base (1:1 salt).

Calculated and observed powder X-ray diffraction patterns of the Example 19 Form N-4 hydrochloric acid salt are shown in Figure 2.

### EXAMPLE 20

### Preparation of Form N-4 Crystals of HCl Salt of Example 2 Compound by Slurrying Form N-1 Crystals

Dry Form N-1 crystals prepared as described in Example 4 were slurried in acetonitrile, THF or ethanol with seeds of Form N-4 crystals and the suspension was stirred at 40°C for 4 days and cooled. The slurry was filtered and the filter cake washed with cold THF or acetone. The wet cake was dried in vacuum at 40-45°C to produce a white crystalline powder identified as Form N-4 crystals (1:1 salt).

Calculated and observed powder X-ray diffraction patterns of the resulting white crystalline powder are shown in Figure 2.

### EXAMPLE 21

### Preparation of the Sesquihydrate Form H1.5-3 of HCl Salt of Example 2 Free Base

60 mg of Example 2 free base was suspended in 1 ml of ethanol. 15 µl of HCl solution (37% aqueous) (1.25 molar equivalent) was added to the suspension of Example 2 free base. The mixture turned into a cloudy solution. Additional 15 µl of HCl solution (37% aqueous) (1.25 molar equivalent) was added to the suspension, and the cloudy solution became clear. The solution was stirred at 20°C for 15 hr. A white slurry was obtained. The slurry was air-dried under ambient condition (approx. 20% and 1 atm) to produce title sesquihydrate.

The fractional atomic coordinates for the title sesquihydrate are shown in Table 9.

### EXAMPLE 22

### Preparation of Form SA-2 Solvate of the HCl Salt of Example 2 Compound

15 mg of Form N-4 crystals of the HCl salt of the Example 2 compound was dissolved in ∼ 1/2 ml of 1:1 MEK/MeOH. The sample was allowed to evaporate at room temperature until crystals of the title solvate appeared.

The fractional atomic coordinates for the title SA-2 solvate are shown in Table 7.

### EXAMPLE 23

### Preparation of Form SB-2 Solvate of the HCl Salt of Example 2 Compound

A concentrated solution was prepared by heating Form N-4 crystals of the HCl salt of the Example 2 compound in isopropyl alcohol followed by cooling to ambient temperature and slow evaporation to produce title solvate.

The fractional atomic coordinates for the title SB-2 solvate are shown in Table 8.

### EXAMPLE 24

### Studies of Crystal Forms Prepared in Previous Examples

X-ray powder diffraction (PXRD) data were obtained using a Bruker C2 GADDS (General Area Detector Diffraction System). The radiation was Cu Kα (40 KV, 50mA). The sample-detector distance was 15 cm. Powder samples were placed in sealed glass capillaries of 1mm or less in diameter; the capillary was rotated during data collection. Data were collected for 3 ≤2θ≤35° with a sample exposure time of at least 2000 seconds. The resulting two-dimensional diffraction arcs were integrated to create a traditional 1-dimensional PXRD pattern with a step size of 0.02 degrees 2θ in the range of 3 to 35 degrees 2θ.

Single crystal X-ray data were collected on a Bruker-Nonius CAD4 serial diffractometer (Bruker Axs, Inc., Madison WI). Unit cell parameters were obtained through least-squares analysis of the experimental diffractometer settings of 25 high-angle reflections. Intensities were measured using Cu Kα radiation (λ = 1.5418 Å) at a constant temperature with the θ-2θ variable scan technique and were corrected only for Lorentz-polarization factors. Background counts were collected at the extremes of the scan for half of the time of the scan. Alternately, single crystal data were collected on a Bruker-Nonius Kappa CCD 2000 system using Cu Kα radiation (λ = 1.5418 Å). Indexing and processing of the measured intensity data were carried out with the HKL2000 software package in the Collect program suite R. Hooft, Nonius B.V. (1998). When indicated, crystals were cooled in the cold stream of an Oxford cryogenic system during data collection.

The structures were solved by direct methods and refined on the basis of observed reflections using either the SDP software package SDP, Structure Determination Package, Enraf-Nonius, Bohemia, N.Y.) with minor local modifications or the crystallographic package, MAXUS (maXus solution and refinement software suit: S. Mackay, C.J. Gilmore, C. Edwards, M. Tremayne, N. Stewart, and K. Shankland. maXus is a computer program for the solution and refinement of crystal structures from diffraction data.

The derived atomic parameters (coordinates and temperature factors) were refined through full matrix least-squares. The function minimized in the refinements was Σ_{W}(|F_{O}| - |F_{C}|)². R is defined as Σ ∥F| - |F∥/Σ |F_{O}| while R_{W} = [Σ_{W}( |F_{O}| - |F_{C}|)²/Σ_{W} |F_{O}|²]^{1/2} where w is an appropriate weighting function based on errors in the observed intensities. Difference maps were examined at all stages of refinement. Hydrogen atoms were introduced in idealized positions with isotropic temperature factors, but no hydrogen parameters were varied.

"Hybrid" simulated powder X-ray patterns were generated as described in the literature (Yin. S.; Scaringe, R. P.; DiMarco, J.; Galella, M. and Gougoutas, J. Z., American Pharmaceutical Review (2003), 6(2), 80). The room temperature cell parameters were obtained by performing a cell refinement using the CellRefine.xls program. Input to the program includes the 2-theta position of ca. 10 reflections, obtained from the experimental room temperature powder pattern; the corresponding Miller indices, *hkl*, were assigned based on the single-crystal data collected at low temperature. A new (hybrid) PXRD was calculated (by either of the software programs, Alex or LatticeView) by inserting the molecular structure determined at low temperature into the room temperature cell obtained in the first step of the procedure. The molecules are inserted in a manner that retains the size and shape of the molecule and the position of the molecules with respect to the cell origin, but, allows intermolecular distances to expand with the cell.

The characteristic diffraction peak positions (degrees 2θ ± 0.1) at RT of PXRD patterns shown in the accompanying Figures are based on high quality patterns collected with a diffractometer (CuKα) with a spinning capillary with 2θ calibrated with a NIST or other suitable standard.

All solid-state C-13 NMR measurements were made with a Bruker DSX-400, 400 MHz NMR spectrometer. High resolution spectra were obtained using high-power proton decoupling and the TPPM pulse sequence and ramp amplitude cross-polarization (RAMP-CP) with magic-angle spinning (MAS) at approximately 12 kHz. (A.E. Bennett et al, J. Chem. Phys. (1995), 103, 6951), (G. Metz, X. Wu and S.O. Smith, J. Magn. Reson. A (1994), 110, 219-227). Approximately 70 mg of sample, packed into a canister-design zirconia rotor was used for each experiment. Chemical shifts (δ) were referenced to external adamantane with the high frequency resonance being set to 38.56 ppm (W.L. Earl and D.L. VanderHart, J. Magn. Reson. (1982), 48, 35-54).

Differential scanning calorimetry (DSC) experiments were performed in a TA Instruments™ model Q1000. The sample (about 2-6 mg) was weighed in an aluminum pan and recorded accurately recorded to a hundredth of a milligram, and transferred to the DSC. The instrument was purged with nitrogen gas at 50mL/min. Data were collected between room temperature and 300°C at 10°C/min heating rate. The plot was made with the endothermic peaks pointing down.

Thermal gravimetric analysis (TGA) experiments were performed in a TA Instruments™ model Q500. The sample (about 10-30 mg) was placed in a platinum pan previously tared. The weight of the sample was measured accurately and recorded to a thousand of a milligram by the instrument. The furnace was purged with nitrogen gas at 100mL/min. Data were collected between room temperature and 300°C at 10°C/min heating rate.

Moisture sorption isotherms were collected in a VTI SGA-100 Symmetric Vapor Analyzer using approximately 10 mg of sample. The sample was dried at 60°C until the loss rate of 0.0005 wt %/min was obtained for 10 minutes. The sample was tested at 25°C (for free base 1 and N-1 HCl salt) or 30°C (for N-4 HCl salt) and 3 or 4, 5, 15, 25, 35, 45, 50, 65, 75, 85, and 95% RH. Equilibration at each RH was reached when the rate of 0.0003 wt %/min for 35 minutes was achieved or a maximum of 600 minutes.

Various crystalline forms of free base I and its solvates were prepared and are tabulated in Table 1. The unit cell data and other properties for all crystalline forms of the invention are tabulated and summarized in Table 2. The unit cell parameters were obtained from single crystal X-ray crystallographic analysis. A detailed account of unit cells can be found in Chapter 3 of Stout & Jensen, "X-Ray Structure Determination: A Practical Guide", (MacMillian, 1968).

**TABLE 1**

| Form | Description |
|---|---|
| N-1 HCl salt | Neat crystal |
| N-4 HCl salt | Neat crystal |
| N-1 MSA salt | Neat crystal |
| SA-2 | Hydrated methanolate crystal |
| SB-2 | Hydrated isopropylate crystal |
| H1.5-3 | Sesquihydrate crystal |

**TABLE 2**

| **Crystallographic Data** **Unit Cell Parameters** | | | | | | | | | | | | | | |
|---|---|---|---|---|---|---|---|---|---|---|---|---|---|---|
| Salt | Form | Solvent % (w/w) | Solvate | T°C | a(Å) | b(Å) | c(Å) | α,° | β,° | γ,° | Z' | SG | R | Vₘ |
| MSA | N-1 | - | None | +22 | 9.818(1) | 11.127(1) | 13.004(1) | 97.32(1) | 110.17(1) | 111.48(1) | 1 | P-1 | .06 | 594 |
| HCl | N-1 | - | None | -50 | 22.50(1) | 14.667(8) | 14.96(1) | 90 | 116.78(5) | 90 | 1 | C2/c | .10 | 551 |
| HCl | N-4 | - | None | +22 | 20.9498(5) | 13.8719(3) | 7.9133(2) | 90 | 100.052(1) | 90 | 1 | P2₁/n | .06 | 566 |
| HCl | SA-2 | 9.8 | 1 MeOH, 1H₂O | -50 | 11.747(3) | 14.233(2) | 8.118(3) | 105.95(2) | 104.02(2) | 90.80(2) | 1 | P-1 | .16 | 631 |
| HCl | SB-2 | 11.5 | 1 IPA, 1 H₂O | -50 | 12.226(7) | 14.653(5) | 8.083(4) | 107.31(3) | 103.90(5) | 85.79(4) | 1 | P-1 | .16 | 671 |
| HCl | H1.5-3 | 5.8 | 1.5 H2O | -80 | 12.140(1) | 17.623(1) | 11.983(1) | 92.83(1) | 96.76(1) | 108.25(1) | 2 | P-1 | .06 | 602 |
| HCl | N-1* | | None | RT | 22.73 | 14.710 | 15.04 | 90 | 117.13° | 90 | 1 | C2/c | na | 559 |

| | | | | | | | | | | | | | | |
|---|---|---|---|---|---|---|---|---|---|---|---|---|---|---|
| * generated from hybrid (refined) calculations cell parameters for single cell and hybrid substantially as listed above T (°C) is the temperature for the crystallographic data Z' is the number of molecules of free base I in each asymmetric unit (not unit cell) Vₘ is the molar volume, V(unit cell)/(Z drug molecules per cell) SG is the crystallographic space group R is the R-factor (measure of the quality of the refinement) | | | | | | | | | | | | | | |

The fractional atomic coordinates for the various crystalline forms are substantially as tabulated in Tables 4 to 9.

The Carbon 13 SSNMR chemical shifts for each form are substantially as tabulated in Table 3.

**TABLE 3**

| **Carbon-13 SSNMR Chemical Shifts for Each of the Forms** | | |
|---|---|---|
| N-1 MSA | N-1 HCl | N-4 HCl |
| 6 | 3 | 4.1 |
| 8.8 | 7.3 | 6.3 |
| 10.4 | 12.9 | 12.4 |
| 14.3 | 15.2 | 14.4 |
| 20.4 | 19.4 | 17.1 |
| 24.4 | 24.6 | 21 |
| 40.3 | 40.5 | 23.5 |
| 110.3 | 109.8 | 43.1 |
| 123.5 | 122.3 | 110 |
| 126 | 125 | 122.4 |
| 126.9 | 127.3 | 125.1 |
| 129.8 | 128.1 | 128.1 |
| 132.3 | 131.7 | 129.1 |
| 135.6 | 136.9 | 130.4 |
| 138.1 | 142.9 | 131.7 |
| 139.2 | 147.3 | 134.7 |
| 148.8 | 162.5 | 135.7 |
| 163.1 | 167.1 | 138.9 |
| 172.6 | | 147.1 |
| | | 163.2 |
| | | 171.8 |

**TABLE 4**

| **Positional Parameters and Isotropic Temperature Factors for Free Base I HCl Salt Form N-1 at -50°C** | | | | |
|---|---|---|---|---|
| Name | x | y | z | B(iso) |
| CL1 | -0.1274 | 0.7316 | 0.1928 | 2.6 |
| O11 | -0.0911 | 0.3308 | 0.0631 | 3.0 |
| 025 | 0.0768 | 1.0932 | 0.1927 | 2.5 |
| N3 | -0.0789 | 0.5597 | -0.1360 | 1.4 |
| N-4 | -0.0726 | 0.6044 | -0.2111 | 2.0 |
| N6 | -0.0627 | 0.7370 | -0.1109 | 2.0 |
| N-12 | -0.1338 | 0.2956 | -0.1016 | 2.4 |
| N-16 | -0.0670 | 0.7356 | 0.0417 | 1.5 |
| N28 | 0.1170 | 0.9587 | 0.2589 | 2.6 |
| C1 | -0.0898 | 0.4443 | -0.0493 | 1.3 |
| C2 | -0.0889 | 0.4657 | -0.1398 | 1.5 |
| C5 | -0.0632 | 0.6929 | -0.1963 | 2.5 |
| C7 | -0.0689 | 0.6963 | -0.0354 | 2.2 |
| C8 | -0.0755 | 0.5942 | -0.0480 | 1.7 |
| C9 | -0.0808 | 0.5253 | 0.0083 | 1.0 |
| C10 | -0.1055 | 0.3490 | -0.0243 | 1.8 |
| C13 | -0.1599 | 0.2059 | -0.0880 | 3.3 |
| C14 | -0.2373 | 0.2200 | -0.1225 | 6.2 |
| C15 | -0.2651 | 0.1334 | -0.1018 | 8.3 |
| C17 | -0.0610 | 0.8346 | 0.0471 | 1.7 |
| C18 | -0.0007 | 0.8710 | 0.1155 | 1.1 |
| C19 | 0.0047 | 0.9629 | 0.1264 | 1.0 |
| C20 | -0.0480 | 1.0206 | 0.0738 | 2.2 |
| C21 | -0.1086 | 0.9820 | 0.0094 | 2.3 |
| C22 | -0.1188 | 0.8899 | -0.0051 | 1.8 |
| C23 | -0.0769 | 0.5314 | 0.1117 | 2.5 |
| C24 | 0.0678 | 1.0108 | 0.1954 | 2.0 |
| C27 | -0.1847 | 0.8464 | -0.0730 | 2.9 |
| C29 | 0.1795 | 1.0002 | 0.3324 | 3.0 |
| C30 | 0.2351 | 0.9973 | 0.3046 | 5.6 |
| C31 | 0.2374 | 0.9376 | 0.3874 | 4.9 |
| H61 | -0.0563 | 0.8116 | -0.1063 | 2.8 |
| H121 | -0.1406 | 0.3168 | -0.1761 | 3.4 |
| H161 | -0.0714 | 0.6963 | 0.1014 | 2.5 |
| H281 | 0.1109 | 0.8848 | 0.2553 | 3.6 |

**TABLE 5**

| **Positional Parameters and Isotropic Temperature Factors for Free Base I HCl Salt Form N-4 at RT** | | | | |
|---|---|---|---|---|
| Name | x | y | z | B(iso) |
| CL | 0.0341 | 0.7162 | 0.4308 | 5.5 |
| O1 | 0.1111 | 1.0617 | 0.3458 | 5.4 |
| O2 | -0.2021 | 0.5613 | -0.6888 | 6.5 |
| N-1 | -0.0710 | 0.5099 | 0.0187 | 5.0 |
| N2 | -0.0544 | 0.6737 | 0.0953 | 4.4 |
| N3 | -0.0913 | 0.5440 | -0.1460 | 4.2 |
| N-4 | -0.0762 | 0.8037 | -0.0894 | 4.3 |
| N5 | 0.1228 | 0.9001 | 0.3778 | 4.5 |
| N6 | -0.1487 | 0.4216 | -0.6361 | 5.1 |
| C1 | -0.0540 | 0.5768 | 0.1292 | 4.9 |
| C2 | -0.0752 | 0.7092 | -0.0651 | 3.8 |
| C3 | -0.1215 | 0.6429 | -0.3703 | 4.0 |
| C4 | -0.1318 | 0.5462 | -0.4222 | 4.0 |
| C5 | -0.1137 | 0.4874 | -0.2809 | 4.6 |
| C6 | -0.0956 | 0.6414 | -0.1958 | 3.7 |
| C7 | -0.0604 | 0.8753 | 0.0446 | 3.9 |
| C8 | 0.0035 | 0.8873 | 0.1212 | 3.9 |
| C9 | 0.0202 | 0.9626 | 0.2399 | 3.8 |
| C10 | -0.0285 | 1.0247 | 0.2755 | 4.4 |
| C11 | -0.0924 | 1.0089 | 0.1989 | 4.6 |
| C12 | -0.1101 | 0.9344 | 0.0833 | 4.3 |
| C13 | 0.0882 | 0.9795 | 0.3247 | 4.3 |
| C14 | 0.1885 | 0.9076 | 0.4700 | 4.8 |
| C15 | 0.2433 | 0.8987 | 0.3757 | 7.1 |
| C16 | 0.2285 | 0.8180 | 0.4863 | 7.3 |
| C17 | -0.1632 | 0.5116 | -0.5947 | 4.6 |
| C18 | -0.1796 | 0.3769 | -0.7945 | 5.8 |
| C19 | -0.1408 | 0.2925 | -0.8428 | 7.6 |
| C20 | -0.1734 | 0.2467 | -1.0053 | 9.0 |
| C21 | -0.1342 | 0.7314 | -0.4822 | 5.3 |
| C22 | -0.1802 | 0.9169 | 0.0035 | 6.0 |
| H11 | -0.0396 | 0.5549 | 0.2601 | 5.9 |
| H21 | -0.0375 | 0.7237 | 0.1998 | 5.4 |
| H41 | -0.0910 | 0.8303 | -0.2198 | 5.3 |
| H51 | -0.1153 | 0.4087 | -0.2785 | 5.6 |
| H51 | 0.1006 | 0.8297 | 0.3463 | 5.5 |
| H61 | -0.1122 | 0.3809 | -0.5485 | 4.0 |
| H81 | 0.0407 | 0.8396 | 0.0891 | 4.8 |
| H101 | -0.0165 | 1.0872 | 0.3579 | 5.4 |
| H111 | -0.1294 | 1.0551 | 0.2302 | 5.4 |
| H141 | 0.1928 | 0.9796 | 0.5314 | 5.9 |

**TABLE 6**

| **Positional Parameters and Isotropic Temperature Factors for Free Base I MSA Salt Form N-1 at RT** | | | | |
|---|---|---|---|---|
| Name | x | y | z | B(iso) |
| S1 | -0.2392 | -0.0718 | 0.2812 | 3.9 |
| O19 | -0.3458 | 0.3140 | 0.5245 | 3.9 |
| O25 | 0.1495 | 0.5491 | -0.1489 | 4.5 |
| O96 | -0.0747 | -0.0117 | 0.2977 | 7.2 |
| O97 | -0.3175 | 0.0086 | 0.2347 | 6.2 |
| O98 | -0.3273 | -0.2110 | 0.2187 | 6.2 |
| N2 | 0.0159 | 0.2025 | 0.2128 | 3.5 |
| N-4 | 0.0134 | 0.1493 | 0.0309 | 4.3 |
| N5 | 0.0858 | 0.2860 | 0.0493 | 3.4 |
| N-10 | 0.1369 | 0.4167 | 0.3376 | 3.1 |
| N20 | -0.4171 | 0.1644 | 0.3592 | 3.7 |
| N26 | 0.3707 | 0.6920 | 0.0118 | 3.9 |
| C1 | 0.0952 | 0.3375 | 0.2357 | 2.9 |
| C3 | -0.0185 | 0.1153 | 0.1135 | 4.0 |
| C6 | 0.1200 | 0.3448 | -0.0287 | 3.8 |
| C7 | 0.1918 | 0.4820 | 0.0194 | 3.3 |
| C8 | 0.2006 | 0.5099 | 0.1308 | 3.1 |
| C9 | 0.1335 | 0.3851 | 0.1494 | 2.9 |
| C11 | 0.0777 | 0.3716 | 0.4202 | 2.8 |
| C12 | -0.0828 | 0.3343 | 0.3948 | 3.0 |
| C13 | -0.1378 | 0.3039 | 0.4767 | 2.8 |
| C14 | -0.0287 | 0.3161 | 0.5846 | 3.3 |
| C15 | 0.1288 | 0.3502 | 0.6066 | 3.4 |
| C16 | 0.1867 | 0.3782 | 0.5256 | 3.2 |
| C17 | 0.3599 | 0.4139 | 0.5526 | 4.5 |
| C18 | -0.3085 | 0.2627 | 0.4558 | 3.0 |
| C21 | -0.5877 | 0.1142 | 0.3265 | 4.7 |
| C22 | -0.6743 | -0.0158 | 0.3494 | 6.0 |
| C23 | -0.6918 | -0.0137 | 0.2327 | 5.2 |
| C24 | 0.2358 | 0.5773 | -0.0471 | 3.4 |
| C27 | 0.4320 | 0.7945 | -0.0402 | 4.8 |
| C28 | 0.5627 | 0.7822 | -0.0713 | 6.1 |
| C29 | 0.5061 | 0.6520 | -0.1574 | 6.3 |
| C30 | 0.2560 | 0.6437 | 0.2098 | 4.3 |
| C99 | -0.2317 | -0.0630 | 0.4193 | 5.7 |
| H21 | -0.0223 | 0.1622 | 0.2752 | 3.7 |
| H101 | 0.2234 | 0.5211 | 0.3609 | 3.3 |
| H201 | -0.3748 | 0.1234 | 0.3044 | 4.1 |
| H261 | 0.4413 | 0.7077 | 0.1023 | 4.0 |

**TABLE 7**

| **Positional Parameters and Isotropic Temperature Factors for Free Base I HCl Salt Form SA-2 at -50°C** | | | | | |
|---|---|---|---|---|---|
| Atom | x | y | z | Occupancy* | B(iso) |
| CL1 | -0.3428 | 0.2986 | 0.4057 | | 4.0 |
| O11 | 0.1415 | -0.1288 | 0.0915 | | 3.9 |
| O25 | -0.1148 | 0.6339 | 0.9119 | | 3.5 |
| O97 | -0.1278 | 0.2166 | 0.2592 | | 3.5 |
| O99 | -0.6027 | 0.1956 | 0.2912 | | 6.9 |
| N3 | 0.1841 | 0.0359 | 0.6720 | | 2.1 |
| N-4 | 0.2227 | 0.0672 | 0.8548 | | 3.0 |
| N6 | 0.1458 | 0.2173 | 0.8268 | | 2.7 |
| N-12 | 0.2592 | -0.1971 | 0.2792 | | 2.7 |
| N-16 | 0.0417 | 0.2432 | 0.5638 | | 2.2 |
| N26 | -0.2462 | 0.5099 | 0.7277 | | 2.9 |
| C1 | 0.1709 | -0.0518 | 0.3977 | | 1.9 |
| C2 | 0.2115 | -0.0502 | 0.5723 | | 2.1 |
| C5 | 0.2016 | 0.1581 | 0.9223 | | 3.1 |
| C7 | 0.1021 | 0.1869 | 0.6506 | | 1.8 |
| C8 | 0.1275 | 0.0916 | 0.5638 | | 1.9 |
| C9 | 0.1185 | 0.0380 | 0.3895 | | 2.3 |
| C10 | 0.1872 | -0.1291 | 0.2423 | | 2.3 |
| C13 | 0.2931 | -0.2744 | 0.1384 | | 3.6 |
| C14' | 0.4246 | -0.2681 | 0.1706 | .4 | 3.2 |
| C14 | 0.3896 | -0.2366 | 0.0787 | .6 | 5.5 |
| C15' | 0.4489 | -0.1753 | 0.1127 | .4 | 4.9 |
| C15 | 0.5057 | -0.1862 | 0.2099 | .6 | 6.8 |
| C17 | 0.0494 | 0.3502 | 0.6400 | | 2.0 |
| C18 | -0.0449 | 0.3928 | 0.6869 | | 2.1 |
| C19 | -0.0398 | 0.4954 | 0.7501 | | 1.9 |
| C20 | 0.0607 | 0.5480 | 0.7619 | | 2.8 |
| C21 | 0.1563 | 0.5053 | 0.7135 | | 2.8 |
| C22 | 0.1506 | 0.4021 | 0.6486 | | 2.3 |
| C23 | 0.0726 | 0.0704 | 0.2292 | | 2.5 |
| C24 | -0.1395 | 0.5477 | 0.7991 | | 2.3 |
| C27 | -0.3462 | 0.5629 | 0.7636 | | 3.4 |
| C28 | -0.3912 | 0.5530 | 0.9148 | | 5.9 |
| C29 | -0.4584 | 0.5075 | 0.7298 | | 5.0 |
| C30 | 0.2530 | 0.3528 | 0.5864 | | 4.2 |
| C98 | -0.5787 | 0.0988 | 0.2738 | | 7.5 |
| H21 | 0.2597 | -0.1054 | 0.6260 | | 3.6 |
| H51 | 0.2303 | 0.1880 | 1.0674 | | 4.2 |
| H121 | 0.2948 | -0.1958 | 0.4160 | | 4.2 |
| H161 | -0.0156 | 0.2110 | 0.4308 | | 3.8 |
| H261 | -0.2643 | 0.4358 | 0.6360 | | 4. |

| | | | | | |
|---|---|---|---|---|---|
| Occupancy is 1 unless otherwise noted | | | | | |

**TABLE 8**

| **Positional Parameters and Isotropic Temperature Factors for Free Base HCl Salt Form SB-2 at -50°C** | | | | |
|---|---|---|---|---|
| Atom | x | y | z | B(iso) |
| CL | -0.3194 | 0.3110 | 0.3979 | 3.3 |
| O12 | 0.1098 | -0.1439 | 0.0940 | 2.8 |
| O25 | -0.1316 | 0.6306 | 0.8959 | 3.8 |
| O96 | -0.1083 | 0.2272 | 0.2612 | 3.0 |
| O99 | -0.5607 | 0.2241 | 0.3457 | 7.8 |
| N-1 | 0.1740 | 0.0347 | 0.6778 | 1.6 |
| N2 | 0.2104 | 0.0654 | 0.8632 | 1.8 |
| N-4 | 0.1532 | 0.2174 | 0.8323 | 2.6 |
| N-13 | 0.2301 | -0.2130 | 0.2814 | 1.9 |
| N-17 | 0.0517 | 0.2466 | 0.5651 | 1.8 |
| N24 | -0.2474 | 0.5107 | 0.7138 | 2.5 |
| C3 | 0.2010 | 0.1560 | 0.9256 | 2.9 |
| C5 | 0.1066 | 0.1875 | 0.6510 | 1.6 |
| C6 | 0.1235 | 0.0878 | 0.5664 | 1.7 |
| C7 | 0.1131 | 0.0337 | 0.3959 | 1.8 |
| C8 | 0.1525 | -0.0608 | 0.4036 | 1.0 |
| C9 | 0.1907 | -0.0578 | 0.5837 | 2.0 |
| C10 | 0.0678 | 0.0602 | 0.2234 | 1.7 |
| C11 | 0.1616 | -0.1381 | 0.2479 | 1.4 |
| C14 | 0.2639 | -0.2961 | 0.1402 | 2.3 |
| C15 | 0.3837 | -0.2824 | 0.1328 | 5.6 |
| C16 | 0.4181 | -0.1943 | 0.1049 | 6.8 |
| C18 | 0.0563 | 0.3514 | 0.6400 | 1.1 |
| C19 | 0.1511 | 0.4008 | 0.6614 | 2.0 |
| C20 | 0.1524 | 0.4993 | 0.7188 | 2.5 |
| C21 | 0.0497 | 0.5468 | 0.7668 | 2.6 |
| C22 | 0.2534 | 0.3490 | 0.6029 | 3.5 |
| C23 | -0.0448 | 0.3958 | 0.6813 | 2.1 |
| C24 | -0.0409 | 0.4935 | 0.7394 | 1.3 |
| C26 | -0.3474 | 0.5677 | 0.7526 | 3.1 |
| C27 | -0.4538 | 0.5122 | 0.7195 | 3.2 |
| C28 | -0.3826 | 0.5649 | 0.9102 | 4.3 |
| C29 | -0.1469 | 0.5490 | 0.7925 | 2.3 |
| C96 | -0.5012 | 0.0658 | 0.2076 | 6.1 |
| C97 | -0.6246 | 0.0925 | 0.4159 | 6.4 |
| C98 | -0.5233 | 0.1304 | 0.3770 | 5.2 |
| H41 | 0.1522 | 0.2938 | 0.9032 | 3.6 |
| H131 | 0.2640 | -0.2097 | 0.4205 | 2.9 |
| H171 | -0.0007 | 0.2159 | 0.4327 | 2.8 |
| H241 | -0.2581 | 0.4367 | 0.6200 | 3.5 |

**TABLE 9**

| **Positional Parameters and Isotropic Temperature Factors for Free Base I HCl Salt Form H1.5-3 at -80°C** | | | | | |
|---|---|---|---|---|---|
| Name | x | y | z | Occupancy* | B(iso) |
| CL1 | 0.5892 | 0.3099 | 0.5880 | | 8 |
| CL2 | -0.0317 | 0.1959 | -0.0352 | | 4.6 |
| O17 | 0.6731 | 0.0637 | 0.2945 | | 3.4 |
| O23 | -0.1865 | -0.0509 | 0.6484 | | 3.9 |
| O47 | 1.1805 | 0.5785 | -0.1968 | | 3.5 |
| O53 | 0.3028 | 0.4127 | 0.1618 | | 3.3 |
| O97 | 0.2355 | 0.2145 | 0.3610 | | 5.5 |
| O98 | 0.1189 | 0.2770 | 0.1935 | | 3.9 |
| O99 | 0.4729 | 0.2641 | 0.3288 | | 4.8 |
| N-1 | 0.1843 | 0.2548 | 0.6420 | | 3.2 |
| N3 | 0.3402 | 0.2074 | 0.6047 | | 2.7 |
| N8 | 0.1235 | 0.1745 | 0.6554 | | 2.7 |
| N-10 | 0.3390 | 0.0760 | 0.6099 | | 2.6 |
| N-18 | 0.5571 | 0.1402 | 0.2550 | | 2.8 |
| N24 | -0.1614 | 0.0332 | 0.8085 | | 3.2 |
| N31 | 0.6409 | 0.7268 | 0.1112 | | 3.0 |
| N33 | 0.8061 | 0.6873 | 0.0833 | | 2.7 |
| N38 | 0.5903 | 0.6475 | 0.1331 | | 2.6 |
| N-40 | 0.8226 | 0.5613 | 0.1034 | | 2.6 |
| N-48 | 1.0561 | 0.6489 | -0.2397 | | 2.8 |
| N54 | 0.3170 | 0.5120 | 0.2994 | | 3.0 |
| C2 | 0.2863 | 0.2651 | 0.6168 | | 3.0 |
| C4 | 0.2817 | 0.1287 | 0.6158 | | 2.3 |
| C5 | 0.0769 | 0.0408 | 0.6513 | | 2.8 |
| C6 | -0.0172 | 0.0656 | 0.6821 | | 2.6 |
| C7 | 0.0158 | 0.1492 | 0.6845 | | 2.8 |
| C9 | 0.1647 | 0.1094 | 0.6372 | | 2.4 |
| C11 | 0.4587 | 0.0958 | 0.5837 | | 2.6 |
| C16 | 0.5518 | 0.1136 | 0.6728 | | 2.8 |
| C15 | 0.6634 | 0.1255 | 0.6448 | | 3.4 |
| C14 | 0.6805 | 0.1203 | 0.5319 | | 3.0 |
| C13 | 0.5865 | 0.1061 | 0.4452 | | 2.3 |
| C12 | 0.4756 | 0.0941 | 0.4708 | | 2.2 |
| C17 | 0.6093 | 0.1015 | 0.3255 | | 2.6 |
| C19 | 0.5700 | 0.1403 | 0.1362 | | 3.1 |
| C20 | 0.6825 | 0.1891 | 0.0997 | | 6.8 |
| C21 | 0.5763 | 0.2148 | 0.0824 | | 6.5 |
| C23 | -0.1288 | 0.0110 | 0.7108 | | 3.0 |
| C25 | -0.2663 | -0.0162 | 0.8493 | | 4.0 |
| C26' | -0.2650 | 0.0038 | 0.9675 | | 6.6 |
| C26 | -0.2371 | -0.0699 | 0.9408 | | 5.1 |
| C27 | -0.1731 | -0.0230 | 1.0502 | | 7.3 |
| C28 | 0.0758 | -0.0450 | 0.6381 | | 3.4 |
| C32 | 0.7428 | 0.7399 | 0.0864 | | 3.0 |
| C34 | 0.7568 | 0.6095 | 0.1016 | | 2.3 |
| C35 | 0.5568 | 0.5146 | 0.1448 | | 2.4 |
| C36 | 0.4588 | 0.5359 | 0.1721 | | 2.4 |
| C37 | 0.4835 | 0.6186 | 0.1635 | | 2.8 |
| C39 | 0.6401 | 0.5854 | 0.1233 | | 2.3 |
| C41 | 0.9441 | 0.5878 | 0.0821 | | 2.7 |
| C46 | 1.0342 | 0.6090 | 0.1737 | | 2.8 |
| C45 | 1.1486 | 0.6299 | 0.1504 | | 3.4 |
| C44 | 1.1709 | 0.6296 | 0.0399 | | 3.1 |
| C43 | 1.0813 | 0.6114 | -0.0503 | | 2.4 |
| C42 | 0.9669 | 0.5895 | -0.0289 | | 2.4 |
| C47 | 1.1100 | 0.6117 | -0.1674 | | 2.6 |
| C49 | 1.0702 | 0.6496 | -0.3586 | | 3.4 |
| C50 | 1.1769 | 0.7051 | -0.3955 | | 7.2 |
| C51 | 1.0631 | 0.7214 | -0.4142 | | 6.4 |
| C53 | 0.3530 | 0.4807 | 0.2105 | | 2.7 |
| C55 | 0.2196 | 0.4669 | 0.3526 | | 3.8 |
| C29 | 0.5327 | 0.1215 | 0.7946 | | 4.2 |
| C56' | 0.2211 | 0.4943 | 0.4684 | .3 | 6.6 |
| C56 | 0.2588 | 0.4279 | 0.4564 | .7 | 5.1 |
| C57 | 0.3287 | 0.4873 | 0.5516 | | 8.5 |
| C58 | 0.5668 | 0.4308 | 0.1444 | | 3.1 |
| C59 | 1.0073 | 0.6093 | 0.2945 | | 4.2 |
| H31 | 0.4308 | 0.2251 | 0.5859 | | 3.4 |
| H101 | 0.2931 | 0.0142 | 0.6247 | | 3.3 |
| H181 | 0.5061 | 0.1747 | 0.2849 | | 3.3 |
| H331 | 0.8968 | 0.7077 | 0.0670 | | 3.4 |
| H401 | 0.7829 | 0.4980 | 0.1201 | | 3.5 |
| H481 | 1.0012 | 0.6813 | -0.2112 | | 3.5 |
| H541 | 0.3604 | 0.5746 | 0.3309 | | 3.6 |
| H241 | -0.1100 | 0.0910 | 0.8551 | | 4.7 |

| | | | | | |
|---|---|---|---|---|---|
| Occupancy is 1 unless otherwise noted | | | | | |

## Claims

1. A crystalline form of 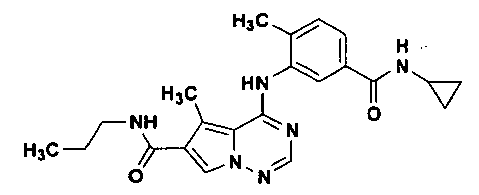 in the form of a salt thereof.

2. Form N-1 of crystalline hydrochloric acid salt of the free base of the structure 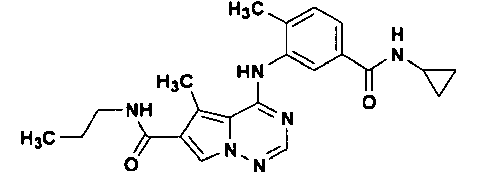 which is **characterized by** unit cell parameters substantially equal to the following:
**Cell dimensions from single crystal:**
a = 22.50(1) Å
b= 14.667(8) Å
c = 14.96(1) Å
α = 90°
β = 116.78(5)°
γ = 90°
Space group C2/c
Molecules/asymmetric unit 1
wherein said crystalline form is at about -50°C.
**Cell dimensions from hybrid at RT:**
| Cell Parameter | Hybrid |
|---|---|
| a (Å) | 22.73 |
| b (Å) | 14.710 |
| c (Å) | 15.04 |
| alpha (°) | 90 |
| beta (°) | 117.13 |
| gamma (°) | 90 |
| V (Å)³ | 4475.02 |

3. The crystalline form as defined in Claim 2 as **characterized by** a powder X-ray diffraction pattern comprising the following 2θ values (CuKα λ - 1.5418 A) 8.7 ± 0.1, 12.1 ± 0.1, 13.3 ± 0.1, 13.7 ± 0.1, 14.6 ± 0.1, 17.5 ± 0.1, 18.2 ± 0.1, 21.7 ± 0.1, 22.8 ± 0.1 and 24.3 ± 0.1 at about room temperature.

4. The crystalline form as defined in Claim 2 which is **characterized by** fractional atomic coordinates substantially as listed in the following table :
**Positional Parameters and Isotropic Temperature Factors for Free Base I HCl Salt Form N-1 at -50°C**
| Name | x | y | z | B(iso) |
|---|---|---|---|---|
| CL1 | -0.1274 | 0.7316 | 0.1928 | 2.6 |
| O11 | -0.0911 | 0.3308 | 0.0631 | 3.0 |
| O25 | 0.0768 | 1.0932 | 0.1927 | 2.5 |
| N3 | -0.0789 | 0.5597 | -0.1360 | 1.4 |
| N-4 | -0.0726 | 0.6044 | -0.2111 | 2.0 |
| N6 | -0.0627 | 0.7370 | -0.1109 | 2.0 |
| N-12 | -0.1338 | 0.2956 | -0.1016 | 2.4 |
| N-16 | -0.0670 | 0.7356 | 0.0417 | 1.5 |
| N28 | 0.1170 | 0.9587 | 0.2589 | 2.6 |
| C1 | -0.0898 | 0.4443 | -0.0493 | 1.3 |
| C2 | -0.0889 | 0.4657 | -0.1398 | 1.5 |
| C5 | -0.0632 | 0.6929 | -0.1963 | 2.5 |
| C7 | -0.0689 | 0.6963 | -0.0354 | 2.2 |
| C8 | -0.0755 | 0.5942 | -0.0480 | 1.7 |
| C9 | -0.0808 | 0.5253 | 0.0083 | 1.0 |
| C10 | -0.1055 | 0.3490 | -0.0243 | 1.8 |
| C13 | -0.1599 | 0.2059 | -0.0880 | 3.3 |
| C14 | -0.2373 | 0.2200 | -0.1225 | 6.2 |
| C15 | -0.2651 | 0.1334 | -0.1018 | 8.3 |
| C17 | -0.0610 | 0.8346 | 0.0471 | 1.7 |
| C18 | -0.0007 | 0.8710 | 0.1155 | 1.1 |
| C19 | 0.0047 | 0.9629 | 0.1264 | 1.0 |
| C20 | -0.0480 | 1.0206 | 0.0738 | 2.2 |
| C21 | -0.1086 | 0.9820 | 0.0094 | 2.3 |
| C22 | -0.1188 | 0.8899 | -0.0051 | 1.8 |
| C23 | -0.0769 | 0.5314 | 0.1117 | 2.5 |
| C24 | 0.0678 | 1.0108 | 0.1954 | 2.0 |
| C27 | -0.1847 | 0.8464 | -0.0730 | 2.9 |
| C29 | 0.1795 | 1.0002 | 0.3324 | 3.0 |
| C30 | 0.2351 | 0.9973 | 0.3046 | 5.6 |
| C31 | 0.2374 | 0.9376 | 0.3874 | 4.9 |
| H61 | -0.0563 | 0.8116 | -0.1063 | 2.8 |
| H121 | -0.1406 | 0.3168 | -0.1761 | 3.4 |
| H161 | -0.0714 | 0.6963 | 0.1014 | 2.5 |
| H281 | 0.1109 | 0.8848 | 0.2553 | 3.6 |

5. The crystalline form as defined in Claim 2 which is **characterized by** the C-13 SSNMR of HCl salt Form N-1 pattern as shown in Figure 4 and by the peaks substantially as listed in the following table :
**Carbon-13 SSNMR Chemical Shifts**
| N-1 HCl |
|---|
| 3 |
| 7.3 |
| 12.9 |
| 15.2 |
| 19.4 |
| 24.6 |
| 40.5 |
| 109.8 |
| 122.3 |
| 125 |
| 127.3 |
| 128.1 |
| 131.7 |
| 136.9 |
| 142.9 |
| 147.3 |
| 162.5 |
| 167.1 |
| |
| |

6. The crystalline form as defined in Claim 2 which is **characterized by** a differential scanning calorimetry thermogram substantially in accordance with that shown in Figure 7, having an endotherm in the range from at about 125 to about 225°C.

7. The crystalline form as defined in Claim 2 which is **characterized by** a thermal gravimetric analysis curve in accordance with that shown in Figure 10 having a negligible weight loss up to about 100°C, and a weight loss of about 8.2% up to about 225°C.

8. Form N-1 methanesulfonic acid salt of the free base of the structure 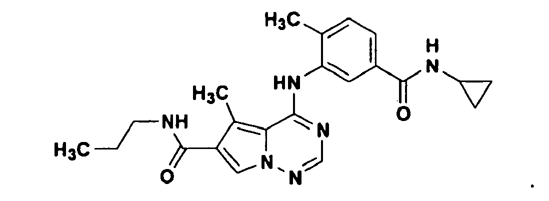 which is **characterized by** unit cell parameters substantially equal to the following:
**Cell dimensions:**
a = 9.818(1) Å
b= 11.127(1) Å
c = 13.004(1) Å
α = 97.32(1)°
β = 110.17(1)°
γ = 111.48(1)°
Space group P-1
Molecules/asymmetric unit 1
wherein the crystalline form is at about +22°C.

9. The crystalline form as defined in Claim 8 which is **characterized by** a powder X-ray diffraction pattern comprising the following 2θ values (CuKα λ = 1.5418 A) 10.7 ± 0.1, 11.7 ± 0.1, 13.3 ± 0.1, 14.0 ± 0.1, 15.2 ± 0.1, 19.8 ± 0.1, 21.0 ± 0.1, 22.0 ± 0.1, 23.0 ± 0.1 and 24.4 ± 0.1 at room temperature.

10. The crystalline form as defined in Claim 8 which is **characterized by** fractional atomic coordinates substantially as listed in the following table :
**Positional Parameters and Isotropic Temperature Factors for Free Base I MSA Salt Form N-1 at RT**
| Name | x | y | z | B(iso) |
|---|---|---|---|---|
| S1 | -0.2392 | -0.0718 | 0.2812 | 3.9 |
| O19 | -0.3458 | 0.3140 | 0.5245 | 3.9 |
| O25 | 0.1495 | 0.5491 | -0.1489 | 4.5 |
| O96 | -0.0747 | -0.0117 | 0.2977 | 7.2 |
| O97 | -0.3175 | 0.0086 | 0.2347 | 6.2 |
| O98 | -0.3273 | -0.2110 | 0.2187 | 6.2 |
| N2 | 0.0159 | 0.2025 | 0.2128 | 3.5 |
| N-4 | 0.0134 | 0.1493 | 0.0309 | 4.3 |
| N5 | 0.0858 | 0.2860 | 0.0493 | 3.4 |
| N-10 | 0.1369 | 0.4167 | 0.3376 | 3.1 |
| N20 | -0.4171 | 0.1644 | 0.3592 | 3.7 |
| N26 | 0.3707 | 0.6920 | 0.0118 | 3.9 |
| C1 | 0.0952 | 0.3375 | 0.2357 | 2.9 |
| C3 | -0.0185 | 0.1153 | 0.1135 | 4.0 |
| C6 | 0.1200 | 0.3448 | -0.0287 | 3.8 |
| C7 | 0.1918 | 0.4820 | 0.0194 | 3.3 |
| C8 | 0.2006 | 0.5099 | 0.1308 | 3.1 |
| C9 | 0.1335 | 0.3851 | 0.1494 | 2.9 |
| C11 | 0.0777 | 0.3716 | 0.4202 | 2.8 |
| C12 | -0.0828 | 0.3343 | 0.3948 | 3.0 |
| C13 | -0.1378 | 0.3039 | 0.4767 | 2.8 |
| C14 | -0.0287 | 0.3161 | 0.5846 | 3.3 |
| C15 | 0.1288 | 0.3502 | 0.6066 | 3.4 |
| C16 | 0.1867 | 0.3782 | 0.5256 | 3.2 |
| C17 | 0.3599 | 0.4139 | 0.5526 | 4.5 |
| C18 | -0.3085 | 0.2627 | 0.4558 | 3.0 |
| C21 | -0.5877 | 0.1142 | 0.3265 | 4.7 |
| C22 | -0.6743 | -0.0158 | 0.3494 | 6.0 |
| C23 | -0.6918 | -0.0137 | 0.2327 | 5.2 |
| C24 | 0.2358 | 0.5773 | -0.0471 | 3.4 |
| C27 | 0.4320 | 0.7945 | -0.0402 | 4.8 |
| C28 | 0.5627 | 0.7822 | -0.0713 | 6.1 |
| C29 | 0.5061 | 0.6520 | -0.1574 | 6.3 |
| C30 | 0.2560 | 0.6437 | 0.2098 | 4.3 |
| C99 | -0.2317 | -0.0630 | 0.4193 | 5.7 |
| H21 | -0.0223 | 0.1622 | 0.2752 | 3.7 |
| H101 | 0.2234 | 0.5211 | 0.3609 | 3.3 |
| H201 | -0.3748 | 0.1234 | 0.3044 | 4.1 |
| H261 | 0.4413 | 0.7077 | 0.1023 | 4.0 |

11. The crystalline form as defined in Claim 8 which is **characterized by** a differential scanning calorimetry thermogram substantially in accordance with that shown in Figure 9, having an endotherm with peak onset at about 216°C.

12. The crystalline form as defined in Claim 8 which is **characterized by** a thermal gravimetric analysis curve in accordance with that shown in Figure 12 having a negligible weight loss up to about 150°C.

13. The crystalline form as defined in Claim 8 which is **characterized by** the C-13 SSNMR of Form N-1 MSA salt of the free base pattern shown in Figure 6 and by the peaks substantially as listed in the following table :
**Carbon-13 SSNMR Chemical Shifts**
| N-1 MSA |
|---|
| 6 |
| 8.8 |
| 10.4 |
| 14.3 |
| 20.4 |
| 24.4 |
| 40.3 |
| 110.3 |
| 123.5 |
| 126 |
| 126.9 |
| 129.8 |
| 132.3 |
| 135.6 |
| 138.1 |
| 139.2 |
| 148.8 |
| 163.1 |
| 172.6 |

14. Form N-4 hydrochloric acid salt of the free base of the structure 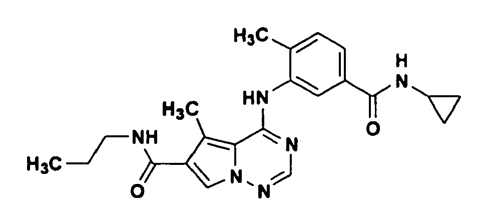 which is **characterized by** unit cell parameters substantially equal to the following:
**Cell dimensions:**
a = 20.9498(5) Å
b= 13.8719(3) Å
c = 7.9133(2) Å
α = 90°
β = 100.052(1)°
γ = 90°
Space group P2₁/n
Molecules/asymmetric unit 1
wherein said crystalline form is at about +22°C.

15. The crystalline form as defined in Claim 14 which is **characterized by** fractional atomic coordinates substantially as listed in the following table :
**Positional Parameters and Isotropic Temperature Factors for Free Base I HCl Salt Form N-4 at RT**
| Name | x | y | z | B(iso) |
|---|---|---|---|---|
| CL | 0.0341 | 0.7162 | 0.4308 | 5.5 |
| O1 | 0.1111 | 1.0617 | 0.3458 | 5.4 |
| O2 | -0.2021 | 0.5613 | -0.6888 | 6.5 |
| N-1 | -0.0710 | 0.5099 | 0.0187 | 5.0 |
| N2 | -0.0544 | 0.6737 | 0.0953 | 4.4 |
| N3 | -0.0913 | 0.5440 | -0.1460 | 4.2 |
| N-4 | -0.0762 | 0.8037 | -0.0894 | 4.3 |
| N5 | 0.1228 | 0.9001 | 0.3778 | 4.5 |
| N6 | -0.1487 | 0.4216 | -0.6361 | 5.1 |
| C1 | -0.0540 | 0.5768 | 0.1292 | 4.9 |
| C2 | -0.0752 | 0.7092 | -0.0651 | 3.8 |
| C3 | -0.1215 | 0.6429 | -0.3703 | 4.0 |
| C4 | -0.1318 | 0.5462 | -0.4222 | 4.0 |
| C5 | -0.1137 | 0.4874 | -0.2809 | 4.6 |
| C6 | -0.0956 | 0.6414 | -0.1958 | 3.7 |
| C7 | -0.0604 | 0.8753 | 0.0446 | 3.9 |
| C8 | 0.0035 | 0.8873 | 0.1212 | 3.9 |
| C9 | 0.0202 | 0.9626 | 0.2399 | 3.8 |
| C10 | -0.0285 | 1.0247 | 0.2755 | 4.4 |
| C11 | -0.0924 | 1.0089 | 0.1989 | 4.6 |
| C12 | -0.1101 | 0.9344 | 0.0833 | 4.3 |
| C13 | 0.0882 | 0.9795 | 0.3247 | 4.3 |
| C14 | 0.1885 | 0.9076 | 0.4700 | 4.8 |
| C15 | 0.2433 | 0.8987 | 0.3757 | 7.1 |
| C16 | 0.2285 | 0.8180 | 0.4863 | 7.3 |
| C17 | -0.1632 | 0.5116 | -0.5947 | 4.6 |
| C18 | -0.1796 | 0.3769 | -0.7945 | 5.8 |
| C19 | -0.1408 | 0.2925 | -0.8428 | 7.6 |
| C20 | -0.1734 | 0.2467 | -1.0053 | 9.0 |
| C21 | -0.1342 | 0.7314 | -0.4822 | 5.3 |
| C22 | -0.1802 | 0.9169 | 0.0035 | 6.0 |
| H11 | -0.0396 | 0.5549 | 0.2601 | 5.9 |
| H21 | -0.0375 | 0.7237 | 0.1998 | 5.4 |
| H41 | -0.0910 | 0.8303 | -0.2198 | 5.3 |
| H51 | -0.1153 | 0.4087 | -0.2785 | 5.6 |
| H51 | 0.1006 | 0.8297 | 0.3463 | 5.5 |
| H61 | -0.1122 | 0.3809 | -0.5485 | 4.0 |
| H81 | 0.0407 | 0.8396 | 0.0891 | 4.8 |
| H101 | -0.0165 | 1.0872 | 0.3579 | 5.4 |
| H111 | -0.1294 | 1.0551 | 0.2302 | 5.4 |
| H141 | 0.1928 | 0.9796 | 0.5314 | 5.9 |

16. The crystalline form as defined in Claim 14 having an X-ray powder diffraction comprising the following 2θ values (CuKα λ= 1.5418 A) 8.6 ± 0.1, 10.7 ± 0.1, 11.4 ± 0.1, 12.8 ± 0.1, 14.4 ± 0.1, 15.6 ± 0.1, 16.9 ± 0.1, 18.3 ± 0.1, 20.0 ± 0.1 and 23.4 ± 0.1, at about room temperature.

17. The crystalline form as defined in Claim 14 which is **characterized by** a differential scanning calorimetry thermogram substantially in accordance with that shown in Figure 8, having an endotherm in the range from about 130 to about 220°C (variable).

18. The crystalline form as defined in Claim 14 which is **characterized by** a thermal gravimetric analysis curve in accordance with that shown in Figure 11 having a negligible weight loss up to about 125°C.

19. The crystalline form as defined in Claim 14 which is **characterized by** the C-13 SSNMR of Form N-4 free base pattern shown in Figure 5 and by the peaks substantially as listed in the following table :
**Carbon-13 SSNMR Chemical Shifts for Each of the Forms**
| N-4 HCl |
|---|
| 4.1 |
| 6.3 |
| 12.4 |
| 14.4 |
| 17.1 |
| 21 |
| 23.5 |
| 43.1 |
| 110 |
| 122.4 |
| 125.1 |
| 128.1 |
| 129.1 |
| 130.4 |
| 131.7 |
| 134.7 |
| 135.7 |
| 138.9 |
| 147.1 |
| 163.2 |

20. The crystalline form as defined in Claim 14 having an average particle size distribution of 95% < 60 µm.

21. A process for preparing the hydrochloric acid salt of 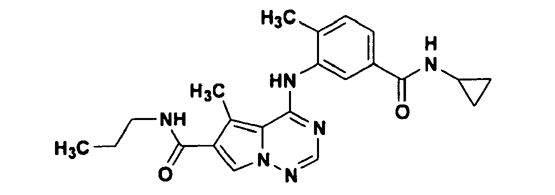 in the form of Form N-1 crystals as defined in Claim 2, which comprises
a) providing a free base having the structure 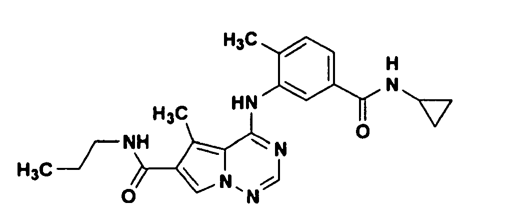 suspended or mixed in an organic solvent;
b) reacting the free base with an aqueous solution of hydrochloric acid;
c) seeding the reaction mixture with Form N-1 crystals of a hydrochloric salt of said free base as defined in Claim 2; and
d) recovering hydrochloric acid salt in the form of Form N-1 crystals.

22. The process as defined in Claim 21 wherein the free base is mixed with:
a) tetrahydrofuran; or
b) N,N-dimethylformamide and acetone is mixed with the seeded reaction mixture.

23. The process as defined in Claim 22 wherein the seeds of Form N-1 crystals of the hydrochloride salt are prepared by:
a) suspending the free base in an organic solvent;
b) reacting the free base with aqueous HCl acid; and
c) recovering seeds of Form N-1 crystals.

24. A process for preparing the Form N-1 methanesulfonic acid salt of a compound having the structure 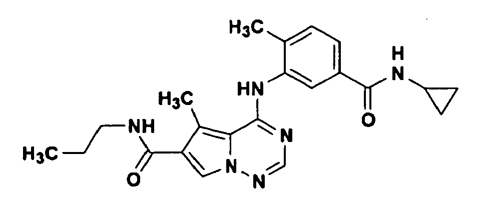 as defined in Claim 8, which comprises
a) providing a solution of a free base having the structure 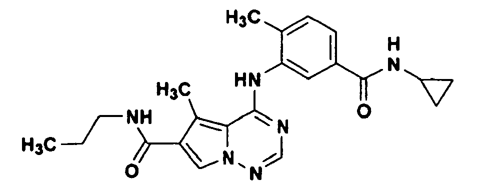 in an organic solvent;
b) reacting the free base with methanesulfonic acid;
c) seeding the reaction mixture with crystals of Form N-1 methanesulfonic acid salt of said free base as defined in Claim 10; and
d) recovering crystals of Form N-1 methanesulfonic salt.

25. The process as defined in Claim 24 wherein the organic solvent is N,N-dimethylformamide, isopropyl alcohol or ethanol.

26. The process as defined in Claim 24 wherein the organic solvent is N,N-dimethylformamide, including the step of adding acetone to the reaction mixture prior to seeding.

27. The process as defined in Claim 24 wherein the seeds of Form N-1 crystals are prepared by:
a) suspending the free base in isopropyl alcohol, ethanol, ethyl acetate or acetonitrile;
b) reacting the free base with methanesulfonic acid; and
c) recovering Form N-1 crystals.

28. A process for preparing the hydrochloric acid salt of the free base of the structure 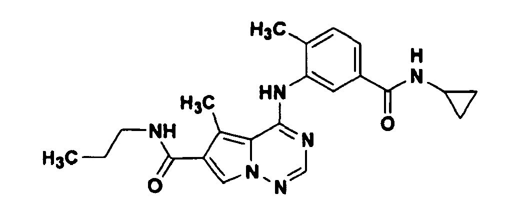 in the form of Form N-4 crystals as defined in Claim 14, which comprises
a) providing a slurry of free base of the structure 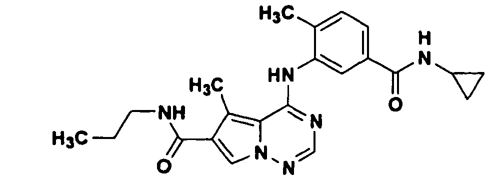 in formic acid and methylethyl ketone or formic acid and acetone;
b) adding an aqueous hydrochloric acid solution to the slurry of step a);
c) optionally filtering the resulting reaction mixture;
d) adding the filtered reaction mixture to a slurry of seeds of Form N-4 crystals of the hydrochloride salt of said free base as defined in Claim 14 in methylethyl ketone or acetone, employing the same solvent as employed in step a); and
e) recovering the hydrochloric acid salt of the free base in the form of Form N-4 crystals.

29. A process for preparing the hydrochloric acid salt of the free base of the structure 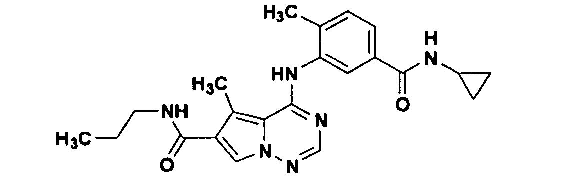 in the form of Form N-4 crystals as defined in Claim 14, which comprises
a) providing a mixture of free base of the structure 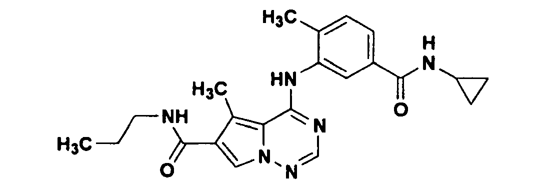 in formic acid and acetone or formic acid and MEK;
b) adding an aqueous hydrochloric acid solution to the mixture of step a);
c) adding seeds of Form N-4 crystals of the hydrochloride salt of said free base as defined in Claim 14 and acetone or MEK to the reaction mixture of step b); and
d) recovering the hydrochloric acid salt of the free base in the form of Form N-4 crystals.

30. A process for preparing the hydrochloric acid salt of a free base of the structure 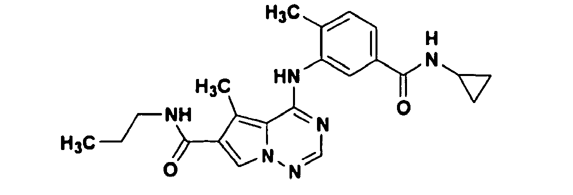 in the form of N-4 crystals as defined in Claim 14, which comprises
a) providing a solution of a free base of the structure 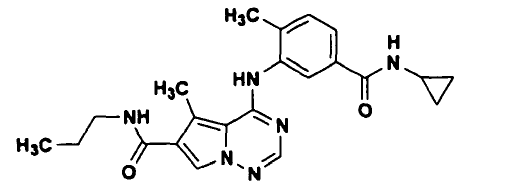 dissolved in N,N-dimethylacetamide at a temperature within the range from about 60 to about 65°C;
b) providing a solution of aqueous hydrochloric acid and cooled acetone or MEK;
c) adding into the acetone/HCl solution seeds of Form N-4 hydrochloric acid salt of the free base as defined in Claim 14;
d) adding the solution of free base in N,N-dimethylacetamide from step a), maintained at a temperature within the range from about 50 to about 65°C, into the seeded cold acetone or MEK/HCl solution of step c) while stirring, to form a slurry; and
e) recovering Form N-4 crystals of the hydrochloric acid salt of the free base.

31. A process for preparing the hydrochloric acid salt of a free base of the structure 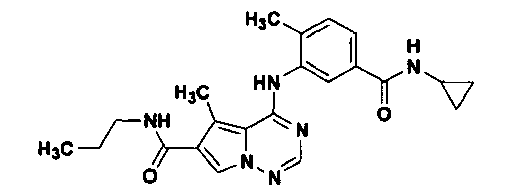 in the form of N-4 crystals as defined in Claim 14,
which comprises
a) providing a solution of a free base of the structure 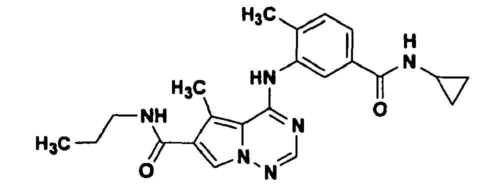 dissolved in N,N-dimethylacetamide;
b) adding a solution of aqueous hydrochloric acid to the solution of step a) to form a solution;
c) adding into the solution of step b) seeds of Form N-4 hydrochloric acid salt of the free base as defined in Claim 14 and acetone or MEK; and
d) recovering Form N-4 crystals of the hydrochloric acid salt of the free base.

32. A process for preparing the hydrochloric acid salt of 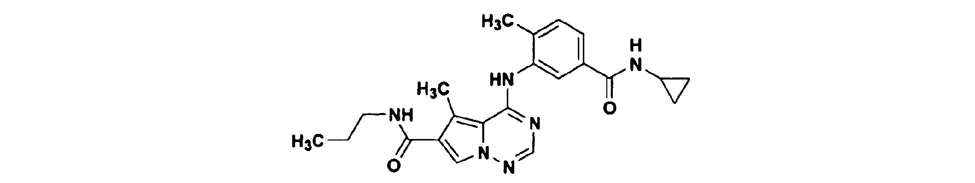 in the form of Form N-4 crystals as defined in Claim 14, which comprises
a) providing a free base having the structure 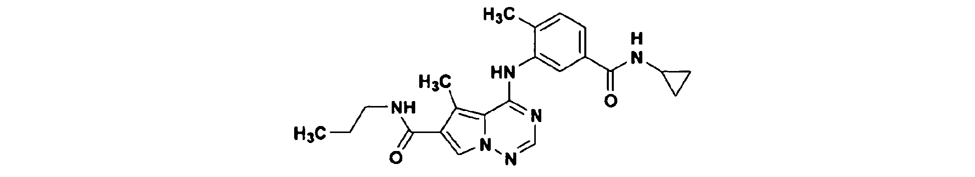 suspended in an organic solvent which is tetrahydrofuran, ethanol or acetone;
b) reacting the free base with an aqueous solution of hydrochloric acid;
c) seeding the reaction mixture with Form N-4 crystals of a hydrochloric salt of said free base as defined in Claim 14; and
d) recovering hydrochloric acid salt in the form of Form N-4 crystals.

33. A process for preparing the Form N-4 hydrochloric acid salt of a compound having the structure 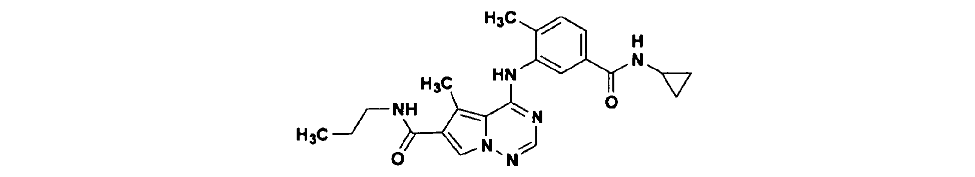 as defined in Claim 14, which comprises
a) providing a solution of a free base having the structure 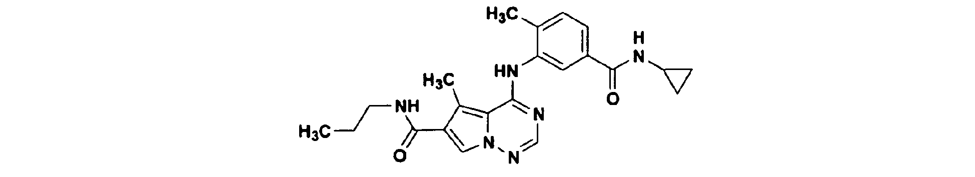 in an organic solvent which is acetonitrile, THF, ethanol or acetone;
b) treating the free base with seeds of crystals of Form N-4 hydrochloric acid salt of said free base as defined in Claim 14; and
c) recovering crystals of Form N-4 hydrochloric acid salt.

34. A process for preparing the hydrochloric acid salt of a free base of the structure 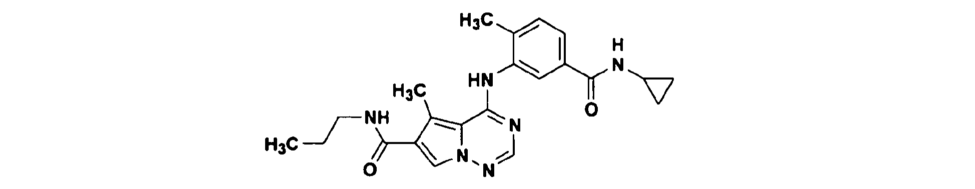 in the form of N-4 crystals as defined in Claim 14, which comprises
a) providing a solution of a free base of the structure 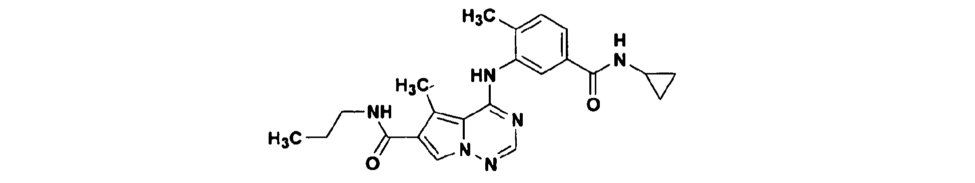 dissolved in formic acid;
b) providing a solution of aqueous hydrochloric acid and acetone or MEK;
c) adding into the acetone or MEK/HCl solution seeds of Form N-4 hydrochloric acid salt of the free base as defined in Claim 14;
d) adding the solution of free base in formic acid from step a), maintained at a temperature within the range from about 15 to about 25°C, into the seeded cold acetone or MEK/HCl solution of step c) while stirring, to form a slurry; and
e) recovering Form N-4 crystals of the hydrochloric acid salt of the free base.

35. A process for preparing the hydrochloric acid salt of free base of the structure 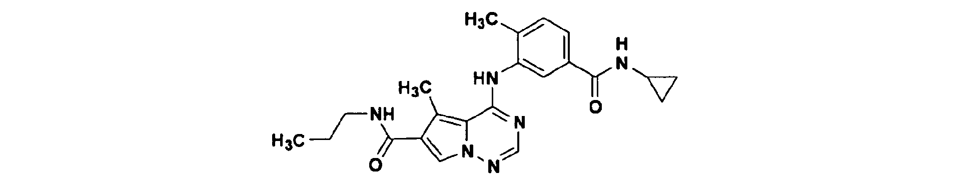 in the form of Form N-4 crystals as defined in Claim 14, which comprises
a) providing a suspension or solution of free base in N,N-dimethylformamide;
b) adding a solution of aqueous hydrochloric acid to the suspension of step a) to form a solution;
c) adding acetone or MEK to the solution of step b);
d) adding to the mixture of step c) seeds of Form N-4 hydrochloric acid salt of the free base as defined in Claim 14; and
e) recovering Form N-4 crystals of the hydrochloric acid salt of the free base.

36. A process for preparing the hydrochloric acid salt of the free base of the structure 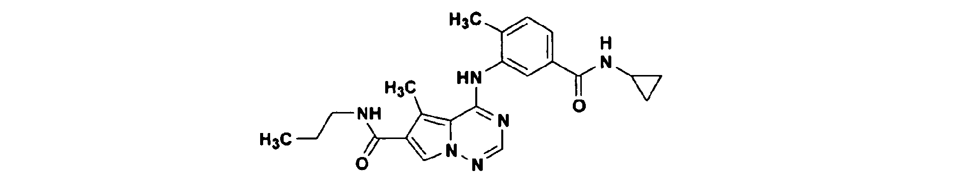 in the form of N-4 crystals as defined in Claim 14, which comprises
a) providing a slurry of a free base of the structure I dissolved in N,N-dimethylformamide, N,N-dimethylformamide/acetone or N,N-dimethylformamide/MEK;
b) adding a solution of aqueous hydrochloric acid and acetone or MEK to the slurry of step a) to form a solution;
c) optionally filtering off insoluble solids from the solution of step b);
d) adding seeds of Form N-4 hydrochloric acid salt of the free base as defined in Claim 18 as a slurry in acetone to the solution obtained in step c); and
e) recovering Form N-4 crystals of the hydrochloric acid salt of the free base I.

37. Form N-1 hydrochloric acid salt of the free base 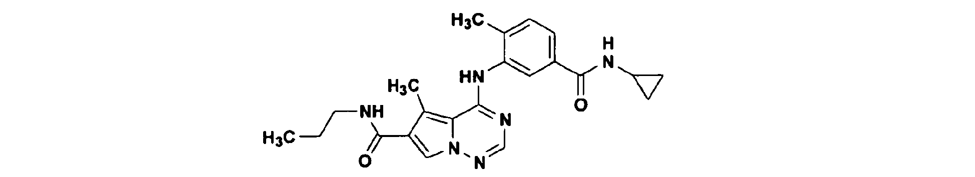 prepared as defined by the process of Claim 21.

38. Form N-1 methanesulfonic acid salt of the free base 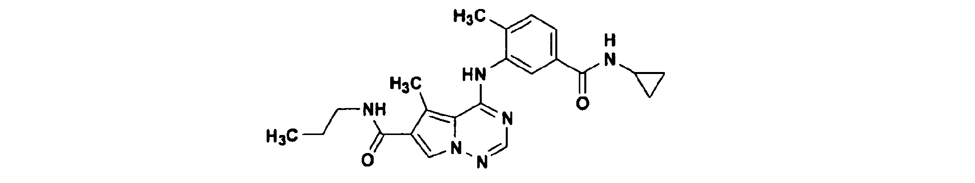 prepared as defined by the process of Claim 24.

39. Form N-4 hydrochloric acid salt of the free base 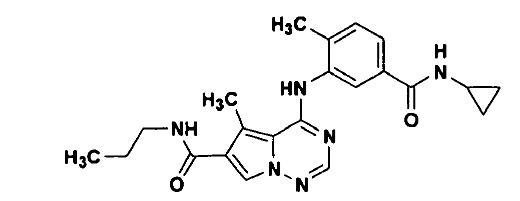 prepared as defined by the process of Claim 28.

40. Form N-4 hydrochloric acid salt of the free base 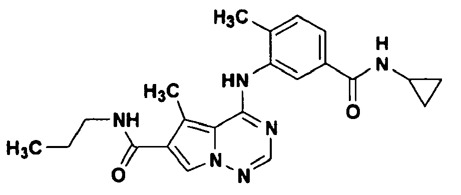 prepared as defined by the process of Claim 29.

41. Form N-4 hydrochloric acid salt of the structure 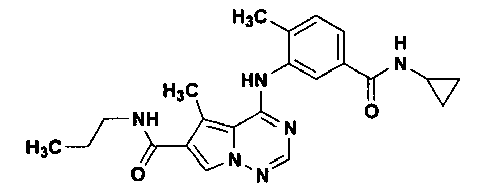 prepared as defined by the process of Claim 30.

42. Form N-4 hydrochloric acid salt of the structure 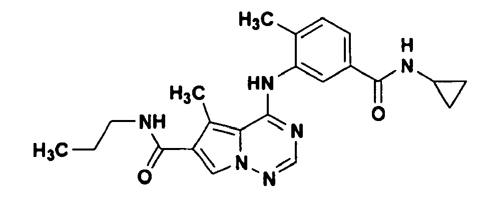 prepared as defined by the process of Claim 31.

43. Form N-4 hydrochloric acid salt of the structure 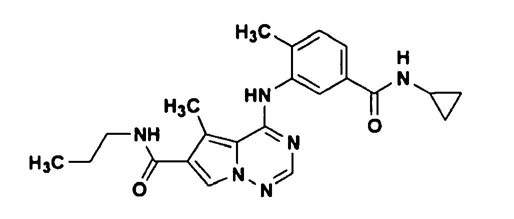 prepared as defined by the process of Claim 32.

44. Form N-4 hydrochloric acid salt of the structure 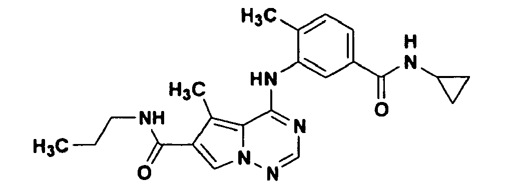 prepared as defined by the process of Claim 33.

45. Form N-4 hydrochloric acid salt of the structure 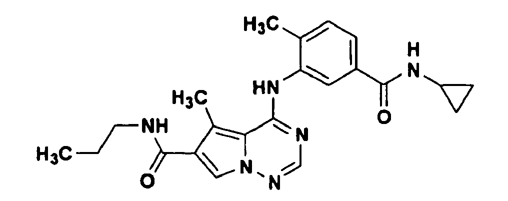 prepared as defined by the process of Claim 34.

46. Form N-4 hydrochloric acid salt of the structure 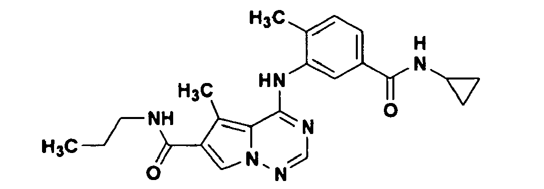 prepared as defined by the process of Claim 35.

47. Form N-4 hydrochloric acid salt of the structure 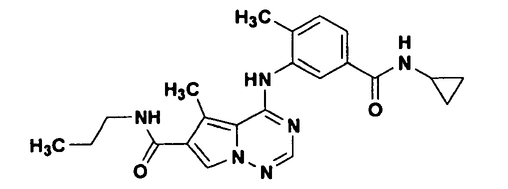 prepared as defined by the process of Claim 36.

48. A pharmaceutical composition comprising at least one compound according to Claim 1 and a pharmaceutically-acceptable carrier or diluent.

49. A pharmaceutical composition comprising at least one compound according to Claim 2 and a pharmaceutically-acceptable carrier or diluent.

50. A pharmaceutical composition comprising at least one compound according to Claim 14 and a pharmaceutically acceptable carrier or diluent.

51. The use of a compound of any one of claims 1 to 20, or 37 to 47 for preparing a pharmaceutical composition for treating an inflammatory disorder.

52. The use of claim 51 in which the inflammatory disorder is selected from asthma, adult respiratory distress syndrome, chronic obstructive pulmonary disease, chronic pulmonary inflammatory disease, diabetes, inflammatory bowel disease, osteoporosis, psoriasis, graft vs. host rejection, atherosclerosis, and arthritis including rheumatoid arthritis, psoriatic arthritis, traumatic arthritis, rubella arthritis, gouty arthritis and osteoarthritis.

53. A compound according to any one of claims 1 to 20 for use in a method of treating an inflammatory disorder.

54. The compound of claim 53, wherein the inflammatory disorder is selected from asthma, adult respiratory distress syndrome, chronic obstructive pulmonary disease, chronic pulmonary inflammatory disease, diabetes, inflammatory bowel disease, osteoporosis, psoriasis, graft vs. host rejection, atherosclerosis, and arthritis including rheumatoid arthritis, psoriatic arthritis, traumatic arthritis, rubella arthritis, gouty arthritis and osteoarthritis.

## Patentansprüche

1. Kristalline Form von 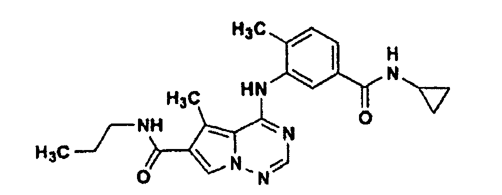 in der Form eines Salzes davon.

2. Form N-1 des kristallinen Chlorwasserstoffsäure-Salzes der freien Base der Struktur 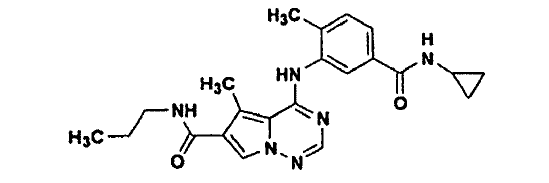 welche **gekennzeichnet ist durch** Parameter der Elementarzelle im wesentlichen gleich den folgenden:
Zellabmessungen vom Einkristall:
a = 22,50(1) Å
b = 14,667(8) Å
c = 14,96(1) Å
α = 90°
β = 116,78(5)°
γ = 90°
Raumgruppe C2/c
Moleküle/asymmetrische Einheit 1
wobei die kristalline Form bei etwa -50°C ist.
**Zellabmessungen vom Hybrid bei RT:**
| Zellparameter | Hybrid |
|---|---|
| a (Å) | 22,73 |
| b (Å) | 14,710 |
| c (Å) | 15,04 |
| alpha (°) | 90 |
| beta (°) | 117,13 |
| gamma (°) | 90 |
| V (Å)³ | 4475,02 |

3. Kristalline Form, wie definiert in Anspruch 2, wie **gekennzeichnet durch** ein Pulverröntgenbeugungsdiagramm, umfassend die folgenden 2θ-Werte (CuKα λ = 1,5418 Å) 8,7±0,1, 12,1±0,1, 13,3±0,1, 13,7±0,1, 14,6±0,1, 17,5±0,1, 18,2±0,1, 21,7±0,1, 22,8±0,1 und 24,3±0,1 bei etwa Raumtemperatur.

4. Kristalline Form, wie definiert in Anspruch 2, welche **gekennzeichnet ist durch** gebrochene Atomkoordinaten, im wesentlichen wie aufgeführt in der folgenden Tabelle:
**Positionsparameter und isotrope Temperaturfaktoren für das HCl-Salz der freien Base I Form N-1 bei -50°C**
| Name | x | y | z | B(iso) |
|---|---|---|---|---|
| CL1 | -0.1274 | 0.7316 | 0.1928 | 2.6 |
| O11 | -0.0911 | 0.3308 | 0.0631 | 3.0 |
| O25 | 0.0768 | 1.0932 | 0.1927 | 2.5 |
| N3 | -0.0789 | 0.5597 | -0.1360 | 1.4 |
| N-4 | -0.0726 | 0.6044 | -0.2111 | 2.0 |
| N6 | -0.0627 | 0.7370 | -0.1109 | 2.0 |
| N-12 | -0.1338 | 0.2956 | -0.1016 | 2.4 |
| N-16 | -0.0670 | 0.7356 | 0.0417 | 1.5 |
| N28 | 0.1170 | 0.9587 | 0.2589 | 2.6 |
| C1 | -0.0898 | 0.4443 | -0.0493 | 1.3 |
| C2 | -0.0889 | 0.4657 | -0.1398 | 1.5 |
| C5 | -0.0632 | 0.6929 | -0.1963 | 2.5 |
| C7 | -0.0689 | 0.6963 | -0.0354 | 2.2 |
| C8 | -0.0755 | 0.5942 | -0.0480 | 1.7 |
| C9 | -0.0808 | 0.5253 | 0.0083 | 1.0 |
| C10 | -0.1055 | 0.3490 | -0.0243 | 1.8 |
| C13 | -0.1599 | 0.2059 | -0.0880 | 3.3 |
| C14 | -0.2373 | 0.2200 | -0.1225 | 6.2 |
| C15 | -0.2651 | 0.1334 | -0.1018 | 8.3 |
| C17 | -0.0610 | 0.8346 | 0.0471 | 1.7 |
| C18 | -0.0007 | 0.8710 | 0.1155 | 1.1 |
| C19 | 0.0047 | 0.9629 | 0.1264 | 1.0 |
| | | | | |
| C20 | -0.0480 | 1.0206 | 0.0738 | 2.2 |
| C21 | -0.1086 | 0.9820 | 0.0094 | 2.3 |
| C22 | -0.1188 | 0.8899 | -0.0051 | 1.8 |
| C23 | -0.0769 | 0.5314 | 0.1117 | 2.5 |
| C24 | 0.0678 | 1.0108 | 0.1954 | 2.0 |
| C27 | -0.1847 | 0.8464 | -0.0730 | 2.9 |
| C29 | 0.1795 | 1.0002 | 0.3324 | 3.0 |
| C30 | 0.2351 | 0.9973 | 0.3046 | 5.6 |
| C31 | 0.2374 | 0.9376 | 0.3874 | 4.9 |
| H61 | -0.0563 | 0.8116 | -0.1063 | 2.8 |
| H121 | -0.1406 | 0.3168 | -0.1761 | 3.4 |
| H161 | -0.0714 | 0.6963 | 0.1014 | 2.5 |
| H281 | 0.1109 | 0.8848 | 0.2553 | 3.6 |

5. Kristalline Form, wie definiert in Anspruch 2, welche **gekennzeichnet ist durch** das C-13-SSNMR-Diagramm des HCl-Salzes Form N-1, wie gezeigt in Figur 4, und **durch** die Peaks, im wesentlichen wie aufgeführt in der folgenden Tabelle:
**Positionsparameter und isotrope Temperaturfaktoren für das HCl-Salz der freien Base I Form N-1 bei -50°C**
| N-1 HCl |
|---|
| 3 |
| 7.3 |
| 12.9 |
| 15.2 |
| 19.4 |
| 24.6 |
| 40.5 |
| 109.8 |
| 122.3 |
| 125 |
| 127.3 |
| 128.1 |
| 131.7 |
| 136.9 |
| 142.9 |
| 147.3 |
| 162.5 |
| 167.1 |
| |
| |

6. Kristalline Form, wie definiert in Anspruch 2, welche **gekennzeichnet ist durch** ein Differentialscanningkalorimetrie-Thermogramm im wesentlichen in Übereinstimmung mit dem, gezeigt in Figur 7, mit einer Endotherme in dem Bereich von bei etwa 125 bis etwa 225°C.

7. Kristalline Form, wie definiert in Anspruch 2, welche **gekennzeichnet ist durch** eine Kurve der thermogravimetrischen Analyse in Übereinstimmung mit der, gezeigt in Figur 10, mit einem vernachlässigbaren Gewichtsverlust bis zu etwa 100°C und einem Gewichtsverlust von etwa 8,2% bis zu etwa 225°C.

8. Methansulfonsäuresalz der Form N-1 der freien Base der Struktur 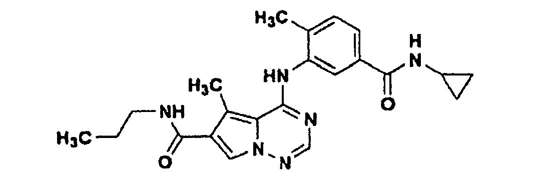 welches **gekennzeichnet ist durch** Parameter der Elementarzelle im wesentlichen gleich den folgenden:
Zellabmessungen:
a = 9,818(1) Å
b = 11,127(1) Å
c = 13,004(1) Å
α = 97,32(1)°
β = 110,17(1)°
γ = 111,48(1)°
Raumgruppe P-1
Moleküle/asymmetrische Einheit 1
wobei die kristalline Form bei etwa +22°C ist.

9. Kristalline Form, wie definiert in Anspruch 8, welche **gekennzeichnet ist durch** ein Pulverröntgenbeugungsdiagramm, umfassend die folgenden 2θ-Werte (CuKα λ = 1,5418 Å) 10,7±0,1, 11,7±0,1, 13,3±0,1, 14,0±0,1, 15,2±0,1, 19,8±0,1, 21,0±0,1, 22,0±0,1, 23,0±0,1 und 24,4±0,1 bei Raumtemperatur.

10. Kristalline Form, wie definiert in Anspruch 8, welche **gekennzeichnet ist durch** gebrochene Atomkoordinaten, im wesentlichen wie aufgeführt in der folgenden Tabelle:
**Positionsparameter und isotrope Temperaturfaktoren für das MSA-Salz Form N-1 der freien Base I bei RT**
| Name | x | y | z | B(iso) |
|---|---|---|---|---|
| S1 | -0.2392 | -0.0718 | 0.2812 | 3.9 |
| O19 | -0.3458 | 0.3140 | 0.5245 | 3.9 |
| O25 | 0.1495 | 0.5491 | -0.1489 | 4.5 |
| O96 | -0.0747 | -0.0117 | 0.2977 | 7.2 |
| O97 | -0.3175 | 0.0086 | 0.2347 | 6.2 |
| O98 | -0.3273 | -0.2110 | 0.2187 | 6.2 |
| N2 | 0.0159 | 0.2025 | 0.2128 | 3.5 |
| N-4 | 0.0134 | 0.1493 | 0.0309 | 4.3 |
| N5 | 0.0858 | 0.2860 | 0.0493 | 3.4 |
| N-10 | 0.1369 | 0.4167 | 0.3376 | 3.1 |
| N20 | -0.4171 | 0.1644 | 0.3592 | 3.7 |
| N26 | 0.3707 | 0.6920 | 0.0118 | 3.9 |
| C1 | 0.0952 | 0.3375 | 0.2357 | 2.9 |
| C3 | -0.0185 | 0.1153 | 0.1135 | 4.0 |
| C6 | 0.1200 | 0.3448 | -0.0287 | 3.8 |
| C7 | 0.1918 | 0.4820 | 0.0194 | 3.3 |
| C8 | 0.2006 | 0.5099 | 0.1308 | 3.1 |
| C9 | 0.1335 | 0.3851 | 0.1494 | 2.9 |
| C11 | 0.0777 | 0.3716 | 0.4202 | 2.8 |
| C12 | -0.0828 | 0.3343 | 0.3948 | 3.0 |
| C13 | -0.1378 | 0.3039 | 0.4767 | 2.8 |
| C14 | -0.0287 | 0.3161 | 0.5846 | 3.3 |
| C15 | 0.1288 | 0.3502 | 0.6066 | 3.4 |
| C16 | 0.1867 | 0.3782 | 0.5256 | 3.2 |
| C17 | 0.3599 | 0.4139 | 0.5526 | 4.5 |
| C18 | -0.3085 | 0.2627 | 0.4558 | 3.0 |
| C21 | -0.5877 | 0.1142 | 0.3265 | 4.7 |
| C22 | -0.6743 | -0.0158 | 0.3494 | 6.0 |
| C23 | -0.6918 | -0.0137 | 0.2327 | 5.2 |
| C24 | 0.2358 | 0.5773 | -0.0471 | 3.4 |
| C27 | 0.4320 | 0.7945 | -0.0402 | 4.8 |
| C28 | 0.5627 | 0.7822 | -0.0713 | 6.1 |
| C29 | 0.5061 | 0.6520 | -0.1574 | 6.3 |
| C30 | 0.2560 | 0.6437 | 0.2098 | 4.3 |
| C99 | -0.2317 | -0.0630 | 0.4193 | 5.7 |
| H21 | -0.0223 | 0.1622 | 0.2752 | 3.7 |
| H101 | 0.2234 | 0.5211 | 0.3609 | 3.3 |
| H201 | -0.3748 | 0.1234 | 0.3044 | 4.1 |
| H261 | 0.4413 | 0.7077 | 0.1023 | 4.0 |

11. Kristalline Form, wie definiert in Anspruch 8, welche **gekennzeichnet ist durch** ein Differentialscanningkalorimetrie-Thermogramm im wesentlichen in Übereinstimmung mit dem, gezeigt in Figur 9, mit einer Endotherme mit Beginn des Peaks bei etwa 216°C.

12. Kristalline Form, wie definiert in Anspruch 8, welche **gekennzeichnet ist durch** eine Kurve der thermogravimetrischen Analyse in Übereinstimmung mit der, gezeigt in Figur 12, mit einem vernachlässigbaren Gewichtsverlust bis zu etwa 150°C.

13. Kristalline Form, wie definiert in Anspruch 8, welche **gekennzeichnet ist durch** das C-13-SSNMR-Diagramm des MSA-Salzes der Form N-1 der freien Base, gezeigt in Figur 6, und **durch** die Peaks, im wesentlichen wie aufgeführt in der folgenden Tabelle:
**Kohlenstoff-13-SSNMR chemische Verschiebungen**
| N-1 MSA |
|---|
| 6 |
| 8.8 |
| 10.4 |
| 14.3 |
| 20.4 |
| 24.4 |
| 40.3 |
| 110.3 |
| 123.5 |
| 126 |
| 126.9 |
| 129.8 |
| 132.3 |
| 135.6 |
| 138.1 |
| 139.2 |
| 148.8 |
| 163.1 |
| 172.6 |

14. Chlorwasserstoffsäure-Salz der Form N-4 der freien Base der Struktur 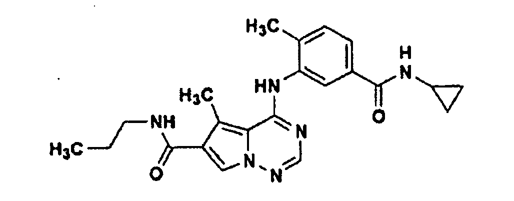 welches **gekennzeichnet ist durch** Parameter der Elementarzelle im wesentlichen gleich den folgenden:
Zellabmessungen:
a = 20,9498(5) Å
b = 13,8719(3) Å
c = 7,9133(2) Å
α = 90°
β = 100,052(1)°
γ = 90°
Raumgruppe P2₁/n
Moleküle/asymmetrische Einheit 1
wobei die kristalline Form bei etwa +22°C ist.

15. Kristalline Form, wie definiert in Anspruch 14, welche **gekennzeichnet ist durch** gebrochene Atomkoordinaten, im wesentlichen wie aufgeführt in der folgenden Tabelle:
**Positionsparameter und isotrope Temperaturfaktoren für das HCl-Salz der freien Base I Form N-4 bei RT**
| Name | x | y | z | B(iso) |
|---|---|---|---|---|
| CL | 0.0341 | 0.7162 | 0.4308 | 5.5 |
| O1 | 0.1111 | 1.0617 | 0.3458 | 5.4 |
| 02 | -0.2021 | 0.5613 | -0.6888 | 6.5 |
| N-1 | -0.0710 | 0.5099 | 0.0187 | 5.0 |
| N2 | -0.0544 | 0.6737 | 0.0953 | 4.4 |
| N3 | -0.0913 | 0.5440 | -0.1460 | 4.2 |
| N-4 | -0.0762 | 0.8037 | -0.0894 | 4.3 |
| N5 | 0.1228 | 0.9001 | 0.3778 | 4.5 |
| N6 | -0.1487 | 0.4216 | -0.6361 | 5.1 |
| C1 | -0.0540 | 0.5768 | 0.1292 | 4.9 |
| C2 | -0.0752 | 0.7092 | -0.0651 | 3.8 |
| C3 | -0.1215 | 0.6429 | -0.3703 | 4.0 |
| C4 | -0.1318 | 0.5462 | -0.4222 | 4.0 |
| C5 | -0.1137 | 0.4874 | -0.2809 | 4.6 |
| C6 | -0.0956 | 0.6414 | -0.1958 | 3.7 |
| C7 | -0.0604 | 0.8753 | 0.0446 | 3.9 |
| C8 | 0.0035 | 0.8873 | 0.1212 | 3.9 |
| C9 | 0.0202 | 0.9626 | 0.2399 | 3.8 |
| C10 | -0.0285 | 1.0247 | 0.2755 | 4.4 |
| C11 | -0.0924 | 1.0089 | 0.1989 | 4.6 |
| C12 | -0.1101 | 0.9344 | 0.0833 | 4.3 |
| C13 | 0.0882 | 0.9795 | 0.3247 | 4.3 |
| C14 | 0.1885 | 0.9076 | 0.4700 | 4.8 |
| C15 | 0.2433 | 0.8987 | 0.3757 | 7.1 |
| C16 | 0.2285 | 0.8180 | 0.4863 | 7.3 |
| C17 | -0.1632 | 0.5116 | -0.5947 | 4.6 |
| C18 | -0.1796 | 0.3769 | -0.7945 | 5.8 |
| C19 | -0.1408 | 0.2925 | -0.8428 | 7.6 |
| C20 | -0.1734 | 0.2467 | -1.0053 | 9.0 |
| C21 | -0.1342 | 0.7314 | -0.4822 | 5.3 |
| C22 | -0.1802 | 0.9169 | 0.0035 | 6.0 |
| H11 | -0.0396 | 0.5549 | 0.2601 | 5.9 |
| H21 | -0.0375 | 0.7237 | 0.1998 | 5.4 |
| H41 | -0.0910 | 0.8303 | -0.2198 | 5.3 |
| H51 | -0.1153 | 0.4087 | -0.2785 | 5.6 |
| H51 | 0.1006 | 0.8297 | 0.3463 | 5.5 |
| H61 | -0.1122 | 0.3809 | -0.5485 | 4.0 |
| H81 | 0.0407 | 0.8396 | 0.0891 | 4.8 |
| H101 | -0.0165 | 1.0872 | 0.3579 | 5.4 |
| H111 | -0.1294 | 1.0551 | 0.2302 | 5.4 |
| H 141 | 0.1928 | 0.9796 | 0.5314 | 5.9 |

16. Kristalline Form, wie definiert in Anspruch 14, mit einer Röntgenpulverbeugung, umfassend die folgenden 2θ-Werte (CuKα λ = 1,5418 Å) 8,6±0,1, 10,7±0,1, 11,4±0,1, 12,8±0,1, 14,4±0,1, 15,6±0,1, 16,9±0,1, 18,3±0,1, 20,0±0,1 und 23,4±0,1, bei etwa Raumtemperatur.

17. Kristalline Form, wie definiert in Anspruch 14, welche **gekennzeichnet ist durch** ein Differentialscanningkalorimetrie-Thermogramm im wesentlichen in Übereinstimmung mit dem, gezeigt in Figur 8, mit einer Endotherme in dem Bereich von etwa 130 bis etwa 220°C (variabel).

18. Kristalline Form, wie definiert in Anspruch 14, welche **gekennzeichnet ist durch** eine Kurve der thermogravimetrischen Analyse in Übereinstimmung mit der, gezeigt in Figur 11, mit einem vernachlässigbaren Gewichtsverlust bis zu etwa 125°C.

19. Kristalline Form, wie definiert in Anspruch 14, welche **gekennzeichnet ist durch** das C-13-SSNMR-Diagramm der freien Base der Form N-4, gezeigt in Figur 5, und **durch** die Peaks, im wesentlichen wie aufgeführt in der folgenden Tabelle.
**Kohlenstoff-13-SSNMR chemische Verschiebungen für jede von den Formen**
| N-4 HCl |
|---|
| 4.1 |
| 6.3 |
| 12.4 |
| 14.4 |
| 17.1 |
| 21 |
| 23.5 |
| 43.1 |
| 110 |
| 122.4 |
| 125.1 |
| 128.1 |
| 129.1 |
| 130.4 |
| 131.7 |
| 134.7 |
| 135.7 |
| 138.9 |
| 147.1 |
| 163.2 |

20. Kristalline Form, wie definiert in Anspruch 14, mit einer mittleren Teilchengrößenverteilung von 95%<60 µm.

21. Verfahren zum Herstellen des Chlorwasserstoffsäure-Salzes von 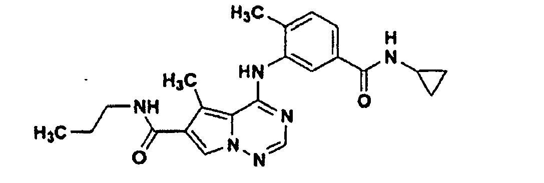 in der Form von Kristallen der Form N-1, wie definiert in Anspruch 2, welches umfasst
a) Bereitstellen einer freien Base mit der Struktur 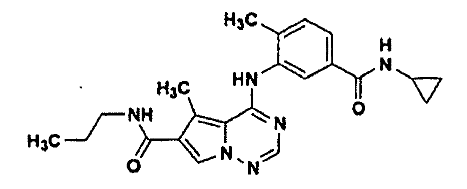 suspendiert oder gemischt in einem organischen Lösungsmittel;
b) Umsetzen der freien Base mit einer wässerigen Lösung von Chlorwasserstoffsäure;
c) Beimpfen des Reaktionsgemisches mit Kristallen der Form N-1 eines Chlorwasserstoffsalzes der freien Base, wie definiert in Anspruch 2; und
d) Gewinnen von Chlorwasserstoffsäure-Salz in der Form von Kristallen der Form N-1.

22. Verfahren, wie definiert in Anspruch 21, wobei die freie Base gemischt wird mit:
a) Tetrahydrofuran; oder
b) N,N-Dimethylformamid und Aceton mit dem beimpften Reaktionsgemisch gemischt werden.

23. Verfahren, wie definiert in Anspruch 22, wobei die Impfkristalle von Kristallen der Form N-1 des Hydrochlorid-Salzes hergestellt werden durch:
a) Suspendieren der freien Base in einem organischen Lösungsmittel;
b) Umsetzen der freien Base mit wässeriger HCl-Säure; und
c) Gewinnen von Impfkristallen von Kristallen der Form N-1.

24. Verfahren zum Herstellen des Methansulfonsäuresalzes der Form N-1 einer Verbindung mit der Struktur 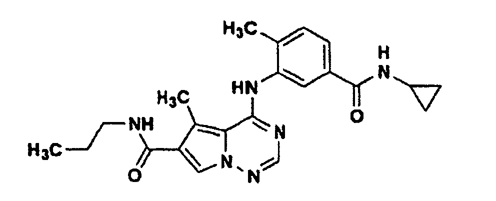 wie definiert in Anspruch 8, welches umfasst
a) Bereitstellen einer Lösung einer freien Base mit der Struktur 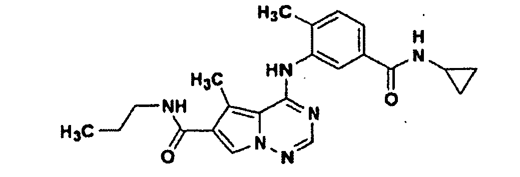 in einem organischen Lösungsmittel;
b) Umsetzen der freien Base mit Methansulfonsäure;
c) Beimpfen des Reaktionsgemisches mit Kristallen des Methansulfonsäuresalzes der freien Base der Form N-1, wie definiert in Anspruch 10; und
d) Gewinnen von Kristallen des Methansulfonsäuresalzes der Form N-1.

25. Verfahren, wie definiert in Anspruch 24, wobei das organische Lösungsmittel N,N-Dimethylformamid, Isopropylalkohol oder Ethanol ist.

26. Verfahren wie definiert in Anspruch 24, wobei das organische Lösungsmittel N,N-Dimethylformamid ist, einschließend den Schritt, vor dem Beimpfen Aceton zu dem Reaktionsgemisch hinzuzufügen.

27. Verfahren, wie definiert in Anspruch 24, wobei die Impfkristalle von Kristallen der Form N-1 hergestellt werden durch:
a) Suspendieren der freien Base in Isopropylalkohol, Ethanol, Ethylacetat oder Acetonitril;
b) Umsetzen der freien Base mit Methansulfonsäure; und
c) Gewinnen von Kristallen der Form N-1.

28. Verfahren zum Herstellen des Chlorwasserstoffsäure-Salzes der freien Base der Struktur 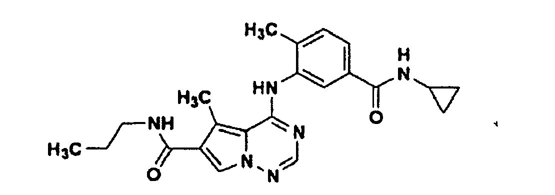 in der Form von Kristallen der Form N-4, wie definiert in Anspruch 14, welches umfasst
a) Bereitstellen einer Aufschlämmung von freier Base der Struktur 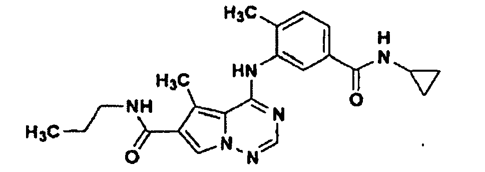 in Ameisensäure und Methylethylketon oder Ameisensäure und Aceton;
b) Hinzufügen einer wässerigen Chlorwasserstoffsäure-Lösung zu der Aufschlämmung von Schritt a);
c) gegebenenfalls Filtrieren des resultierenden Reaktionsgemischs;
d) Hinzufügen des filtrierten Reaktionsgemischs zu einer Aufschlämmung von Impfkristallen von Kristallen der Form N-4 des Hydrochlorid-Salzes der freien Base, wie definiert in Anspruch 14, in Methylethylketon oder Aceton, wobei das gleiche Lösungsmittel, wie angewendet in Schritt a), angewendet wird; und
e) Gewinnen des Chlorwasserstoffsäure-Salzes der freien Base in der Form von Kristallen der Form N-4.

29. Verfahren zum Herstellen des Chlorwasserstoffsäure-Salzes der freien Base der Struktur 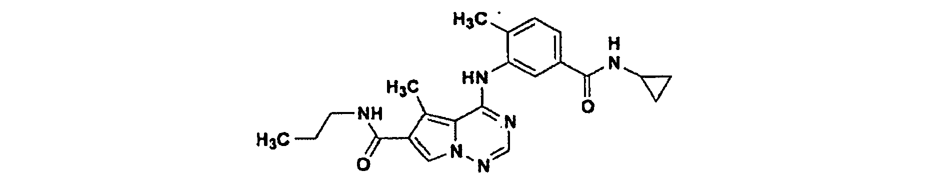 in der Form von Kristallen der Form N-4, wie definiert in Anspruch 14, welches umfasst
a) Bereitstellen eines Gemisches von freier Base der Struktur 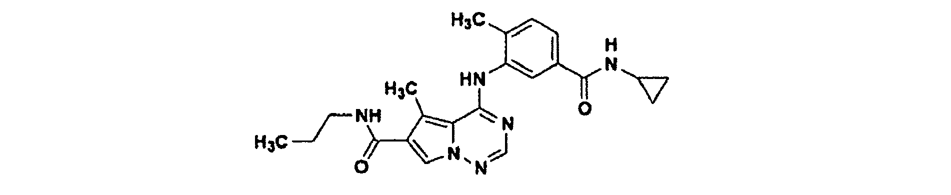 in Ameisensäure und Aceton oder Ameisensäure und MEK;
b) Hinzufügen einer wässerigen Chlorwasserstoffsäure-Lösung zu dem Gemisch von Schritt a);
c) Hinzufügen von Impfkristallen von Kristallen der Form N-4 des Hydrochlorid-Salzes der freien Base, wie definiert in Anspruch 14, und Aceton oder MEK zu dem Reaktionsgemisch von Schritt b); und
d) Gewinnen des Chlorwasserstoffsäure-Salzes der freien Base in der Form von Kristallen der Form N-4.

30. Verfahren zum Herstellen des Chlorwasserstoffsäure-Salzes einer freien Base der Struktur 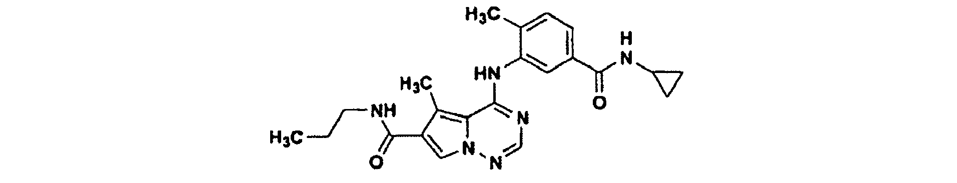 in der Form von N-4-Kristallen, wie definiert in Anspruch 14,
welches umfasst
a) Bereitstellen einer Lösung einer freien Base der Struktur 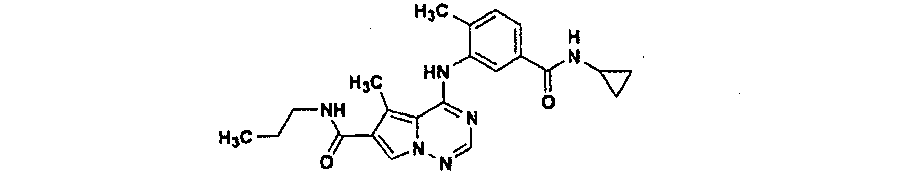 gelöst in N,N-Dimethylacetamid bei einer Temperatur innerhalb des Bereiches von etwa 60 bis etwa 65°C;
b) Bereitstellen einer Lösung von wässeriger Chlorwasserstoffsäure und gekühltem Aceton oder MEK;
c) Hinzufügen von Impfkristallen des Chlorwasserstoffsäure-Salzes der freien Base der Form N-4, wie definiert in Anspruch 14, zu der Aceton/HCl-Lösung;
d) Hinzufügen der Lösung der freien Base in N,N-Dimethylacetamid aus Schritt a), gehalten bei einer Temperatur innerhalb des Bereiches von etwa 50 bis etwa 65°C, in die beimpfte kalte Aceton- oder MEK/HCl-Lösung von Schritt c) unter Rühren, um eine Aufschlämmung zu erzeugen; und
e) Gewinnen von Kristallen der Form N-4 des Chlorwasserstoffsäure-Salzes der freien Base.

31. Verfahren zum Herstellen des Chlorwasserstoffsäure-Salzes einer freien Base der Struktur 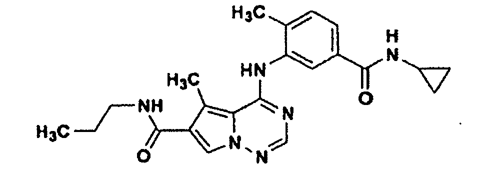 in der Form von N-4-Kristallen, wie definiert in Anspruch 14, welches umfasst
a) Bereitstellen einer Lösung einer freien Base der Struktur 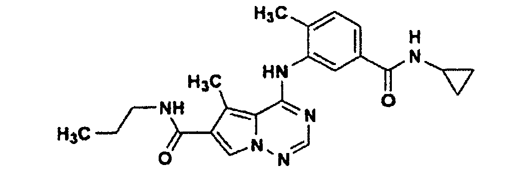 gelöst in N,N-Dimethylacetamid;
b) Hinzufügen einer Lösung von wässeriger Chlorwasserstoffsäure zu der Lösung von Schritt a), um eine Lösung zu erzeugen;
c) Hinzufügen von Impfkristallen des Chlorwasserstoffsäure-Salzes der freien Base der Form N-4, wie definiert in Anspruch 14, und Aceton oder MEK zu der Lösung von Schritt b); und
d) Gewinnen von Kristallen der Form N-4 des Chlorwasserstoffsäure-Salzes der freien Base.

32. Verfahren zum Herstellen des Chlorwasserstoffsäure-Salzes von 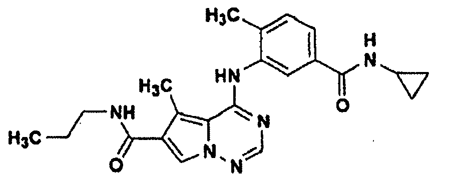 in der Form von Kristallen der Form N-4, wie definiert in Anspruch 14, welches umfasst
a) Bereitstellen einer freien Base mit der Struktur 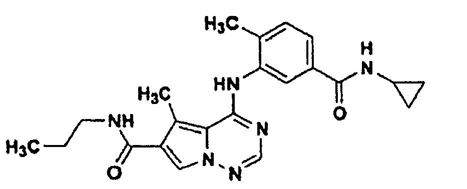 suspendiert in einem organischen Lösungsmittel, welches Tetrahydrofuran, Ethanol oder Aceton ist;
b) Umsetzen der freien Base mit einer wässerigen Lösung von Chlorwasserstoffsäure;
c) Beimpfen des Reaktionsgemisches mit Kristallen der Form N-4 eines Chlorwasserstoffsalzes der freien Base, wie definiert in Anspruch 14; und
d) Gewinnen des Chlorwasserstoffsäure-Salzes in der Form von Kristallen der Form N-4.

33. Verfahren zum Herstellen des Chlorwasserstoffsäure-Salzes der Form N-4 einer Verbindung mit der Struktur 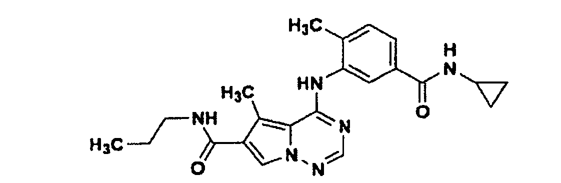 wie definiert in Anspruch 14, welches umfasst
a) Bereitstellen einer Lösung einer freien Base mit der Struktur 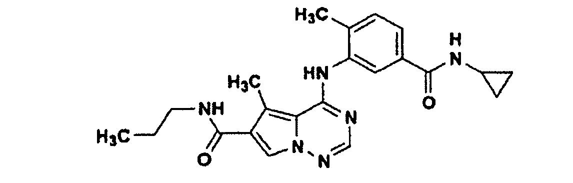 in einem organischen Lösungsmittel, welches Acetonitril, THF, Ethanol oder Aceton ist;
b) Behandeln der freien Base mit Impfkristallen von Kristallen des Chlorwasserstoffsäure-Salzes der freien Base der Form N-4, wie definiert in Anspruch 14; und
c) Gewinnen von Kristallen des Chlorwasserstoffsäuresalzes der Form N-4.

34. Verfahren zum Herstellen des Chlorwasserstoffsäure-Salzes einer freien Base der Struktur 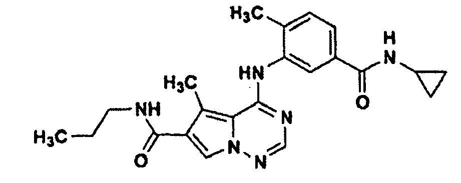 in der Form von N-4-Kristallen, wie definiert in Anspruch 14, welches umfasst
a) Bereitstellen einer Lösung einer freien Base der Struktur 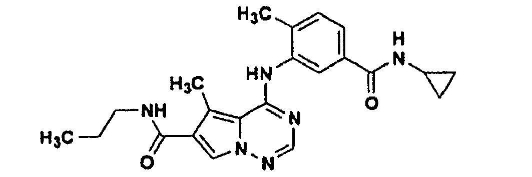 gelöst in Ameisensäure;
b) Bereitstellen einer Lösung von wässeriger Chlorwasserstoffsäure und Aceton oder MEK;
c) Hinzufügen von Impfkristallen des Chlorwasserstoffsäure-Salzes der freien Base der Form N-4, wie definiert in Anspruch 14, in die Aceton- oder MEK/HCl-Lösung;
d) Hinzufügen der Lösung von freier Base in Ameisensäure aus Schritt a), gehalten bei einer Temperatur innerhalb des Bereiches von etwa 15 bis etwa 25°C, in die beimpfte kalte Aceton- oder MEK/HCl-Lösung von Schritt c) unter Rühren, um eine Aufschlämmung zu erzeugen; und
e) Gewinnen von Kristallen der Form N-4 des Chlorwasserstoffsäure-Salzes der freien Base.

35. Verfahren zum Herstellen des Chlorwasserstoffsäure-Salzes von freier Base der Struktur 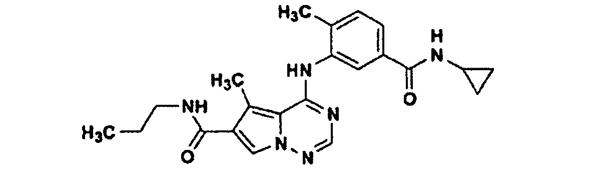 in der Form von Kristallen der Form N-4, wie definiert in Anspruch 14, welches umfasst
a) Bereitstellen einer Suspension oder Lösung von freier Base in N,N-Dimethylformamid;
b) Hinzufügen einer Lösung von wässeriger Chlorwasserstoffsäure zu der Suspension von Schritt a), um eine Lösung zu erzeugen;
c) Hinzufügen von Aceton oder MEK zu der Lösung von Schritt b);
d) Hinzufügen von Impfkristallen des Chlorwasserstoffsäure-Salzes der freien Base der Form N-4, wie definiert in Anspruch 14, zu dem Gemisch von Schritt c); und
e) Gewinnen von Kristallen der Form N-4 des Chlorwasserstoffsäure-Salzes der freien Base.

36. Verfahren zum Herstellen des Chlorwasserstoffsäure-Salzes der freien Base der Struktur 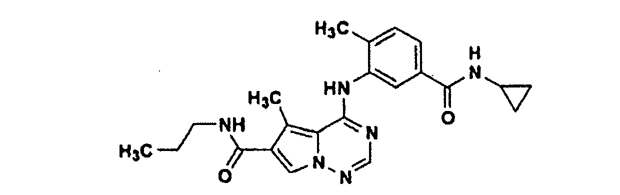 in der Form von N-4-Kristallen, wie definiert in Anspruch 14, welches umfasst
a) Bereitstellen einer Aufschlämmung einer freien Base der Struktur I, gelöst in N,N-Dimethylformamid, N,N-Dimethylformamid/Aceton oder N,N-Dimethylformamid/MEK;
b) Hinzufügen einer Lösung von wässeriger Chlorwasserstoffsäure und Aceton oder MEK zu der Aufschlämmung von Schritt a), um eine Lösung zu erzeugen;
c) gegebenenfalls Abfiltrieren von unlöslichen Feststoffen aus der Lösung von Schritt b);
d) Hinzufügen von Impfkristallen des Chlorwasserstoffsäure-Salzes der freien Base der Form N-4, wie definiert in Anspruch 18, als eine Aufschlämmung in Aceton zu der Lösung, erhalten in Schritt c); und
e) Gewinnen von Kristallen der Form N-4 des Chlorwasserstoffsäure-Salzes der freien Base I.

37. Chlorwasserstoffsäure-Salz der Form N-1 der freien Base 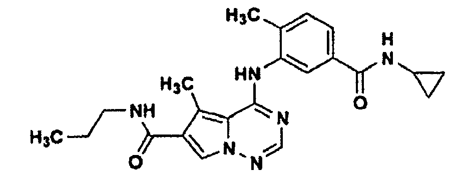 hergestellt wie definiert durch das Verfahren von Anspruch 21.

38. Methansulfonsäure Salz der Form N-1 der freien Base 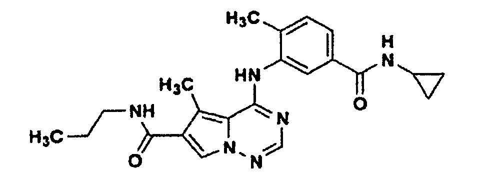 hergestellt wie definiert durch das Verfahren von Anspruch 24.

39. Chlorwasserstoffsäure-Salz der Form N-4 der freien Base 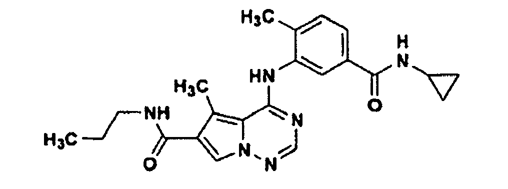 hergestellt wie definiert durch das Verfahren von Anspruch 28.

40. Chlorwasserstoffsäure-Salz der Form N-4 der freien Base 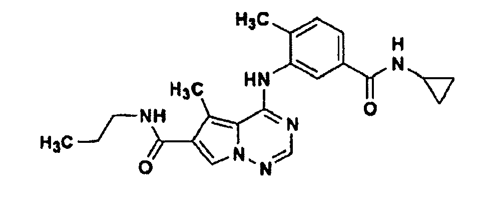 hergestellt wie definiert durch das Verfahren von Anspruch 29.

41. Chlorwasserstoffsäure-Salz der Form N-4 der Struktur 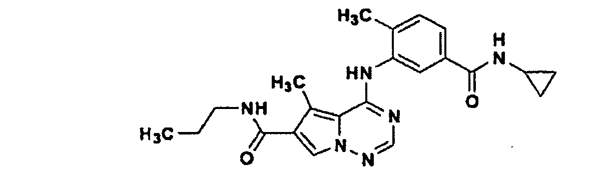 hergestellt wie definiert durch das Verfahren von Anspruch 30.

42. Chlorwasserstoffsäure-Salz der Form N-4 der Struktur 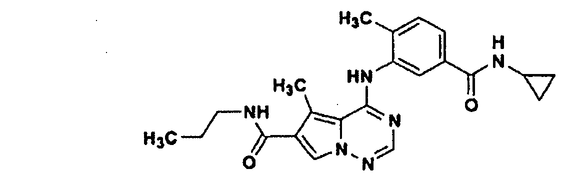 hergestellt wie definiert durch das Verfahren von Anspruch 31.

43. Chlorwasserstoffsäure-Salz der Form N-4 der Struktur 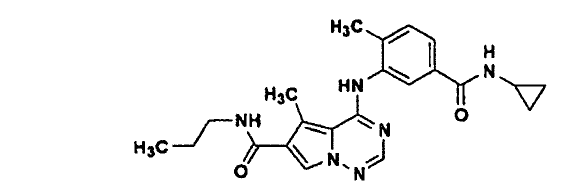 hergestellt wie definiert durch das Verfahren von Anspruch 32.

44. Chlorwasserstoffsäure-Salz der Form N-4 der Struktur 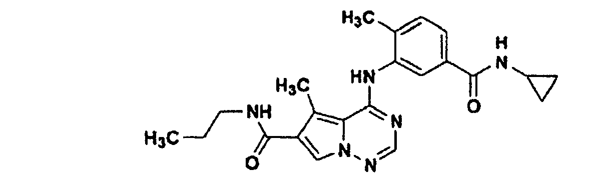 hergestellt wie definiert durch das Verfahren von Anspruch 33.

45. Chlorwasserstoffsäure-Salz der Form N-4 der Struktur 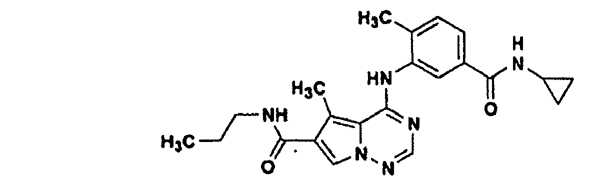 hergestellt wie definiert durch das Verfahren von Anspruch 34.

46. Chlorwasserstoffsäure-Salz der Form N-4 der Struktur 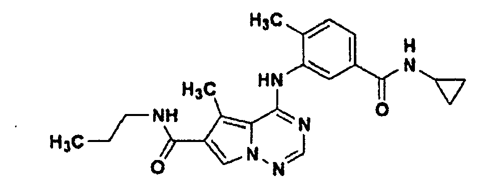 hergestellt wie definiert durch das Verfahren von Anspruch 35.

47. Chlorwasserstoffsäure-Salz der Form N-4 der Struktur 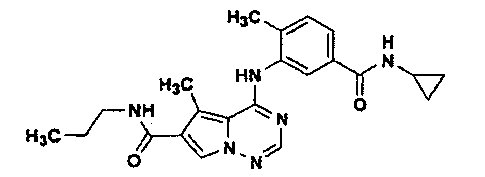 hergestellt wie definiert durch das Verfahren von Anspruch 36.

48. Pharmazeutische Zusammensetzung, umfassend mindestens eine Verbindung gemäß Anspruch 1 und einen pharmazeutisch verträglichen Träger oder ein Verdünnungsmittel.

49. Pharmazeutische Zusammensetzung, umfassend mindestens eine Verbindung gemäß Anspruch 2 und einen pharmazeutisch verträglichen Träger oder ein Verdünnungsmittel.

50. Pharmazeutische Zusammensetzung, umfassend mindestens eine Verbindung gemäß Anspruch 14 und einen pharmazeutisch verträglichen Träger oder ein Verdünnungsmittel.

51. Verwendung einer Verbindung nach einem der Ansprüche 1 bis 20 oder 37 bis 47 zum Herstellen einer pharmazeutischen Zusammensetzung zum Behandeln einer entzündlichen Störung.

52. Verwendung nach Anspruch 51, in welcher die entzündliche Störung aus Asthma, Atemnotsyndrom bei Erwachsenen, chronisch-obstruktiver Lungenerkrankung, chronischer Lungenentzündung, Diabetes, entzündlicher Darmerkrankung, Osteoporose, Psoriasis, Transplantat-gegen-Wirt-Abstoßung, Atherosklerose und Arthritis einschließlich rheumatoider Arthritis, psoriatischer Arthritis, traumatischer Arthritis, Rubella-Arthritis, Gichtarthritis und Osteoarthritis ausgewählt ist.

53. Verbindung nach einem der Ansprüche 1 bis 20 zur Anwendung in einem Verfahren zur Behandlung einer entzündlichen Störung.

54. Verbindung nach Anspruch 53, wobei die entzündliche Störung aus Asthma, Atemnotsyndrom bei Erwachsenen, chronisch-obstruktiver Lungenerkrankung, chronischer Lungenentzündung, Diabetes, entzündlicher Darmerkrankung, Osteoporose, Psoriasis, Transplantat-gegen-Wirt-Abstoßung, Atherosklerose und Arthritis einschließlich rheumatoider Arthritis, psoriatischer Arthritis, traumatischer Arthritis, Rubella-Arthritis, Gichtarthritis und Osteoarthritis ausgewählt ist.

## Revendications

1. Forme cristalline de 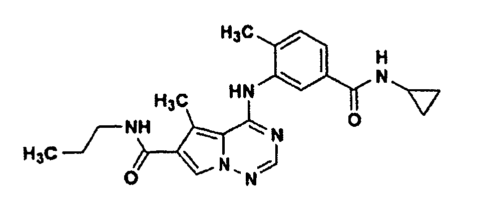 sous forme d'un sel du même.

2. Forme N-1 du sel d'acide chlorhydrique cristallin de la base libre de la structure 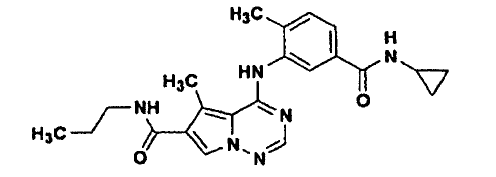 qui est **caractérisée par** des paramètres de cellule unitaire sensiblement égaux aux suivants:
Dimensions cellulaires d'un seul cristal:
a = 22,50(1) Å
b = 14,667(8) Å
c = 14,96(1) Å
α = 90°
β = 116,78(5)°
γ= 90°
groupe spatial C2/c
Molécules/unité asymétrique 1
où ladite forme cristalline est à environ -50°C.
**DIMENSIONS CELLULAIRES D'UN HYBRIDE A TEMPERATURE AMBIANTE:**
| Paramètre cellulaire | Hybride |
|---|---|
| a (Å) | 22,73 |
| b (Å) | 14,710 |
| c (Å) | 15,04 |
| alpha (°) | 90 |
| bêta (°) | 117,13 |
| gamma (°) | 90 |
| V (Å)³ | 4475,02 |

3. La forme cristalline telle que définie dans la revendication 2 telle que **caractérisée par** un réseau de diffraction des rayons X sur poudre comprenant les valeurs 2θ suivantes (Cukα λ - 1,5418 Å) 8,7 ± 0,1, 12,1 ± 0,1, 13,3 ± 0,1, 13,7 ± 0,1, 14,6 ± 0,1, 17,5 ± 0,1, 18,2 ± 0,1, 21,7 ± 0,1, 22,8 ± 0,1 et 24,3 ± 0,1 à environ température ambiante.

4. La forme cristalline telle que définie dans la revendication 2, qui est **caractérisée par** des coordonnées atomiques fractionnaires sensiblement telles qu'énumérées dans la table suivante:
**PARAMETRES POSITIONNELS ET FACTEURS DE TEMPERATURE ISOTROPE POUR LA FORME N-1 DU SEL HCL DE LA BASE LIBRE I À -50°C**
| Nom | x | y | z | B(iso) |
|---|---|---|---|---|
| CL1 | -0.1274 | 0.7316 | 0.1928 | 2.6 |
| O11 | -0.0911 | 0.3308 | 0.0631 | 3.0 |
| 025 | 0.0768 | 1.4932 | 0.1927 | 2.5 |
| N3 | -0.0789 | 0.5597 | -0.1360 | 1.4 |
| N-4 | -0.0726 | 0.6044 | -0.2111 | 2.0 |
| N6 | -0.0627 | 0.7370 | -0.1109 | 2.0 |
| N-12 | -0.1338 | 0.2956 | -0.1016 | 2.4 |
| N-16 | -0.0670 | 0.7356 | 0.0417 | 1.5 |
| N28 | 0.1170 | 0.9587 | 0.2589 | 2.6 |
| C1 | -0.0898 | 0.4443 | -0.0493 | 1.3 |
| C2 | -0.0889 | 0.4657 | -0.1398 | 1.5 |
| C5 | -0.0632 | 0.6929 | -0.1963 | 2.5 |
| C7 | -0.0689 | 0.6963 | -0.0354 | 2.2 |
| C8 | -0.0755 | 0.5942 | -0.0480 | 1.7 |
| C9 | -0.0808 | 0.5253 | 0.0083 | 1.0 |
| C10 | -0.1055 | 0.3490 | -0.0243 | 1.8 |
| C13 | -0.1599 | 0.2059 | -0.0880 | 3.3 |
| C14 | -0.2373 | 0.2200 | -0.1225 | 6.2 |
| C15 | -0.2651 | 0.1334 | -0.1018 | 8.3 |
| C17 | -0.0610 | 0.8346 | 0.0471 | 1.7 |
| C18 | -0.0007 | 0.8710 | 0.1155 | 1.1 |
| C19 | 0.0047 | 0.9629 | 0.1264 | 1.0 |
| C20 | -0.0480 | 1.0206 | 0.0738 | 2.2 |
| C21 | -0.1086 | 0.9820 | 0.0094 | 2.3 |
| C22 | -0.1188 | 0.8899 | -0.0051 | 1.8 |
| C23 | -0.0769 | 0.5314 | 0.1117 | 2.5 |
| C24 | 0.0678 | 1.0108 | 0.1954 | 2.0 |
| C27 | -0.1847 | 0.8464 | -0.0730 | 2.9 |
| C29 | 0.1795 | 1.0002 | 0.3324 | 3.0 |
| C30 | 0.2351 | 0.9973 | 0.3046 | 5.6 |
| C31 | 0.2374 | 0.9376 | 0.3874 | 4.9 |
| H61 | -0.0563 | 0.8116 | -0.1063 | 2.8 |
| H121 | -0.1406 | 0.3168 | -0.1761 | 3.4 |
| H161 | -0.0714 | 0.6963 | 0.1014 | 2.5 |
| H281 | 0.1109 | 0.8848 | 0.2553 | 3.6 |

5. La forme cristalline telle que définie dans la revendication 2, qui est **caractérisée par** la RMN à l'état solide du C-13 de la carte de la Forme N-1 du sel HCL comme il est illustré sur la Figure 4 et par les pics sensiblement tels qu'énumérés dans la table suivante:
**DEPLACEMENTS CHIMIQUES DU CARBONE 13 PAR RMN À L'ETAT SOLIDE**
| N-1 HCl |
|---|
| 3 |
| 7.3 |
| 12.9 |
| 15.2 |
| 19.4 |
| 24.6 |
| 40.5 |
| 109.8 |
| 122.3 |
| 125 |
| 127.3 |
| 128.1 |
| 131.7 |
| 136.9 |
| 142.9 |
| 147.3 |
| 162.5 |
| 167.1 |
| |
| |

6. La forme cristalline telle que définie dans la revendication 2, qui est **caractérisée par** un thermogramme d'analyse calorimétrique différentielle sensiblement conforme à celui qui est illustré sur la Figure 7, ayant un endotherme dans la plage d'environ 125 à environ 225°C.

7. La forme cristalline telle que définie dans la revendication 2, qui est **caractérisée par** une courbe thermogravimétrique conforme à celle qui est illustrée sur la Figure 10 ayant une perte de poids négligeable jusqu'à environ 100°C et une perte de poids d'environ 8,2% jusqu'à environ 225°C.

8. Sel d'acide méthanesulfonique de Forme N-1 de la base libre de la structure 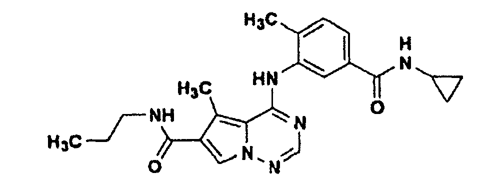 qui est **caractérisée par** des paramètres de cellule unitaire sensiblement égaux aux suivants:
Dimensions cellulaires:
a = 9,818(1) Å
b = 11,127(1) Å
c = 13,004(1) Å
α = 97,32(1)°
β = 110,17(1)°
γ = 111,48(1)°
groupe spatial P-1
Molécules/unité asymétrique 1
où la forme cristalline est à environ +22°C.

9. La forme cristalline telle que définie dans la revendication 8, qui est **caractérisée par** un réseau de diffraction des rayons X sur poudre comprenant les valeurs 29 suivantes (CuKα λ = 1,5418 Å) 10,7 ± 0,1, 11,7 ± 0,1, 13,3 ± 0,1, 14,0 ± 0,1, 15,2 ± 0,1, 19,8 ± 0,1, 21,0 ± 0,1, 22,0 ± 0,1, 23,0 ± 0,1 et 24,4 ± 0,1 à température ambiante.

10. La forme cristalline telle que définie dans la revendication 8, qui est **caractérisée par** des coordonnées atomiques fractionnaires sensiblement telles qu'énumérées dans la table suivante:
**PARAMETRES POSITIONNELS ET FACTEURS DE TEMPERATURE ISOTROPE POUR LA FORME N-1 DU SEL DE L'ACIDE MS DE LA BASE LIBRE I À TEMPERATURE AMBIANTE**
| Nom | x | y | z | B(iso) |
|---|---|---|---|---|
| S1 | -0.2392 | -0.0718 | 0.2812 | 3.9 |
| O19 | -0.3458 | 0.3140 | 0.5245 | 3.9 |
| O25 | 0.1495 | 0.5491 | -0.1489 | 4.5 |
| O96 | -0.0747 | -0.0117 | 0.2977 | 7.2 |
| O97 | -0.3175 | 0.0086 | 0.2347 | 6.2 |
| O98 | -0.3273 | -0.2110 | 0.2187 | 6.2 |
| N2 | 0.0159 | 0.2025 | 0.2128 | 3.5 |
| N-4 | 0.0134 | 0.1493 | 0.0309 | 4.3 |
| N5 | 0.0858 | 0.2860 | 0.0493 | 3.4 |
| N-10 | 0.1369 | 0.4167 | 0.3376 | 3.1 |
| N20 | -0.4171 | 0.1644 | 0.3592 | 3.7 |
| N26 | 0.3707 | 0.6920 | 0.0118 | 3.9 |
| C1 | 0.0952 | 0.3375 | 0.2357 | 2.9 |
| C3 | -0.0185 | 0.1153 | 0.1135 | 4.0 |
| C6 | 0.1200 | 0.3448 | -0.0287 | 3.8 |
| C7 | 0.1918 | 0.4820 | 0.0194 | 3.3 |
| C8 | 0.2006 | 0.5099 | 0.1308 | 3.1 |
| C9 | 0.1335 | 0.3851 | 0.1494 | 2.9 |
| C11 | 0.0777 | 0.3716 | 0.4202 | 2.8 |
| C12 | -0.0828 | 0.3343 | 0.3948 | 3.0 |
| C13 | -0.1378 | 0.3039 | 0.4767 | 2.8 |
| C14 | -0.0287 | 0.3161 | 0.5846 | 3.3 |
| C15 | 0.1288 | 0.3502 | 0.6066 | 3.4 |
| C16 | 0.1867 | 0.3782 | 0.5256 | 3.2 |
| C17 | 0.3599 | 0.4139 | 0.5526 | 4.5 |
| C18 | -0.3085 | 0.2627 | 0.4558 | 3.0 |
| C21 | -0.5877 | 0.1142 | 0.3265 | 4.7 |
| C22 | -0.6743 | -0.0158 | 0.3494 | 6.0 |
| C23 | -0.6918 | -0.0137 | 0.2327 | 5.2 |
| C24 | 0.2358 | 0.5773 | -0.0471 | 3.4 |
| C27 | 0.4320 | 0.7945 | -0.0402 | 4.8 |
| C28 | 0.5627 | 0.7822 | -0.0713 | 6.1 |
| C29 | 0.5061 | 0.6520 | -0.1574 | 6.3 |
| C30 | 0.2560 | 0.6437 | 0.2098 | 4.3 |
| C99 | -0.2317 | -0.0630 | 0.4193 | 5.7 |
| H21 | -0.0223 | 0.1622 | 0.2752 | 3.7 |
| H101 | 0.2234 | 0.5211 | 0.3609 | 3.3 |
| N201 | -0.3748 | 0.1234 | 0.3044 | 4.1 |
| H261 | 0.4413 | 0.7077 | 0.1023 | 4.0 |

11. La forme cristalline telle que définie dans la revendication 8, qui est **caractérisée par** un thermogramme d'analyse calorimétrique différentielle sensiblement conforme à celui qui est illustré sur la Figure 9, ayant un endotherme avec une apparition de pics à environ 216°C.

12. La forme cristalline telle que définie dans la revendication 8, qui est **caractérisée par** une courbe thermogravimétrique conforme à celle qui est illustrée sur la Figure 12 ayant une perte de poids négligeable jusqu'à environ 150°C.

13. La forme cristalline telle que définie dans la revendication 8, qui est **caractérisée par** la RMN à l'état solide du carbone 13 du sel d'acide MS de Forme N-1 de la carte de la base libre, illustrée sur la Figure 6 et par les pics sensiblement tels qu'énumérés dans la table suivante:
**DEPLACEMENTS CHIMIQUES DU CARBONE 13 PAR RMN À L'ETAT SOLIDE**
| N-1 MSA |
|---|
| 6 |
| 8.8 |
| 10.4 |
| 14.3 |
| 20.4 |
| 24.4 |
| 40.3 |
| 110.3 |
| 123.5 |
| 126 |
| 126.9 |
| 129.8 |
| 132.3 |
| 135.6 |
| 138.1 |
| 139.2 |
| 148.8 |
| 163.1 |
| 172.6 |

14. Sel d'acide chlorhydrique de Forme N-4 de la base libre de la structure 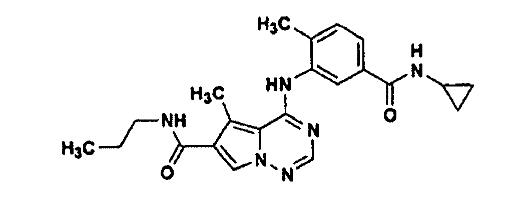 qui est **caractérisée par** des paramètres de cellule unitaire sensiblement égaux aux suivants:
Dimensions cellulaires:
a = 20,9498(5) Å
b = 13,8719(3) Å
c = 7,9133(2) Å
α = 90°
β = 100,052(1)°
γ = 90°
groupe spatial P2₁/n
Molécules/unité asymétrique 1
où ladite forme cristalline est à environ +22°C

15. La forme cristalline telle que définie dans la revendication 14, qui est **caractérisée par** des coordonnées atomiques fractionnaires telles qu'énumérées dans la table suivante:
**PARAMETRES POSITIONNELS ET FACTEURS DE TEMPERATURE ISOTROPE POUR LA FORME N-4 DU SEL HCL DE LA BASE LIBRE I À TEMPERATURE AMBIANTE**
| Nom | x | y | z | B(iso) |
|---|---|---|---|---|
| CL | 0.0341 | 0.7162 | 0.4308 | 5.5 |
| O1 | 0.1111 | 1.0617 | 0.3458 | 5.4 |
| 02 | -0.2021 | 0.5613 | -0.6888 | 6.5 |
| N-1 | -0.0710 | 0.5099 | 0.0187 | 5.0 |
| N2 | -0.0544 | 0.6737 | 0.0953 | 4.4 |
| N3 | -0.0913 | 0.5440 | -0.1460 | 4.2 |
| N-4 | -0.0762 | 0.8037 | -0.0894 | 4.3 |
| N5 | 0.1228 | 0.9001 | 0.3778 | 4.5 |
| N6 | -0.1487 | 0.4216 | -0.6361 | 5.1 |
| C1 | -0.0540 | 0.5768 | 0.1292 | 4.9 |
| C2 | -0.0752 | 0.7092 | -0.0651 | 3.8 |
| C3 | -0.1215 | 0.6429 | -0.3703 | 4.0 |
| C4 | -0.1318 | 0.5462 | -0.4222 | 4.0 |
| C5 | -0.1137 | 0.4874 | -0.2809 | 4.6 |
| C6 | -0.0956 | 0.6414 | -0.1958 | 3.7 |
| C7 | -0.0604 | 0.8753 | 0.0446 | 3.9 |
| C8 | 0.0035 | 0.8873 | 0.1212 | 3.9 |
| C9 | 0.0202 | 0.9626 | 0.2399 | 3.8 |
| C10 | -0.0285 | 1.0247 | 0.2755 | 4.4 |
| C11 | -0.0924 | 1.0089 | 0.1989 | 4.6 |
| C12 | -0.1101 | 0.9344 | 0.0833 | 4.3 |
| C13 | 0.0882 | 0.9795 | 0.3247 | 4.3 |
| C14 | 0.1885 | 0.9076 | 0.4700 | 4.8 |
| C15 | 0.2433 | 0.8987 | 0.3757 | 7.1 |
| C16 | 0.2285 | 0.8180 | 0.4863 | 7.3 |
| C17 | -0.1632 | 0.5116 | -0.5947 | 4.6 |
| C18 | -0.1796 | 0.3769 | -0.7945 | 5.8 |
| C19 | -0.1408 | 0.2925 | -0.8428 | 7.6 |
| C20 | -0.1734 | 0.2467 | -1.0053 | 9.0 |
| C21 | -0.1342 | 0.7314 | -0.4822 | 5.3 |
| C22 | -0.1802 | 0.9169 | 0.0035 | 6.0 |
| H11 | -0.0396 | 0.5549 | 0.2601 | 5.9 |
| H21 | -0.0375 | 0.7237 | 0.1998 | 5.4 |
| H41 | -0.0910 | 0.8303 | -0.2198 | 5.3 |
| H51 | -0.1153 | 0.4087 | -0.2785 | 5.6 |
| H51 | 0.1006 | 0.8297 | 0.3463 | 5.5 |
| H61 | -0.1122 | 0.3809 | -0.5485 | 4.0 |
| H81 | 0.0407 | 0.8396 | 0.0891 | 4.8 |
| H101 | -0.0165 | .1.0872 | 0.3579 | 5.4 |
| H111 | -0.1294 | 1.0551 | 0.2302 | 5.4 |
| H141 | 0.1928 | 0.9796 | 0.5314 | 5.9 |

16. La forme cristalline telle que définie dans la revendication 14, ayant une diffraction des rayons X sur poudre comprenant les valeurs 2θ suivantes (CuKα λ = 1,5418 Å) 8,6 ± 0,1, 10,7 ± 0,1, 11,4 ± 0,1, 12,8 ± 0,1,14,4 ± 0,1, 15,6 ± 0,1, 16,9 ± 0,1, 18,3 ± 0,1, 20,0 ± 0,1 et 23,4 ± 0,1 à environ température ambiante.

17. La forme cristalline telle que définie dans la revendication 14, qui est **caractérisée par** un thermogramme d'analyse calorimétrique différentielle sensiblement conforme à celui qui est illustré sur la Figure 8, ayant un endotherme dans la plage d'environ 130 à environ 220°C (variable).

18. La forme cristalline telle que définie dans la revendication 14, qui est **caractérisée par** une courbe thermogravimétrique conforme à celle qui est illustrée sur la Figure 11 ayant une perte de poids négligeable jusqu'à environ 125°C.

19. La forme cristalline telle que définie dans la revendication 14, qui est **caractérisée par** la RMN à l'état solide de la carte de la base libre de la Forme N-4 illustrée sur la Figure 5 et par les pics sensiblement tels qu'énumérés dans la table suivante:
**DEPLACEMENTS CHIMIQUES DU CARBONE 13 PAR RMN À L'ETAT SOLIDE POUR CHACUNE DES FORMES**
| N-4 HCl |
|---|
| 4.1 |
| 6.3 |
| 12.4 |
| 14.4 |
| 17.1 |
| 21 |
| 23.5 |
| 43.1 |
| 110 |
| 122.4 |
| 125.1 |
| 128.1 |
| 129.1 |
| 130.4 |
| 131.7 |
| 134.7 |
| 135.7 |
| 138.9 |
| 147.1 |
| 163.2 |

20. La forme cristalline telle que définie dans la revendication 14 ayant une distribution de taille de particules moyenne de 95% < 60 µm.

21. Procédé de préparation de sel d'acide chlorhydrique de 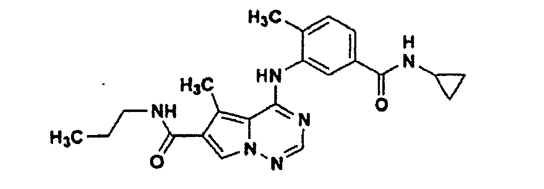 sous la forme de cristaux de Forme N-1 telle que définie dans la revendication 2, qui comprend
a) fournir une base libre ayant la structure 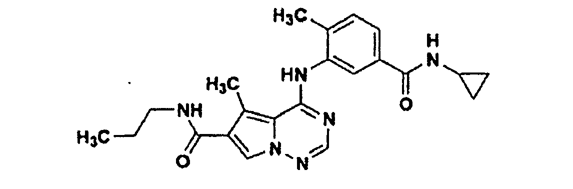 en suspension ou mélangée dans un solvant organique;
b) mettre la base libre à réagir avec une solution aqueuse d'acide chlorhydrique;
c) ensemencer le mélange de réaction avec des cristaux de Forme N-1 d'un sel chlorhydrique de ladite base libre telle que définie dans la revendication 2; et
d) recueillir le sel d'acide chlorhydrique sous la forme de cristaux de Forme N-1.

22. Le procédé tel que défini dans la revendication 21, dans lequel la base libre est mélangée avec:
a) du tétrahydrofurane; ou
b) du N,N-diméthylformamide et de l'acétone est mélangée avec le mélange de réaction ensemencé.

23. Le procédé tel que défini dans la revendication 22, dans lequel les semences de cristaux de Forme N-1 du sel chlorhydrate sont préparées en:
a) mettant la base libre en suspension dans un solvant organique;
b) mettant la base libre à réagir avec de l'acide HCl aqueux; et
c) recueillant les semences de cristaux de Forme N-1.

24. Procédé de préparation de sel d'acide méthanesulfonique de Forme N-1 d'un composé ayant la structure 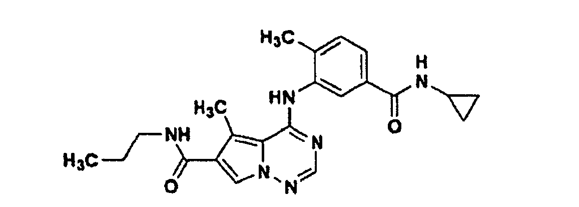 telle que définie dans la revendication 8, lequel comprend:
a) fournir une solution d'une base libre ayant la structure 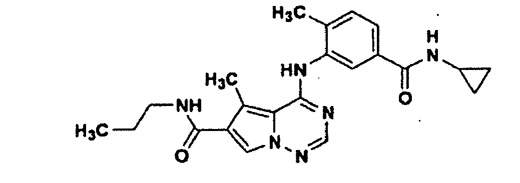 dans un solvant organique;
b) mettre la base libre à réagir avec de l'acide méthanesulfonique;
c) ensemencer le mélange de réaction avec des cristaux de sel d'acide méthanesulfonique de Forme N-1 de ladite base libre telle que définie dans la revendication 10; et
d) recueillir les cristaux de sel d'acide méthanesulfonique de Forme N-1.

25. Le procédé tel que défini dans la revendication 24, dans lequel le solvant organique est du N,N-diméthylformamide, de l'alcool isopropylique ou de l'éthanol.

26. Le procédé tel que défini dans la revendication 24, dans lequel le solvant organique est du N,N-diméthylformamide, incluant l'étape consistant à ajouter de l'acétone au mélange de réaction avant l'ensemencement.

27. Le procédé tel que défini dans la revendication 24, dans lequel les semences de cristaux de Forme N-1 sont préparées en:
a) mettant la base libre en suspension dans de l'alcool isopropylique, de l'éthanol, de l'acétate d'éthyle ou de l'acétonitrile;
b) mettant la base libre à réagir avec de l'acide méthanesulfonique; et
c) recueillant les cristaux de Forme N-1.

28. Procédé de préparation du sel d'acide chlorhydrique de la base libre de la structure 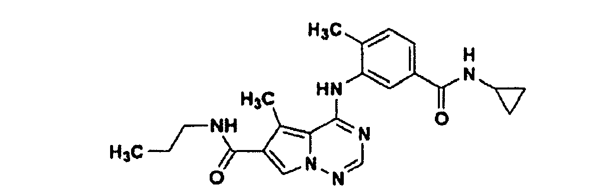 sous la forme de cristaux de Forme N-1 telle que définie dans la revendication 14, lequel comprend
a) fournir une bouillie de base libre de la structure 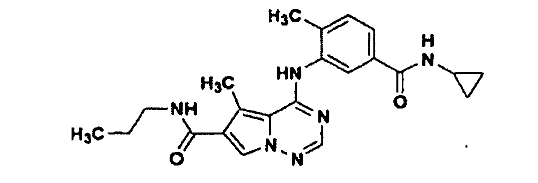 dans de l'acide formique et de la méthyléthylcétone ou de l'acide formique et de l'acétone;
b) ajouter une solution d'acide chlorhydrique aqueux à la bouillie de l'étape a);
c) filtrer optionnellement le mélange de réaction résultant;
d) ajouter le mélange de réaction filtré à une bouillie de semences de cristaux de Forme N-4 du sel chlorhydrate de ladite base libre telle que définie dans la revendication 14 dans de la méthyléthylcétone ou de l'acétone en utilisant le même solvant que celui utilisé à l'étape a); et
e) recueillir le sel d'acide chlorhydrique de la base libre sous forme de cristaux de Forme N-4.

29. Procédé de préparation du sel d'acide chlorhydrique de la base libre de la structure 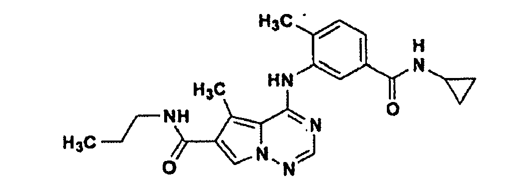 sous forme de cristaux de Forme N-4 telle que définie dans la revendication 14, lequel comprend
a) fournir un mélange de base libre de la structure 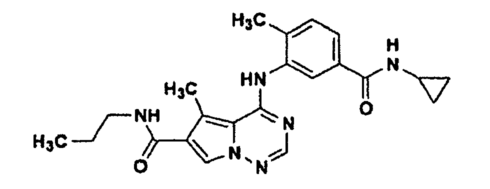 dans de l'acide formique et de l'acétone ou de l'acide formique et de la MEK;
b) ajouter une solution d'acide chlorhydrique aqueux au mélange de l'étape a);
c) ajouter des semences de cristaux de Forme N-4 de sel chlorhydrate de ladite base libre telle que définie dans la revendication 14 et de l'acétone ou de la MEK au mélange de réaction de l'étape b); et
d) recueillir le sel d'acide chlorhydrique de la base libre sous forme de cristaux de Forme N-4.

30. Procédé de préparation du sel d'acide chlorhydrique d'une base libre de la structure 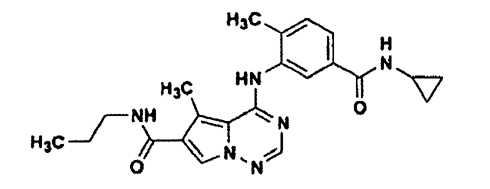 sous forme de cristaux N-4 tels que définis dans la revendication 14,
lequel comprend
a) fournir une solution d'une base libre de la structure 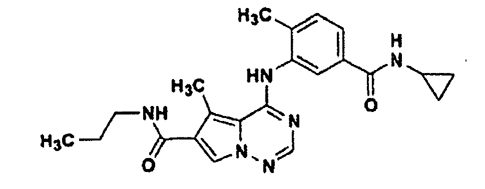 dissoute dans du N,N-diméthylacétamide à une température dans la plage d'environ 60 à environ 65°C;
b) fournir une solution d'acide chlorhydrique aqueux et d'acétone ou de MEK refroidie;
c) ajouter des semences du sel d'acide chlorhydrique de Forme N-4 de la base libre telle que définie dans la revendication 14, dans la solution d'acétone/HCl;
d) ajouter la solution de base libre dans du N,N-diméthylacétamide de l'étape a), maintenue à une température dans la plage d'environ 50 à environ 65°C, dans la solution d'acétone ou de MEK/HCl froide ensemencée de l'étape c) tout en agitant pour former une bouillie; et
e) recueillir des cristaux de Forme N-4 du sel d'acide chlorhydrique de la base libre.

31. Procédé de préparation du sel d'acide chlorhydrique d'une base libre de la structure 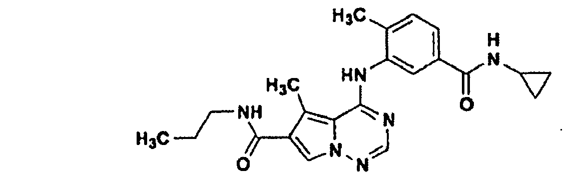 sous forme de cristaux N-4 tels que définis dans la revendication 14,
lequel comprend
a) fournir une solution d'une base libre de la structure 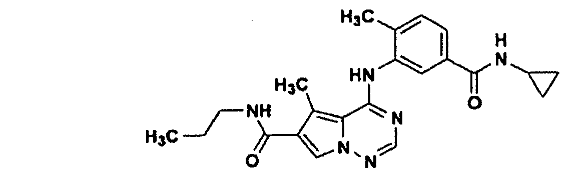 dissoute dans du N,N-diméthylacétamide;
b) ajouter une solution d'acide chlorhydrique aqueux à la solution de l'étape a) pour former une solution;
c) ajouter dans la solution de l'étape b) les semences de sel d'acide chlorhydrique de Forme N-4 de la base libre telle que définie dans la revendication 14 et de l'acétone ou de la MEK; et
d) recueillir les cristaux de Forme N-4 du sel d'acide chlorhydrique de la base libre.

32. Procédé de préparation de sel d'acide chlorhydrique de 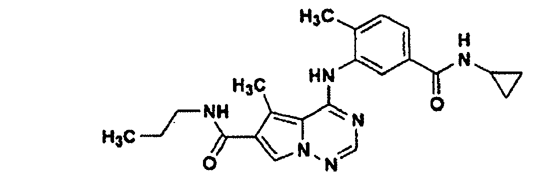 sous forme de cristaux de forme N-4 telle que définie dans la revendication 14, lequel comprend
a) fournir une base libre ayant la structure 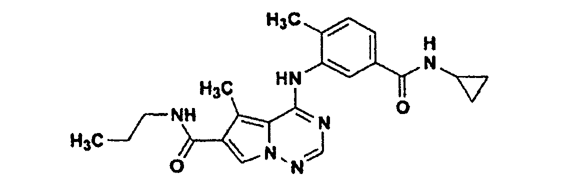 en suspension dans un solvant organique qui est du tétrahydrofurane, de l'éthanol ou de l'acétone;
b) mettre la base libre à réagir avec une solution aqueuse d'acide chlorhydrique;
c) ensemencer le mélange de réaction avec des cristaux de Forme N-4 d'un sel chlorhydrique de ladite base libre telle que définie dans la revendication 14; et
d) recueillir le sel d'acide chlorhydrique sous forme de cristaux de Forme N-4.

33. Procédé de préparation de sel d'acide chlorhydrique de Forme N-4 d'un composé ayant la structure 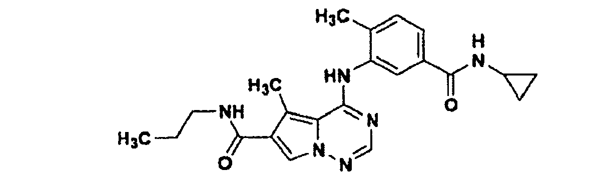 telle que définie dans la revendication 14, lequel comprend
a) fournir une solution d'une base libre ayant la structure 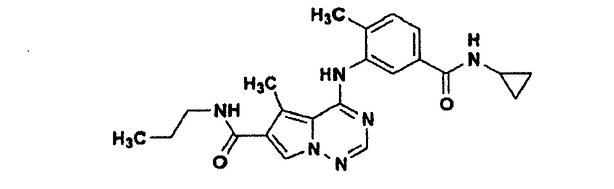 dans un solvant organique qui est de l'acétonitrile, du THF, de l'éthanol ou de l'acétone;
b) traiter la base libre avec des semences de cristaux du sel d'acide chlorhydrique de Forme N-4 de ladite base libre telle que définie dans la revendication 14; et
c) recueillir les cristaux de sel d'acide chlorhydrique de Forme N-4.

34. Procédé de préparation du sel d'acide chlorhydrique d'une base libre de la structure 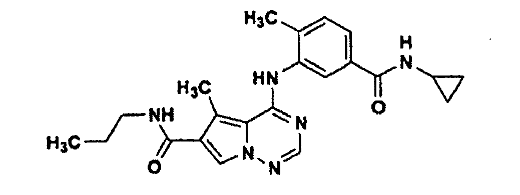 sous forme de cristaux N-4 tels que définis dans la revendication 14,
lequel comprend
a) fournir une solution d'une base libre de la structure 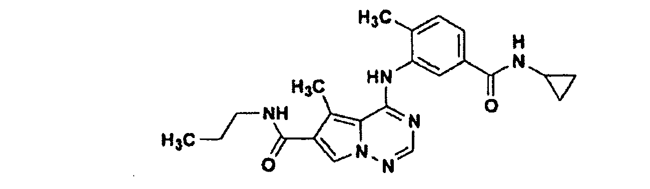 dissoute dans de l'acide formique;
b) fournir une solution d'acide chlorhydrique aqueux et d'acétone ou de MEK;
c) ajouter dans la solution d'acétone ou de MEK/HCl des semences de sel d'acide chlorhydrique de Forme N-4 de la base libre telle que définie dans la revendication 14;
d) ajouter la solution de base libre dans de l'acide formique de l'étape a), maintenue à une température dans la plage d'environ 15 à environ 25°C, dans la solution d'acétone ou de MEK/HCl froide ensemencée de l'étape c) tout en agitant pour former une bouillie; et
e) recueillir les cristaux de Forme N-4 du sel d'acide chlorhydrique de la base libre.

35. Procédé de préparation du sel d'acide chlorhydrique de la base libre de la structure 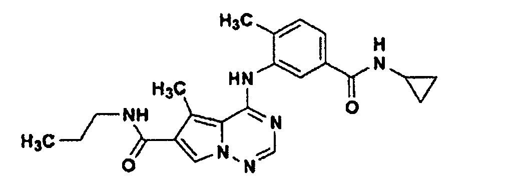 sous forme de cristaux de Forme N-4 telle que définie dans la revendication 14, lequel comprend
a) fournir une suspension ou une solution de base libre dans du N,N-diméthylformamide;
b) ajouter une solution d'acide chlorhydrique aqueux à la suspension de l'étape a) pour former une solution;
c) ajouter de l'acétone ou de la MEK à la solution de l'étape b);
d) ajouter au mélange de l'étape c) des semences de sel d'acide chlorhydrique de Forme N-4 de la base libre telle que définie dans la revendication 14; et
e) recueillir les cristaux de Forme N-4 du sel d'acide chlorhydrique de la base libre.

36. Procédé de préparation du sel d'acide chlorhydrique de la base libre de la structure 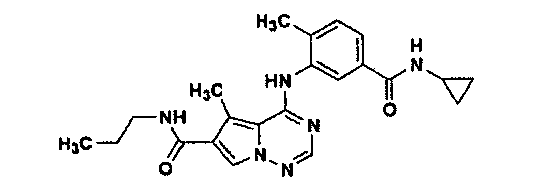 sous forme de cristaux N-4 tels que définis dans la revendication 14, lequel comprend
a) fournir une bouillie d'une base libre de la structure 1 dissoute dans du N,N-diméthylformamide, du N,N-diméthylformamide/acétone ou du N,N-diméthylformamide/MEK;
b) ajouter une solution d'acide chlorhydrique aqueux et d'acétone ou de MEK à la bouillie de l'étape a) pour former une solution;
c) retirer optionnellement par filtration les solides insolubles de la solution de l'étape b);
d) ajouter des semences de sel d'acide chlorhydrique de Forme N-4 de la base libre telle que définie dans la revendication 18 sous forme de bouillie dans de l'acétone, à la solution de l'étape c); et
e) recueillir les cristaux de Forme N-4 du sel d'acide chlorhydrique de la base libre I.

37. Sel d'acide chlorhydrique de Forme N-1 de la base libre 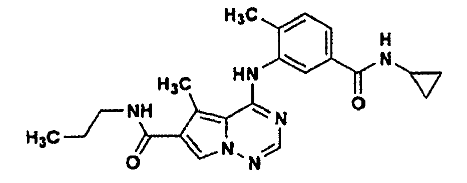 préparé comme il est défini par le procédé de la revendication 21.

38. Sel d'acide méthanesulfonique de Forme N-1 de la base libre 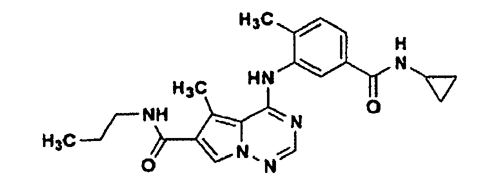 préparé comme il est défini par le procédé de la revendication 24.

39. Sel d'acide chlorhydrique de Forme N-4 de la base libre 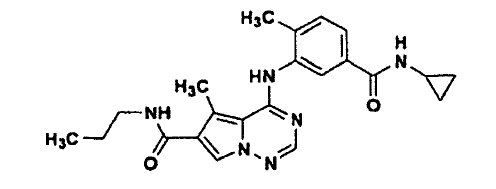 préparé comme il est défini par le procédé de la revendication 28.

40. Sel d'acide chlorhydrique de Forme N-4 de la base libre 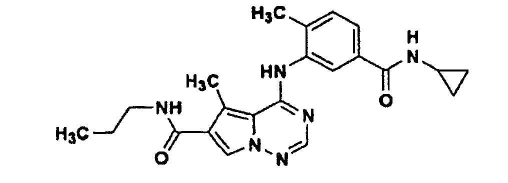 préparé comme il est défini par le procédé de la revendication 29.

41. Sel d'acide chlorhydrique de Forme N-4 de la structure 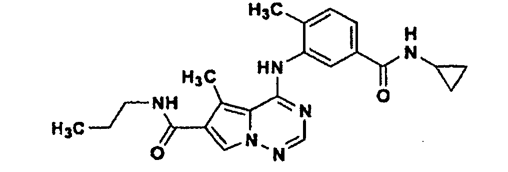 préparé comme il est défini par le procédé de la revendication 30.

42. Sel d'acide chlorhydrique de Forme N-4 de la structure 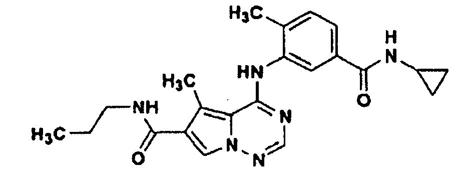 préparé comme il est défini par le procédé de la revendication 31.

43. Sel d'acide chlorhydrique de Forme N-4 de la structure 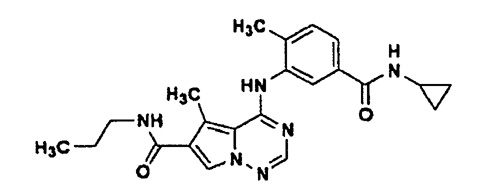 préparé comme il est défini par le procédé de la revendication 32.

44. Sel d'acide chlorhydrique de Forme N-4 de la structure 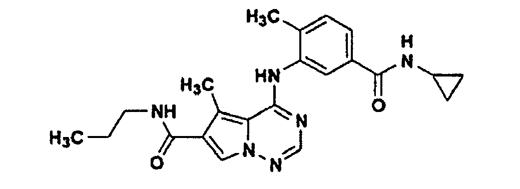 préparé comme il est défini par le procédé de la revendication 33.

45. Sel d'acide chlorhydrique de Forme N-4 de la structure 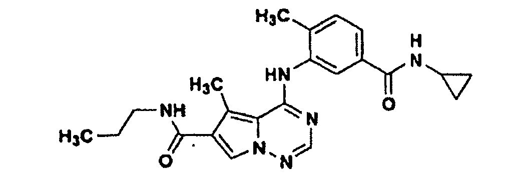 préparé comme il est défini par le procédé de la revendication 34.

46. Sel d'acide chlorhydrique de Forme N-4 de la structure 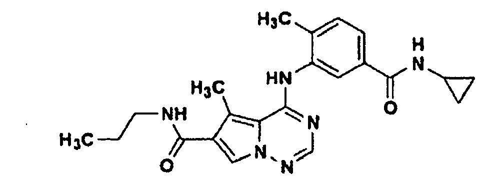 préparé comme il est défini par le procédé de la revendication 35.

47. Sel d'acide chlorhydrique de Forme N-4 de la structure 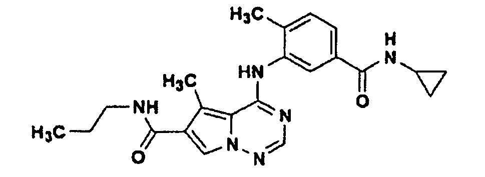 préparé comme il est défini par le procédé de la revendication 36.

48. Composition pharmaceutique comprenant au moins un composé selon la revendication 1 et un excipient ou diluant pharmaceutiquement acceptable.

49. Composition pharmaceutique comprenant au moins un composé selon la revendication 2 et un excipient ou diluant pharmaceutiquement acceptable.

50. Composition pharmaceutique comprenant au moins un composé selon la revendication 14 et un excipient ou diluant pharmaceutiquement acceptable.

51. Utilisation d'un composé de l'une quelconque des revendications 1 à 20, ou 37 à 47 dans la préparation d'une composition pharmaceutique pour le traitement d'un trouble inflammatoire.

52. L'utilisation de la revendication 51, dans laquelle le trouble inflammatoire est sélectionné parmi asthme, syndrome de détresse respiratoire de l'adulte, maladie pulmonaire obstructive chronique, maladie pulmonaire inflammatoire chronique, diabète, maladie inflammatoire de l'intestin, ostéoporose, psoriasis, rejet du greffon contre l'hôte, athérosclérose et arthrite dont polyarthrite rhumatoïde, arthrite psoriasique, arthrite traumatique, arthrite causée par la rubéole, arthrite associée à la goutte et ostéoarthrite.

53. Composé selon l'une quelconque des revendications 1 à 20, à utiliser dans une méthode de traitement d'un trouble inflammatoire.

54. Le composé de la revendication 53, où le trouble inflammatoire est sélectionné parmi asthme, syndrome de détresse respiratoire de l'adulte, maladie pulmonaire obstructive chronique, maladie pulmonaire inflammatoire chronique, diabète, maladie inflammatoire de l'intestin, ostéoporose, psoriasis, rejet du greffon contre l'hôte, athérosclérose et arthrite dont polyarthrite rhumatoïde, arthrite psoriasique, arthrite traumatique, arthrite causée par la rubéole, arthrite associée à la goutte et ostéoarthrite.
